(19) **Europäisches Patentamt / European Patent Office / Office européen des brevets**

(11) **EP 4 253 410 A1**

(12) ## EUROPEAN PATENT APPLICATION
published in accordance with Art. 153(4) EPC

(43) Date of publication:
04.10.2023 Bulletin 2023/40

(21) Application number: 21896952.5

(22) Date of filing: 23.11.2021

(51) International Patent Classification (IPC):
$C07K\ 14/82^{(2006.01)}$  $C07K\ 14/725^{(2006.01)}$
$A61K\ 39/00^{(2006.01)}$  $A61K\ 35/17^{(2015.01)}$
$A61P\ 35/00^{(2006.01)}$

(52) Cooperative Patent Classification (CPC):
A61K 35/17; A61K 39/00; A61P 35/00;
C07K 14/705; C07K 14/82

(86) International application number:
PCT/CN2021/132337

(87) International publication number:
WO 2022/111451 (02.06.2022 Gazette 2022/22)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 24.11.2020 PCT/CN2020/131149

(71) Applicant: Shanghai Genbase Biotechnology Co.,
Ltd.
China (Shanghai) Pilot Free Trade Zone
Shanghai 201203 (CN)

(72) Inventors:
• MOU, Nan
  Shanghai 201203 (CN)
• YU, Yue
  Shanghai 201203 (CN)
• YUAN, Jijun
  Shanghai 201203 (CN)

(74) Representative: Mewburn Ellis LLP
Aurora Building
Counterslip
Bristol BS1 6BX (GB)

Remarks:
The complete document including Reference
Table(s) and the Sequence Listing(s) can be
downloaded from the EPO website

(54) **RAS MUTANT EPITOPE PEPTIDE AND T CELL RECEPTOR RECOGNIZING RAS MUTANT**

(57) The present invention relates to the field of immunology and tumor treatment. Specifically, an Ras G12V mutant epitope peptide, an antigen presenting cell expressing the epitope peptide, a tumor vaccine containing same, and a use of the tumor vaccine in preventing or treating a tumor having RAS G12V mutation. The present invention further relates to a T cell receptor (TCR) specifically recognizing an Ras G12V mutant, a conjugate and a fusion protein containing the TCR, an immune cell expressing the TCR, a T cell drug containing same, and a use of the T cell drug in preventing or treating a tumor having RAS G12V mutation.

FIG. 7

EP 4 253 410 A1

**Description**

**Technical Field**

The present invention relates to the fields of immunology and tumor therapy. Specifically, the present invention relates to a RAS G12V mutant epitope peptide, an antigen-presenting cell expressing the epitope peptide, a tumor vaccine containing the same, and use thereof for preventing or treating a tumor with RAS G12V mutation. The present invention also relates to a T cell receptor (TCR) specifically recognizing RAS G12V mutant, a conjugate and fusion protein comprising the TCR, an immune cell expressing the TCR, and a T cell drug comprising the same, and use thereof for preventing or treating a tumor with RAS G12V mutation.

**Background Art**

RAS is a proto-oncogene that has GTPase activity and participates in many signaling pathways, such as MAPK, PI3K, and STAT signaling pathways that regulate cell proliferation, differentiation and apoptosis. In 1982, Der CJ et al. first confirmed that RAS gene mutation is a driving factor of cancers (Der CJ et al., 1982). There are three RAS-encoding genes in human gene, namely HRAS (GeneID: 3265), NRAS (GeneID: 4893) and KRAS (GeneID: 3845). The three RAS genes have a high degree of sequence homology (>90%). About 33% of human tumors carry RAS gene mutations, and RAS gene mutations have become the most frequent proto-oncogene mutations (Karnoub AE, 2008). The RAS gene mutation with the highest incidence rate in human tumors is KRAS, which accounts for about 22%, while NRAS accounts for about 8.0%, and HRAS accounts for about 3.3%.

KRAS gene mutations have the highest incidence (accounting for about 86% of the three RAS mutations) in solid tumors, such as colorectal cancer (30% to 50%), pancreatic cancer (about 85%) and non-small cell lung cancer (15% to 25%); >97% KRAS mutations mainly occur in Exon2 and Exon3, the mutation frequency at Exon2 is the highest (e.g., G12C, G12V, G12D, G13D, etc.), G12D and G12V mutations account for about 20% to 30% of colorectal cancers, 60% to 70% of pancreatic cancers, and about 38% of non-small cell lung cancers. KRAS mutation is a driving gene of tumor drug resistance. For example, when EGFR TKI and EGFR monoclonal antibody drugs (Cetuximab, etc.) are administrated, the KRAS mutation status in tumor patients should be detected, and the patients with KRAS mutation have a very low response (about 0~5%) to EGFR inhibitors (Jackman DM et al., 2009). The tumor patients with KRAS mutations have shorter progression-free survival and overall survival as compared to the tumor patients with KRAS wild-type; at the same time, the patients with KRAS mutations also have a higher possibility of postoperative recurrence and metastasis.

In recent decades, scientists' studies on the structure and biology of KRAS have shown that GTP binds to KRAS protein with an extremely high affinity (pM level), and it is difficult for small molecule inhibitors to inhibit KRAS activity by competing with GTP; other proteins that interact with KRAS in cells are involved in signaling, and small molecular compounds are also difficult to inhibit KRAS downstream signaling through competitive inhibition of protein-protein interaction; at the same time, the structure of KRAS protein shows that it has a relatively smooth structure and lacks a "pocket" to which a small molecule inhibitor binds, so that it is extremely difficult to develop a small molecule inhibitor targeting to KRAS protein itself and its related protein. The development of KRAS inhibitors mainly focused on the interference of modification of KRAS to block the synthesis thereof for the treatment of KRAS mutant tumors, such as the development of Farnesyl transferase inhibitors, but all of them ended in failure (Heidi Ledford, 2015). In recent years, the drug development for KRAS mutants has mainly focused on the KRAS G12C mutant. By designing compounds that can irreversibly and covalently bind to the cysteine residue in the G12C mutation, the KRAS G12C mutant is locked in an inactive state, thereby inhibiting KRAS mutant G12C activity; currently, no drugs are in development for other KRAS mutants (e.g., G12V and G13D).

Statistics from the World Health Organization (WHO) show that among the high-frequency cancers with RAS mutations in China (e.g., pancreatic cancer, colorectal cancer, lung cancer, endometrial cancer, ovarian cancer, and prostate cancer), the number of annual incidences reaches 1.6 million, in which 449,000 patients have RAS mutations (including G12, G13, Q61, etc.), and 108,000 patients have RAS G12V gene mutations. The patients with RAS mutations have higher tumor recurrence, drug resistance, poorer prognosis and shorter overall survival, so there is an urgent need for new treatments for these patient groups.

T cell receptor (TCR) recognizes the peptide sequences of viral protein and mutant gene transcription products presented by HLA, and TCR can specifically recognize peptides with mutations, therefore KRAS gene mutation is an ideal TCR target. Qiong JW et al. used KRAS G12V p7-16 and KRAS G12D p7-16 peptides to immunize transgenic mice expressing HLA-A*11:01, and screened to obtain the mouse TCR that recognizes HLA-A*11:01-restricted G12V and G12D (Qiong JW, 2015); Steven A. Rosenberg et al. found a TCR that recognizes HLA-C*08:02-restricted KRAS G12D by isolating tumor-infiltrating T cells from a patient with advanced metastatic colorectal disease, a good clinical response was obtained after in vitro expansion and reinfusion to the patient (Steven A. Rosenberg, 2016); and Steven A. Rosenberg et al. isolated and obtained a T cell receptor that recognizes KRAS G12V mutant from the T cells in peripheral blood of a patient with advanced endometrial cancer metastasis by using HLA-A*11:01 tetramer staining method (Steven A. Rosenberg, 2017).

T cell receptors are generated from VDJ gene rearrangement, and the naturally occurring T cell receptor library has a capacity of about $10^{16-20}$ (Harlan S. Robins, 2009); the T cell receptor library capacity is about 1000 to 10,000 times that of B cell receptors, such a huge library capacity corresponds to the human leukocyte antigen system. HLA is divided into type I (A, B, C, etc.) and type II (DP, DR, DQ, etc.), presenting peptides of different lengths (8 to 16 mer) respectively. The discovery of anti-KRAS mutant TCR is mainly carried out by the National Cancer Research Center of the United States. The currently discovered RAS mutant TCRs are HLA-A*11:01-restricted recognition of KRAS G12V and HLA-C*08:02-restricted recognition of KRAS G12D.

However, HLA alleles are half-inherited, with strong regional genetic distribution. The distribution of HLA in the Caucasian population is very different from that in the Asia-Pacific population. Therefore, it is urgent to discover novel T cell receptors against KRAS mutants that can be applied to the Asia-Pacific population.

## Contents of the present invention

The present invention provides a RAS G12V mutant epitope peptide, a T cell receptor (TCR) that specifically recognizes the epitope peptide, a cell and pharmaceutical composition comprising the epitope peptide or TCR, a nucleic acid encoding the epitope peptide or TCR, a vector and host cell for preparing the epitope peptide or TCR, and a method for treating a subject by using the epitope peptide or TCR. The epitope peptide and TCR provided by the present invention can be used to induce an immune response against a tumor with RAS G12V mutation and thus to treat the tumor in the subject. In addition, the epitope peptide and TCR provided by the present invention are MHC-II-restricted, and the MHC-II restriction is an allele showing predominantly high frequency in the Asia-Pacific populations, so it is especially suitable for patients in the Asia-Pacific region.

### Epitope peptide

Therefore, in a first aspect, the present invention provides an isolated epitope peptide or variant thereof, in which the epitope peptide consists of 11-30 (e.g., 30, 29, 28, 27, 26, 25, 24, 23, 22, 21, 20, 19, 18, 17, 16, 15, 14, 13, 12, or 11) consecutive amino acid residues of RAS G12V mutant, and comprises the amino acid residues at positions 3-13 of the RAS G12V mutant;

The variant differs from the epitope peptide from which it is derived only in a substitution of one or several (e.g., 1, 2 or 3) amino acid residues, and does not comprise an amino acid substitution at positions corresponding to amino acid positions 4, 5, 6, 9 and 12 of the RAS G12V mutant, and retains a biological function of the epitope peptide from which it is derived. The biological function comprises: an ability of being presented by an MHC-II molecule and subsequently recognized by a T cell, for example, being recognized by an antigen-specific T cell receptor on the T cell.

In certain embodiments, the epitope peptide or variant thereof of the present invention is an MHC-II restricted antigen, that is, the epitope peptide or variant thereof of the present invention can exhibit or present or form a complex with the background of an MHC-II molecule expressed on the surface of a cell. In certain embodiments, the epitope peptide or variant thereof of the present invention is capable of being presented by an MHC-II molecule, and the epitope peptide or variant thereof associated with the MHC-II molecule is capable of being recognized by a T cell, for example by an antigen-specific T cell receptor on the T cell.

In certain embodiments, the MHC-II molecule is HLA-DP. In certain embodiments, the HLA-DP comprises HLA-DPB1*03:01 or HLA-DPB1*14:01. In certain embodiments, the HLA-DP further comprises one selected from the group consisting of HLA-DPA1*02:02, HLA-DPA1*02:01, and HLA-DPA1*01:03. In certain embodiments, the MHC-II molecule is HLA-DPB1*03:01 or HLA-DPB1*14:01, and HLA-DPA1 *02:02. In certain embodiments, the MHC-II molecule is HLA-DPB1*03:01 or HLA-DPB1*14:01, and HLA-DPA1*02:01. In certain embodiments, the MHC-II molecule is HLA-DPB1*03:01 or HLA-DPB1*14:01, and HLA-DPA1*01:03.

In certain embodiments, the epitope peptide consists of 11-25 (e.g., 11-23, 11-13) consecutive amino acid residues of the RAS G12V mutant.

In certain embodiments, the RAS G12V mutant is KRAS G12V mutant. In certain embodiments, the RAS G12V mutant has a sequence set forth in SEQ ID NO: 1 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto.

In certain embodiments, the amino acid residues at positions 3-13 of RAS G12V mutant have a sequence set forth in SEQ ID NO: 2.

In certain embodiments, the epitope peptide comprises or consists of the amino acid residues at positions 3-13 (e.g., set forth in SEQ ID NO: 2) of the RAS G12V mutant. In certain embodiments, the epitope peptide comprises or consists of the amino acid residues at positions 2-13 (e.g., set forth in SEQ ID NO: 3) of the RAS G12V mutant. In certain embodiments, the epitope peptide comprises or consists of the amino acid residues at positions 3-14 (e.g., set forth in SEQ ID NO: 4) of the RAS G12V mutant. In certain embodiments, the epitope peptide comprises or consists of the amino acid residues at positions 2-14 (e.g., set forth in SEQ ID NO: 5) of the RAS G12V mutant.

In certain embodiments, the epitope peptide consists of the amino acid residues at positions 2-14 (e.g., set forth in SEQ ID NO: 5) of the RAS G12V mutant.

In certain embodiments, the epitope peptide comprises or consists of a sequence set forth in any one of SEQ ID NOs: 2-5. In certain embodiments, the variant comprises or consists of a sequence selected from the group consisting of (i) a sequence having a substitution, deletion or addition of one or several amino acids (e.g., a substitution, deletion or addition of 1, 2, 3, 4 or 5 amino acids) compared to the sequence set forth in any one of SEQ ID NOs: 2-5; (ii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in any one of SEQ ID NOs: 2-5.

In another aspect, the present invention provides an MHC-peptide complex, which comprises the epitope peptide or variant thereof of the present invention and an MHC-II molecule associated therewith. In certain embodiments, the MHC-II molecule is HLA-DP. In certain embodiments, the HLA-DP comprises HLA-DPB1*03:01 or HLA-DPB1*14:01. In some embodiments, the HLA-DP further comprises one selected from the group consisting of HLA-DPA1*02:02, HLA-DPA1*02:01, and HLA-DPA1*01:03. In certain embodiments, the MHC-II molecule is HLA-DPB1*03:01 or HLA-DPB1*14:01, and HLA-DPA1*02:02. In certain embodiments, the MHC-II molecule is HLA-DPB1*03:01 or HLA-DPB1*14:01, and HLA-DPA1*02:01. In certain embodiments, the MHC-II molecule is HLA-DPB1*03:01 or HLA-DPB1*14:01, and HLA-DPA1*01:03.

In certain embodiments, the MHC-peptide complex is present on the surface of a cell. Thus, the present invention also encompasses a cell expressing the MHC-peptide complex.

T cell receptor

In a second aspect, the present invention provides an isolated T cell receptor or antigen-binding fragment thereof, which is capable of specifically recognizing the epitope peptide or variant thereof of the present invention. In certain embodiments, the epitope peptide or variant thereof is presented by an MHC-II molecule. In certain embodiments, the MHC-II molecule is HLA-DP. In certain embodiments, the HLA-DP comprises HLA-DPB1*03:01 or HLA-DPB1*14:01. In some embodiments, the HLA-DP further comprises one selected from the group consisting of HLA-DPA1*02:02, HLA-DPA1*02:01, and HLA-DPA1*01:03.

In a third aspect, the present invention provides an isolated T cell receptor or antigen-binding fragment thereof, which is capable of specifically recognizing a RAS G12V mutant, the TCR or antigen-binding fragment thereof comprising an $\alpha$-chain variable region ($V\alpha$) and/or a $\beta$-chain variable region ($Y\beta$), wherein,

(a) the $V\alpha$ comprises CDR1$\alpha$, CDR2$\alpha$ and CDR3$\alpha$, wherein the CDR3$\alpha$ comprises a sequence as set forth in AVRDX$_1$X$_2$X$_3$GGNKLT (SEQ ID NO: 25); wherein:

X$_1$ is selected from the group consisting of G, A, D, L, M, Q, R, S, V, W, Y; preferably G, A, D, R;

X$_2$ is selected from the group consisting of R, A, D, G, H, I, K, L, M, N, P, Q, S, T, V, W, Y; preferably R or T;

X$_3$ is selected from the group consisting of G, A, D, E, F, H, I, K, L, M, N, P, Q, R, S, T, V, W, Y; preferably G, A, H, N, S or W;
and/or,

(b) the $V\beta$ comprises CDR1$\beta$, CDR2$\beta$ and CDR3$\beta$, wherein the CDR3$\beta$ comprises a sequence as set forth in ASSX$_4$GX$_5$RDNSPLH (SEQ ID NO: 26); wherein:

X$_4$ is selected from the group consisting of P, A, H, M, S, T, V, Y; preferably P, A, or T;

X$_5$ is selected from the group consisting of Q or S.

The TCR of the second or third aspect may be used in any TCR structure.

In certain embodiments, the TCR may be a full-length TCR comprising a full-length $\alpha$-chain and a full-length $\beta$-chain.

In certain embodiments, the TCR is a soluble TCR that lacks one or more transmembrane and/or cytoplasmic regions. In certain embodiments, the soluble TCR is generated by fusing an extracellular domain of the TCR of the present invention to another protein domain (e.g., maltose-binding protein, thioredoxin, human constant κ domain, or leucine zipper), see for example, Front Oncol., 2014;4:378, which is hereby incorporated by reference in its entirety.

In certain embodiments, the TCR of the present invention may also be a single-chain TCR (scTCR), which comprises Vα and Vβ linked by a peptide linker. Such scTCR may comprise Vα and Vβ, and the Vα and Vβ are each linked to a TCR constant region. Alternatively, the scTCR may comprise Vα and Vβ, wherein Vα, Vβ, or both Vα and Vβ are not linked to a TCR constant region. Exemplary scTCRs are described in PCT Publication Nos: WO2003/020763, WO 2004/033685, and WO 2011/044186, and each of these disclosures is incorporated herein by reference in its entirety.

In certain embodiments, the TCR of the present invention may comprise two polypeptide chains (e.g.,α-chain and β-chain), wherein the chains have been engineered to comprise cysteine residues capable of forming an interchain disulfide bond. Thus, the TCR of the present invention may comprise two polypeptide chains linked by an engineered disulfide bond. Exemplary TCRs with engineered disulfide bond are described in US Patent Nos: 8,361,794 and 8,906,383, each of which is incorporated herein by reference in its entirety.

In certain embodiments, the T cell receptor of the second or third aspect is a membrane-bound or soluble T cell receptor. In certain embodiments, the T cell receptor of the second or third aspect is a full-length TCR, a soluble TCR or a single-chain TCR.

In certain embodiments, the TCR or antigen-binding fragment thereof has characteristics of one or more of the following items:

(i) the CDR1α comprises a sequence as set forth in VSGX$_6$PY (SEQ ID NO: 27); wherein, X$_6$ is selected from the group consisting of N, A, D, E, F, G, H, I, K, L, M, P, Q, R, S, T, V, W, Y (preferably N, E, P, Q, R or S);

(ii) the CDR2α comprises a sequence as set forth in YX$_7$X$_8$GDNLV (SEQ ID NO: 28); wherein, X$_7$ is selected from the group consisting of I, A, D, E, F, G, H, K, L, M, N, P, Q, R, S, T, V, W, Y (preferably I, D, E, F, G, H, N, P, R, W or Y); X$_8$ is selected from the group consisting of T, A, D, E, H, I, L, N, Q, R, S, W, Y (preferably T, A, D, H, I, Q or R);

(iii) the CDR1β comprises a sequence set forth in SEQ ID NO: 11;

(iv) the CDR2β comprises a sequence as set forth in SX$_9$X$_{10}$VNX$_{11}$ (SEQ ID NO: 29); wherein, X$_9$ is selected from the group consisting of Q, A, F, G, H, I, K, L, M, N, P, R, S, T, V, W, Y (preferably Q, A, I, M, N, S, T or Y); X$_{10}$ is selected from the group consisting of I, F, H, T, V (preferably I or T); X$_{11}$ is selected from the group consisting of D, A, E, F, G, H, I, K, L, M, N, P, Q, R, S, T, V, W, Y (preferably D, G, L, M, N, T or Y).

In certain embodiments, the TCR or antigen-binding fragment thereof comprises:

(1) CDR1α as set forth in SEQ ID NO: 8, CDR2α as set forth in SEQ ID NO: 9, CDR3α as set forth in SEQ ID NO: 25; and/or, CDR1β as set forth in SEQ ID NO: 11, CDR2β as set forth in SEQ ID NO: 12, CDR3β as set forth in SEQ ID NO: 13;

(2) CDR1α as set forth in SEQ ID NO: 8, CDR2α as set forth in SEQ ID NO: 9, CDR3α as set forth in SEQ ID NO: 10; and/or, CDR1β as set forth in SEQ ID NO: 11, CDR2β as set forth in SEQ ID NO: 12, CDR3β as set forth in SEQ ID NO: 26;

(3) CDR1α as set forth in SEQ ID NO: 27, CDR2α as set forth in SEQ ID NO: 9, CDR3α as set forth in SEQ ID NO: 10; and/or, CDR1β as set forth in SEQ ID NO: 11, CDR2β as set forth in SEQ ID NO: 12, CDR3β as set forth in SEQ ID NO: 13;

(4) CDR1α as set forth in SEQ ID NO: 8, CDR2α as set forth in SEQ ID NO: 28, CDR3α as set forth in SEQ ID NO: 10; and/or, CDR1β as set forth in SEQ ID NO: 11, CDR2β as set forth in SEQ ID NO: 12, CDR3β as set forth in SEQ ID NO: 13;

(5) CDR1α as set forth in SEQ ID NO: 8, CDR2α as set forth in SEQ ID NO: 9, CDR3α as set forth in SEQ ID NO: 10; and/or, CDR1β as set forth in SEQ ID NO: 11, CDR2β as set forth in SEQ ID NO: 29, CDR3β as set forth in SEQ ID NO: 13; or

(6) CDR1α as set forth in SEQ ID NO: 8, CDR2α as set forth in SEQ ID NO: 9, CDR3α as set forth in SEQ ID NO: 10; and/or, CDR1β as set forth in SEQ ID NO: 11, CDR2β as set forth in SEQ ID NO: 12, CDR3β as set forth in SEQ ID NO: 13.

In certain embodiments, the Vα comprises FR1α, FR2α, FR3α, and FR4α, wherein:

the FR1α comprises a sequence set forth in SEQ ID NO: 30;

the FR2α comprises a sequence as set forth in LFWYVQYPNRGLQFLLX$_{12}$ (SEQ ID NO: 38); wherein, X$_{12}$ is selected from the group consisting of K, A, D, E, F, G, H, I, L, M, N, P, Q, R, S, T, V, W, Y (preferably K, G, H, N, P, R, S, V, W or Y);

the FR3α comprises a sequence set forth in SEQ ID NO: 32;

the FR4α comprises a sequence set forth in SEQ ID NO: 33.

In certain embodiments, the Vβ comprises FR1β, FR2β, FR3β, and FR4β, wherein:

the FR1β comprises a sequence set forth in SEQ ID NO: 34;

the FR2β comprises a sequence as set forth in MYWYRQDPGQGLRLIYX$_{13}$ (SEQ ID NO: 39); wherein, X$_{13}$ is selected from the group consisting of Y, A, E, F, I, K, L, M, N, P, Q, R, S, T, V, W (preferably Y, A, E, F or K);

the FR3β comprises a sequence as set forth in FX$_{14}$KGDIAEGYSVSREKKESFPLTVTSAQKNPTAFYLC (SEQ ID NO: 40); wherein, X$_{14}$ is selected from the group consisting of Q, A, D, E, F, G, H, I, K, L, M, N, P, R, S, T, V, W, Y (preferably Q, A, D, E, F, H, L, R, T or W);

the FR4β comprises a sequence set forth in SEQ ID NO: 37.

In certain embodiments, the TCR or antigen-binding fragment thereof comprises:

(1) FR1α as set forth in SEQ ID NO: 30, FR2α as set forth in SEQ ID NO: 31, FR3α as set forth in SEQ ID NO: 32, FR4α as set forth in SEQ ID NO: 33; and/or, FR1β as set forth in SEQ ID NO: 34, FR2β as set forth in SEQ ID NO: 35, FR3β as set forth in SEQ ID NO: 36, FR4β as set forth in SEQ ID NO: 37;

(2) FR1α as set forth in SEQ ID NO: 30, FR2α as set forth in SEQ ID NO: 38, FR3α as set forth in SEQ ID NO: 32, FR4α as set forth in SEQ ID NO: 33; and/or, FR1β as set forth in SEQ ID NO: 34, FR2β as set forth in SEQ ID NO: 35, FR3β as set forth in SEQ ID NO: 36, FR4β as set forth in SEQ ID NO: 37;

(3) FR1α as set forth in SEQ ID NO: 30, FR2α as set forth in SEQ ID NO: 31, FR3α as set forth in SEQ ID NO: 32, FR4α as set forth in SEQ ID NO: 33; and/or, FR1β as set forth in SEQ ID NO: 34, FR2β as set forth in SEQ ID NO: 39, FR3β as set forth in SEQ ID NO: 36, FR4β as set forth in SEQ ID NO: 37; or

(4) FR1α as set forth in SEQ ID NO: 30, FR2α as set forth in SEQ ID NO: 31, FR3α as set forth in SEQ ID NO: 32, FR4α as set forth in SEQ ID NO: 33; and/or, FR1β as set forth in SEQ ID NO: 34, FR2β as set forth in SEQ ID NO: 35, FR3β as set forth in SEQ ID NO: 40, FR4β as set forth in SEQ ID NO: 37.

In certain embodiments, the Vα comprises a sequence set forth in SEQ ID NO: 6 or variant thereof, and the Vβ comprises a sequence set forth in SEQ ID NO: 7 or variant thereof, the variant has a substitution, deletion or addition of one or several amino acids (e.g., a substitution, deletion or addition of 1, 2, 3, 4 or 5 amino acids) as compared to the sequence from which it is derived.
In certain embodiments, the Vα comprises a sequence set forth in SEQ ID NO: 6 or variant thereof, wherein the variant has an amino acid substitution at one or more (e.g., 1, 2, 3 or 4) amino acid positions selected from the group consisting of positions 30, 49, 51, 52, 96, 97, 98 as determined according to the IMGT TCR numbering system.
In certain embodiments, the Vβ comprises a sequence set forth in SEQ ID NO: 7 or variant thereof, wherein the variant has an amino acid substitution at one or more (e.g., 1, 2, 3 or 4) amino acid positions selected from the group consisting of positions 48, 50, 51, 54, 56, 95, 97 as determined according to the IMGT TCR numbering system.
In certain embodiments, the Vα comprises a variant of the sequence set forth in SEQ ID NO: 6, the variant comprises one or more (e.g., 1, 2, 3 or 4) amino acid substitutions selected from the followings, in which the amino acid position is determined according to the IMGT TCR numbering system: substitution of the amino acid at position 30 with A, D, E, F, G, H, I, K, L, M, P, Q, R, S, T, V, W or Y (preferably E, P, Q, R or S); substitution of the amino acid at position 49 with A, D, E, F, G, H, I, L, M, N, P, Q, R, S, T, V, W or Y (preferably G, H, N, P, R, S, V, W or Y); substitution of the amino acid at position 51 with A, D, E, F, G, H, K, L, M, N, P, Q, R, S, T, V, W or Y (preferably D, E, F, G, H, N, P, R, W or Y); substitution of the amino acid at position 52 with A, D, E, H, I, L, N, Q, R, S, W or Y (preferably A, D, H, I, Q or R); substitution of the amino acid at position 96 with A, D, L, M, Q, R, S, V, W or Y (preferably A, D or R); substitution of the amino acid at position 97 with A, D, G, H, I, K, L, M, N, P, Q, S, T, V, W or Y (preferably T); substitution of the amino acid at position 98 with A, D, E, F, H, I, K, L,M, N, P, Q, R, S, T, V, W or Y (preferably A, H, N, S or W). In such embodiments, the Vβ preferably comprises a sequence set forth in SEQ ID NO:7.

In certain embodiments, the Vβ comprises a variant of the sequence set forth in SEQ ID NO: 7, the variant comprises one or more (e.g., 1, 2, 3 or 4) amino acid substitutions selected from the followings, in which the amino acid position is determined according to the IMGT TCR numbering system: substitution of the amino acid at position 48 with A, E, F, I, K, L, M, N, P, Q, R, S, T, V or W (preferably A, E, F or K); substitution of the amino acid at position 50 with A, F, G, H, I, K, L, M, N, P, R, S, T, V, W or Y (preferably A, I, M, N, S, T or Y); substitution of the amino acid at position 51 with F, H, T or V (preferably T); substitution of the amino acid at position 54 with A, E, F, G, H, I, K, L, M, N, P, Q, R, S, T, V, W or Y (preferably G, L, M, N, T or Y); substitution of the amino acid at position 56 with A, D, E, F, G, H, I, K, L, M, N, P, R, S, T, V, W or Y (preferably A, D, E, F, H, L, R, T or W); substitution of the amino acid at position 95 with A, H, M, S, T, V or Y (preferably A or T); substitution of the amino acid at position 97 with S. In such embodiments, the Vα preferably comprises a sequence set forth in SEQ ID NO:6.

In certain embodiments, the TCR or antigen-binding fragment thereof of the present invention comprises a Vα set forth in SEQ ID NO: 6 and/or a Vβ set forth in SEQ ID NO: 7.

In certain embodiments, the TCR or antigen-binding fragment thereof can specifically recognize the epitope peptide or variant thereof of the present invention (e.g., the sequence set forth in any one of SEQ ID NOs: 2-5). In certain embodiments, the epitope peptide or variant thereof is presented by an MHC-II molecule. In certain embodiments, the MHC-II molecule is HLA-DP. In certain embodiments, the HLA-DP comprises HLA-DPB1*03:01 or HLA-DPB1*14:01. In some embodiments, the HLA-DP further comprises one selected from the group consisting of HLA-DPA1*02:02, HLA-DPA1*02:01, and HLA-DPA1*01:03.

In certain embodiments, a T cell expressing on its surface the TCR or antigen-binding fragment thereof is activated under co-cultivation with a second cell displaying the epitope peptide or variant thereof of the present invention (e.g., displaying in the context of MHC-II). In certain embodiments, the activation of the T cell can be measured using any suitable indicator known in the art. Non-limiting examples of such suitable indicators include: increases in secretion level of cytokine (e.g., IL-2, IFN-γ, etc.), proliferation activity, and/or expression level of activation marker (e.g., CD25, CD69, CD107a, etc.). In certain embodiments, the activation of the T cell also comprises the apoptosis or death induced by the T cell of a second cell displaying (e.g., displaying in the context of MHC-II) the epitope peptide or variant thereof of the present invention.

Conjugate and fusion protein

In a fourth aspect, the present invention provides a conjugate, which comprises the TCR or antigen-binding fragment thereof described in the second or third aspect and an effector moiety conjugated thereto.

In this context, the term "effector moiety" refers to a component or functional group that is capable of modulating (e.g., increasing or decreasing) a natural activity of a molecule to which it is linked or conferring a novel activity on the molecule. In some embodiments, the effector moiety is a compound or polypeptide with biological activity (e.g., a compound or polypeptide having an effect on a cell targeted by the TCR), or a detectable label.

In this context, the term "conjugation" refers to any method known in the art for functionally linking a protein domain, including but not limited to: recombinant fusion with or without a linker, intein-mediated fusion, non-covalent bonding and covalent bonding, such as disulfide bonding, peptide bonding, hydrogen bonding, electrostatic bonding, and conformational bonding, such as biotin-avidin interaction. In certain embodiments, the conjugation to effector moiety can be performed by chemical or recombinant method, and the chemical method comprises forming a covalent bond between two molecules so as to form one molecule.

In certain embodiments, the effector moiety may be a therapeutic moiety. A therapeutic moiety refers to a compound or polypeptide that is useful as a therapeutic agent. The conjugate takes advantage of the targeting ability of the TCR to allow the therapeutic moiety to exert a therapeutic effect on a cell targeted by the TCR.

In certain embodiments, the TCR or antigen-binding fragment thereof of the present invention is optionally conjugated to an effector moiety via a linker (e.g., a peptide linker). In certain embodiments, the effector moiety is linked to the N- or C-terminal of the TCR or antigen-binding fragment thereof of the present invention.

In certain embodiments, when the effector moiety is a peptide or protein, the conjugate is preferably a fusion protein.

Therefore, in the fifth aspect, the present invention also provides a fusion protein, which comprises the TCR or antigen-binding fragment thereof as described in the second or third aspect and an additional peptide or protein.

In certain embodiments, the TCR or antigen-binding fragment thereof of the present invention is fused to an additional peptide or protein, optionally via a peptide linker. In certain embodiments, the additional peptide or protein is linked to the N- or C-terminal of the TCR or antigen-binding fragment thereof of the present invention.

In certain embodiments, the additional peptide or protein may be selected from the effector moieties described in the fourth aspect that are peptides or proteins.

Preparation of epitope peptide, TCR and fusion protein

The epitope peptide, TCR or fusion protein containing the TCR of the present invention can be prepared by various methods known in the art, for example, by genetic engineering recombination technology. For example, DNA molecules encoding the same can be obtained by chemical synthesis or PCR amplification; the resulting DNA molecules can be inserted into expression vectors, and then transfected into host cells; then, the transfected host cells can be cultivated under specific conditions to express the epitope peptide, TCR or fusion protein comprising the TCR of the present invention.

Accordingly, in a sixth aspect, the present invention provides an isolated nucleic acid molecule, which comprises:

(i) a nucleotide sequence encoding the epitope peptide or variant thereof in the first aspect;

(ii) a nucleotide sequence encoding the TCR or antigen-binding fragment thereof described in the second or third aspect or $\alpha$-chain variable region and/or $\beta$-chain variable region thereof;

(iii) a nucleotide sequence encoding the fusion protein described in the fifth aspect.

In certain embodiments, the isolated nucleic acid molecule comprises a first nucleotide sequence encoding the $\alpha$-chain variable region and a second nucleotide sequence encoding the $\beta$-chain of the TCR or antigen-binding fragment thereof described in the second or third aspect. In certain embodiments, the first nucleotide sequence and the second nucleotide sequence are optionally linked by a nucleotide sequence encoding a self-cleaving peptide (e.g., P2A, E2A, F2A or T2A). In certain embodiments, the self-cleaving peptide is P2A.

In certain embodiments, the isolated nucleic acid molecule comprises a first nucleotide sequence encoding the TCR $\alpha$-chain variable region and a second nucleotide sequence encoding the TCR $\beta$-chain variable region.

In certain embodiments, the isolated nucleic acid molecule comprises a first nucleotide sequence encoding the $\alpha$-chain of the TCR or antigen-binding fragment thereof and a second nucleotide sequence encoding the $\beta$-chain of the TCR or antigen-binding fragment thereof.

In certain embodiments, the first nucleotide sequence and the second nucleotide sequence are present on different isolated nucleic acid molecules.

In certain embodiments, the first nucleotide sequence and the second nucleotide sequence are present in any order on the same isolated nucleic acid molecule; in certain embodiments, the first nucleotide sequence and the second nucleotide sequence are linked in any order by a nucleotide sequence encoding a self-cleaving peptide (e.g., P2A).

In a seventh aspect, the present invention provides a vector (e.g., a cloning vector or an expression vector) comprising the isolated nucleic acid molecule of the sixth aspect. In certain embodiments, the vector of the present invention is, for example, plasmid, cosmid, phage, and the like. In certain embodiments, the vector comprises a nucleotide sequence encoding the TCR or antigen-binding fragment thereof as described above, or $\alpha$-chain variable region and/or $\beta$-chain variable region thereof, or $\alpha$-chain and/or $\beta$-chain thereof.

In certain embodiments, the vector comprises a first nucleotide sequence encoding the TCR $\alpha$-chain variable region and a second nucleotide sequence encoding the TCR $\beta$-chain variable region.

In certain embodiments, the vector comprises a first nucleotide sequence encoding the $\alpha$-chain of the TCR or antigen-binding fragment thereof and a second nucleotide sequence encoding the $\beta$-chain of the TCR or antigen-binding fragment thereof.

In certain embodiments, the first nucleotide sequence and the second nucleotide sequence are present on different vectors.

In certain embodiments, the first nucleotide sequence and the second nucleotide sequence are present in any order on the same vector. In certain embodiments, the first nucleotide sequence and the second nucleotide sequence are linked in any order by a nucleotide sequence encoding a self-cleaving peptide (e.g., P2A).

In certain embodiments, the vector comprises a nucleotide sequence encoding the fusion protein as described above.

In certain embodiments, the vector is a viral vector, such as a lentiviral vector, a retroviral vector, an adenoviral vector, an adeno-associated viral vector, or a baculoviral vector.

In an eighth aspect, the present invention provides a host cell, which comprises the isolated nucleic acid molecule of the sixth aspect or the vector of the seventh aspect. Such host cells include, but are not limited to, prokaryotic cells such as E. coli cells, and eukaryotic cells such as yeast cells, insect cells, plant cells, and animal cells (e.g., mammalian cells, such as mouse cells, human cells, etc.).

In another aspect, there is also provided a method for preparing the epitope peptide, TCR or fusion protein comprising the TCR of the present invention, which comprises culturing the host cell described in the eighth aspect under conditions that allow protein expression, recovering the epitope peptide, TCR or fusion protein comprising TCR from a culture of the cultured host cell.

Engineered antigen-presenting cell (APC)

The epitope peptide and variant thereof of the present invention can be used in T cell-based immunotherapy. In some cases, T cells can recognize an MHC-peptide complex presented on the surface of APC through its TCR to induce an MHC-restricted immune response to RAS mutant.

Therefore, in a ninth aspect, the present invention provides an engineered antigen-presenting cell (APC), which presents on its surface the epitope peptide or variant thereof of the first aspect.

In certain embodiments, the epitope peptide or variant thereof is presented by an MHC-II molecule. In certain embodiments, the MHC-II molecule is HLA-DP. In certain embodiments, the HLA-DP comprises HLA-DPB1*03:01 or HLA-DPB1*14:01. In some embodiments, the HLA-DP further comprises one selected from the group consisting of HLA-DPA1*02:02, HLA-DPA1*02:01, and HLA-DPA1*01:03.

In certain embodiments, the APC is selected from the group consisting of dendritic cell, monocyte, macrophage, B lymphocyte (e.g., B-lymphoblastoid cell B-LCL), or any combination thereof.

In certain embodiments, the APC is positive for HLA-DPB1*03:01 or positive for HLA-DPB1*14:01. In certain embodiments, the APC is further positive for HLA-DPA1*02:02, positive for HLA-DPA1*02:01, or positive for HLA-DPA1*01:03. In certain embodiments, the APC has an HLA genotype of HLA-DPB1*03:01/HLA-DPA1*02:02, HLA-DPB1*03:01/HLA-DPA1*02:01, or HLA-DPB1*03:01/HLA-DPA1*01:03, HLA-DPB1*14:01/HLA-DPA1*02:02, HLA-DPB1*14:01/HLA-DPA1*02:01, or HLA-DPB1*14:01/HLA-DPA1*01:03.

In certain embodiments, the APC is isolated from an HLA-DPB1*03:01 positive subject or an HLA-DPB1*14:01 positive subject. In certain embodiments, the subject is further positive for HLA-DPA1*02:02, positive for HLA-DPA1*02:01, or positive for HLA-DPA1*01:03. In certain embodiments, the subject has an HLA genotype of HLA-DPB1*03:01/HLA-DPA1*02:02, HLA-DPB1*03:01/HLA-DPA1*02:01, or HLA-DPB1*03:01/HLA-DPA1*01:03, HLA-DPB1*14:01/HLA-DPA1*02:02, HLA-DPB1*14:01/HLA-DPA1*02:01, or HLA-DPB1*14:01/HLA-DPA1*01:03.

In certain embodiments, the engineered APC is obtained by contacting the APC with the epitope peptide or variant thereof described in the first aspect (i.e., exposing the APC to a sufficient amount of the epitope peptide or variant thereof) in vitro. In some embodiments, the engineered APC is obtained by introducing an expression vector comprising a nucleotide sequence encoding the epitope peptide or variant thereof described in the first aspect into the APC in vitro.

The APC of the ninth aspect may be an autologous/autogenous ("self") or non-autologous ("non-self", for example, allogeneic) cell. "Autologous" cell refers to a cell from the same subject; "allogeneic" cell refers to a cell of the same species that is genetically distinct from the cell being compared.

The APC of the ninth aspect may be isolated or obtained from any tissue in which such cell is found, or may be otherwise cultured and provided. For example, the APC can be found in bone marrow or peripheral blood mononuclear cells (PBMC) of mammal, in spleen of mammal, or in skin of mammal (i.e., Langerhans cells that can be found in skin, which possess some characteristics similar to those of DC), and then cultured in a medium containing appropriate cytokines, followed by sorting, so as to obtain the APC.

In another aspect, the present invention provides a method for preparing the above-mentioned engineered APC, which comprises: (1) providing the APC from a subject; (2) in vitro contacting the APC with the epitope peptide or variant thereof described in the first aspect, or introducing an expression vector comprising a nucleotide sequence encoding the epitope peptide or variant thereof described in the first aspect into the APC, to obtain an APC capable of presenting on its surface the epitope peptide or variant thereof.

Engineered immune cell

The TCR or antigen-binding fragment thereof of the present invention can be used in T cell-based immunotherapy. In some instances, T cells expressing the TCR of the present invention induces an MHC-restricted immune response to RAS mutant by recognizing an MHC-peptide complex.

Therefore, in the tenth aspect, the present invention provides an engineered immune cell that expresses on its surface the TCR or antigen-binding fragment thereof described in the second or third aspect. The engineered immune cell of the present invention has an antigen-specificity to RAS G12V mutant. In certain embodiments, the engineered immune cell of the present invention has one or more characteristics selected from the following items:

(i) specifically binding to RAS G12V mutant, but not binding or with a lower affinity binding to other RAS proteins (comprising wild-type RAS protein or other mutants);

(ii) specifically binding to the epitope peptide or variant thereof described in the first aspect (e.g., the sequence set forth in any one of SEQ ID NOs: 2-5, especially SEQ ID NO: 5);

(iii) activation upon co-culturing with an APC displaying (e.g., displaying in the context of MHC-II) the epitope peptide or variant thereof of the present invention, the non-limiting examples of such activation including: increases in secretion level of cytokine (e.g., IL-2, IFN-γ, etc.), proliferation activity, and/or expression level of activation marker (e.g., CD25, CD69, CD107a, etc.), and increase in killing activity on a second cell displaying (e.g., displaying in the context of MHC-II) the epitope peptide or variant thereof of the present invention.

In certain embodiments, the engineered immune cell comprises a nucleotide sequence encoding the TCR or antigen-binding fragment thereof as described in the second or third aspect.

The immune cell of the tenth aspect may be isolated or obtained from any tissue in which such cell is found. For example, the APC can be found in mammalian peripheral blood mononuclear cells, bone marrow, lymph node tissue, umbilical cord blood, thymus tissue, ascitic fluid, pleural effusion, spleen tissue or tumors, and then optionally cultured in a culture medium containing appropriate cytokines and sorted, thereby obtaining the desired immune cell. Alternatively, the immune cell can also be cultured and provided in other ways, for example, obtained from a precursor cell of the immune cell (e.g., precursors of T lymphocyte) through induction and differentiation, and the precursor cell can be, for example, pluripotent stem cell (e.g., embryonic stem cell, induced pluripotent stem cell), hematopoietic stem cell or lymphocyte progenitor cell, hematopoietic stem cell or lymphocyte progenitor cell that is isolated and/or enriched from, for example, bone marrow, umbilical cord blood or peripheral blood.

In certain embodiments, the immune cell is a lymphocyte. In certain embodiments, the immune cell is selected from the group consisting of T cell, tumor infiltrating lymphocyte (TIL), natural killer (NK) cell, natural killer T (NKT) cell, or any combination thereof. Exemplary immune cells that can be used to express the TCR of the present invention include PBMC, TIL and/or T cell. In certain embodiments, the T cell is selected from the group consisting of αβ T cell, γδ T cell, iPSC-derived T cell, CD8+ cytotoxic T cell, CD4+ cytotoxic T cell, CD4+ helper T cell (e.g., Th1 or Th2 cell), CD4/CD8 double positive T cell, tumor infiltrating T cell, thymocyte, memory T cell, natural killer T cell, for example, invariant natural killer T cell. In certain embodiments, the immune cell comprises CD4+ T cell. Those skilled in the art will understand that immune cell may also include a progenitor cell (precursor cell) of immune cell, wherein the progenitor cell may be induced to differentiate into the immune cell in vivo or in vitro. Thus, in certain embodiments, the immune cell comprises a progenitor cell of the immune cell, such as hematopoietic stem cell (HSC) contained within a population of CD34+ cells derived from cord blood, bone marrow, or flowing peripheral blood, which is differentiated into a mature immune cell upon administration to a subject, or it can be induced to differentiate into a mature immune cell in vitro.

The immune cell of the tenth aspect may be an autologous/autogenous ("self") or non-autologous ("non-self", for example, allogeneic) cell. "Autologous" cell refers to a cell from the same subject; and "allogeneic" cell refers to a cell of the same species that is genetically distinct from the cell being compared.

In certain embodiments, the immune cell is isolated from a subject positive for HLA-DPB1*03:01 or a subject positive for HLA-DPB1*14:01. In certain embodiments, the subject is further positive for HLA-DPA1*02:02, positive for HLA-DPA1*02:01, or positive for HLA-DPA1*01:03. In certain embodiments, the subject has an HLA genotype of HLA-DPB1*03:01/HLA-DPA1*02:02, HLA-DPB1*03:01/HLA-DPA1*02:01, or HLA-DPB1*03:01/HLA-DPA1*01:03, HLA-DPB1*14:01/HLA-DPA1*02:02, HLA-DPB1*14:01/HLA-DPA1*02:01, or HLA-DPB1*14:01/HLA-DPA1*01:03.

It is understood that the engineered immune cell of the present invention may be contained in an isolated cell population. The cell population may be a heterogeneous population, for example, the cell population comprises, in addition to the engineered immune cell of the present invention, at least one other cell that has no antigen-specificity for the RAS G12V mutant; alternatively, for example, the cell population comprises more than one type of immune cells, but these types of immune cells all express the TCR of the present invention so as to have antigen-specificity for the RAS G12V mutant. Furthermore, the cell population can also be a substantially homogeneous population, for example, wherein the population mainly comprises (e.g., consists essentially of) T cells that have antigen-specificity for the RAS G12V mutant.

In another aspect, the present invention provides a method for preparing the above-mentioned engineered immune cell, which comprises: (1) providing an immune cell from a subject; (2) introducing a nucleic acid molecule or vector comprising a nucleotide sequence encoding the TCR or antigen-binding fragment thereof of the present invention into the immune cell described in step (1), so as to obtain an immune cell expressing the TCR or antigen-binding fragment thereof.

In some embodiments, in step (1), the immune cell undergoes pretreatment; the pretreatment comprises sorting, activating and/or proliferating the immune cell. In certain embodiments, the pretreatment comprises contacting the immune cell with one or more selected from the group consisting of anti-CD3 antibody, anti-CD28 antibody, IL-2 and IL-15, thereby stimulating the immune cell and inducing proliferation thereof, and generating a pretreated immune cell.

In some embodiments, in step (2), the nucleic acid molecule or vector can be introduced into the immune cell by various suitable methods, such as calcium phosphate transfection, DEAE-dextran-mediated transfection, microinjection, electroporation, TALEN method, ZFN method, non-viral vector-mediated transfection (e.g. liposome) or viral vector-mediated transfection (e.g. lentiviral infection, retroviral infection, adenoviral infection), and other physical, chemical or biological means for transferring into host cell, such as transposon technology, CRISPR-Cas9 and other technologies.

In some embodiments, after step (2), the method further comprises: expanding the immune cell obtained in step (2).

Epitope peptide-based therapy

The epitope peptide of the present invention or APC presenting the epitope peptide can be used in T cell-based immunotherapy to induce an anti-tumor immune response.

Therefore, in the eleventh aspect, the present invention provides a pharmaceutical composition, which comprises: the epitope peptide or variant thereof described in the first aspect, the nucleic acid molecule or vector or host cell comprising a nucleotide sequence encoding the epitope peptide or variant thereof, or the engineered antigen-presenting cell (APC) described in the ninth aspect; and a pharmaceutically acceptable carrier and/or excipient.

In certain embodiments, the pharmaceutical composition is a tumor vaccine.

In certain embodiments, the pharmaceutical composition comprises an adjuvant. The adjuvant is a substance that can enhance the immune response in a non-specific manner, and examples thereof include Freund's complete adjuvant, Freund's incomplete adjuvant, Toll receptor ligand, immunostimulatory antibody (e.g., anti-CD3 antibody, anti-CD28 antibody, anti-CD40L (CD154) antibody, anti-41BB (CD137) antibody, anti-OX40 antibody, anti-GITR antibody, or any combination thereof), or immunostimulatory cytokine (e.g., IL-2, IL-3, IL-12, IL-15, IL-18, IFN-$\gamma$, IL-10, TGF-$\beta$, GM-CSF, or any combination thereof), etc.

In certain embodiments, the pharmaceutical composition further comprises an additional therapeutic agent, such as an antitumor agent or an immunopotentiator.

In certain embodiments, the antitumor agent is selected from the group consisting of alkylating agent, mitotic inhibitor, antineoplastic antibiotic, antimetabolite, topoisomerase inhibitor, tyrosine kinase inhibitor, radionuclide agent, radiation sensitizer (e.g., gemcitabine, 5-fluorouracil, taxane, cisplatin, etc.), antiangiogenic agent, cytokine (e.g., GM-CSF, IL-7, IL-12, IL-15, IL-18, IL-21, etc.), antibody specifically targeting tumor cell (e.g., CD20 antibody, such as rituximab, Her2 antibody such as trastuzumab, VEGF antibody such as bevacizumab, EGFR antibody such as cetuximab etc.), immune checkpoint inhibitor (e.g., PD-1 antibody, PD-L1 antibody, CTLA-4 antibody, LAG-3 antibody, or TIM3 antibody).

In certain embodiments, the immunopotentiator is selected from the group consisting of immunostimulatory antibody (e.g., anti-CD3 antibody, anti-CD28 antibody, anti-CD40L (CD154) antibody, anti-41BB (CD137) antibody, anti-OX40 antibody, anti-GITR antibody or any combination thereof), or immunostimulatory cytokine (e.g., IL-2, IL-3, IL-12, IL-15, IL-18, IFN-$\gamma$, IL-10, TGF-$\beta$, GM-CSF, or any combination thereof).

In certain embodiments, in the pharmaceutical composition, the epitope peptide or variant thereof of the present invention, the engineered APC and the additional therapeutic agent may be provided as separate components or as admixed components.

In a twelfth aspect, the present invention provides a method for inducing an immune response against a tumor with RAS G12V mutation in a subject, and/or preventing or treating a tumor with RAS G12V mutation in a subject, wherein the method comprises administering to the subject in need thereof an effective amount of the epitope peptide or variant thereof described in the first aspect, a nucleic acid molecule or vector or host cell comprising a nucleotide sequence encoding the epitope peptide or variant thereof, or the engineered antigen-presenting cell (APC) described in the ninth aspect, or the pharmaceutical composition described in the eleventh aspect.

In certain embodiments, the tumor having RAS G12V mutation is selected from the group consisting of colorectal cancer, pancreatic cancer, gastric cancer, lung cancer, endometrial cancer, ovarian cancer, multiple myeloma, melanoma, thyroid cancer, bladder cancer, prostate cancer, breast cancer, head and neck cancer, or acute myeloid leukemia.

In certain embodiments, the subject is a human. In certain embodiments, the subject is positive for HLA-DPB1*03:01. In certain embodiments, the subject is further positive for HLA-DPA1*02:02, positive for HLA-DPA1*02:01, or positive for HLA-DPA1*01:03 or positive for HLA-DPB1*14:01. In certain embodiments, the subject has an HLA genotype of HLA-DPB1*03:01/HLA-DPA1*02:02, HLA-DPB1*03:01/HLA-DPA1*02:01, or HLA-DPB1*03:01/HLA-DPA1*01:03, HLA-DPB1*14:01/HLA-DPA1*02:02, HLA-DPB1*14:01/HLA-DPA1*02:01, or HLA-DPB1*14:01/HLA-DPA1*01:03.

In certain embodiments, the epitope peptide or variant thereof described in the first aspect, the nucleic acid molecule or vector or host cell comprising a nucleotide sequence encoding the epitope peptide or variant thereof, or the engineered antigen-presenting cell (APC) described in the ninth aspect, or the pharmaceutical composition described in the eleventh aspect can be administered in combination with an additional therapeutic agent (e.g., an immunopotentiator or an anti-tumor agent). Accordingly, in certain embodiments, the method further comprises administering to the subject an additional therapeutic agent (e.g., an immunopotentiator or an anti-tumor agent), for example, simultaneously, separately, or sequentially.

In certain embodiments, the epitope peptide or variant thereof described in the first aspect, the nucleic acid molecule or vector or host cell comprising a nucleotide sequence encoding the epitope peptide or variant thereof, or the engineered antigen presenting cell (APC) described in the ninth aspect, or the pharmaceutical composition described in the eleventh aspect may be administered in combination with an additional therapy, for example, simultaneously, separately or sequentially. The additional therapy can be any therapy known to be used on tumors, such as surgery, chemotherapy, radiation therapy, targeted therapy, immunotherapy, hormone therapy, gene therapy or palliative care.

The epitope peptide or variant thereof of the present invention, the nucleic acid molecule or vector or host cell comprising a nucleotide sequence encoding the epitope peptide or variant thereof, the engineered antigen-presenting cell (APC), or the pharmaceutical composition containing the same can be formulated into any dosage form known in the medical field, for example, tablet, pill, suspension, emulsion, solution, gel, capsule, powder, granule, elixir, lozenge, suppository, injection (including injection, sterile powder for injection and concentrated solution for injection), inhalant, spray, etc. The preferred dosage form depends on the intended mode of administration and therapeutic use. The medicament of the present invention should be sterile and stable under the conditions of manufacture and storage. A preferred dosage form is injection. Such injection can be a sterile injectable solution. For example, the sterile injectable solution can be prepared by the following method: incorporating the epitope peptide or variant thereof, engineered antigen-presenting cell (APC), or pharmaceutical composition comprising the same of the present invention in an necessary dose into an appropriate solvent, and, optionally, incorporating with other desired ingredients (including but not limited to, pH adjuster, surfactant, adjuvant, ionic strength enhancer, isotonic agent, preservative, diluent, or any combination thereof), then subjecting to filtration and sterilization. In addition, the sterile injectable solution can be prepared as a sterile lyophilized powder (e.g., by vacuum drying or freeze-drying) for ease of storage and use. Such sterile lyophilized powder can be dispersed in a suitable vehicle before use, such as water for injection (WFI), bacteriostatic water for injection (BWFI), sodium chloride solution (e.g., 0.9% (w/v) NaCl), dextrose solution (e.g., 5% dextrose), surfactant-containing solution (e.g., 0.01% polysorbate 20), pH buffered solution (e.g., phosphate buffered saline), Ringer's solution and any combination thereof.

Accordingly, in certain exemplary embodiments, the pharmaceutical composition of the eleventh aspect comprises a sterile injectable liquid (e.g., aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% dextrose), surfactant-containing solution (e.g., 0.01% polysorbate 20), pH buffered solution (e.g., phosphate buffered saline), Ringer's solution, and any combination thereof.

The epitope peptide or variant thereof, nucleic acid molecule or vector or host cell comprising a nucleotide sequence encoding the epitope peptide or variant thereof, engineered antigen-presenting cell (APCs), or pharmaceutical composition comprising the same of the present invention may be administered by any suitable method known in the art, including, but not limited to, oral, buccal, sublingual, ocular, local, parenteral, rectal, intrathecal, intra-cisterna, inguinal, intravesical, topical (e.g., powder, ointment, or drops), or nasal route. However, for many therapeutic uses, the preferred route/mode of administration is parenteral (e.g., intravenous or bolus injection, subcutaneous injection, intraperitoneal injection, intramuscular injection). The skilled artisan will understand that the route and/or mode of administration will vary depending on the intended purpose. In certain embodiments, the epitope peptide or variant thereof, nucleic acid molecule or vector or host cell comprising a nucleotide sequence encoding the epitope peptide or variant thereof, engineered antigen-presenting cell (APC), or pharmaceutical compositions containing the same of the present invention are administrated by intravenous injection or bolus injection.

The pharmaceutical composition described in the eleventh aspect may comprises a "therapeutically effective amount" or "prophylactically effective amount" of the epitope peptide or variant thereof, nucleic acid molecule or vector or host cell comprising a nucleotide sequence encoding the epitope peptide or variant thereof, or engineered antigen-presenting cell (APC) of the present invention. In this context, the "therapeutically effective amount" refers to an amount capable of generating an immune response in a treated subject, in which the immune response is capable of reducing or inhibiting proliferation of tumor cells and/or eliminating tumor cells. The "prophylactically effective amount" refers to an amount capable of generating an immune response against a target cell (e.g., a tumor cell with RAS mutation) in a treated subject, in which the immune response is capable of preventing the formation of tumors in the subject, or capable of substantially reducing the chance of developing a tumor or continuing to develop a tumor in the subject.

In another aspect, the present invention provides a use of the epitope peptide or variant thereof described in the first aspect, a nucleic acid molecule or vector or host cell comprising a nucleotide sequence encoding the epitope peptide or variant thereof, or the engineered antigen-presenting cell (APC) described in the ninth aspect, or the pharmaceutical composition described in the eleventh aspect, in the manufacture of a medicament, and the medicament is used for inducing an immune response against a tumor with RAS G12V mutation in a subject and/or preventing or treating a tumor with RAS G12V mutation in a subject.

TCR-based therapy

The TCR of the present invention or the immune cell expressing the TCR can be used in T cell-based immunotherapy to kill tumors with RAS G12V mutation.

Therefore, in the thirteenth aspect, the present invention provides a pharmaceutical composition, which comprises: the TCR or antigen-binding fragment thereof described in the second or third aspect, the conjugate described in the fourth aspect, or the fusion protein described in the fifth aspect, the nucleic acid molecule or vector or host cell comprising a nucleotide sequence encoding the TCR or antigen-binding fragment thereof or the conjugate or the fusion protein, or the engineered immune cell described in the tenth aspect; and a pharmaceutically acceptable carrier and/or excipient.

In certain embodiments, the pharmaceutical composition further comprises an additional therapeutic agent, such as an antitumor agent or an immunopotentiator.

In certain embodiments, the antitumor agent is selected from the group consisting of alkylating agent, mitotic inhibitor, antineoplastic antibiotic, antimetabolite, topoisomerase inhibitor, tyrosine kinase inhibitor, radionuclide agent, radiation sensitizer (e.g., gemcitabine, 5-fluorouracil, taxane, cisplatin, etc.), antiangiogenic agent, cytokine (e.g., GM-CSF, IL-7, IL-12, IL-15, IL-18, IL-21, etc.), antibody specifically targeting tumor cell (e.g., CD20 antibody such as rituximab, Her2 antibody such as trastuzumab, VEGF antibody such as bevacizumab, EGFR antibody such as cetuximab etc.), immune checkpoint inhibitor (e.g., PD-1 antibody, PD-L1 antibody, CTLA-4 antibody, LAG-3 antibody, or TIM3 antibody).

In certain embodiments, the immunopotentiator is selected from the group consisting of immunostimulatory antibody (e.g., anti-CD3 antibody, anti-CD28 antibody, anti-CD40L (CD154) antibody, anti-41BB (CD137) antibody, anti-OX40 antibody, anti-GITR antibody or any combination thereof) or immunostimulatory cytokine (e.g., IL-2, IL-3, IL-12, IL-15, IL-18, IFN-γ, IL-10, TGF-β, GM-CSF, or any combination thereof).

In certain embodiments, the TCR or antigen-binding fragment thereof, conjugate, fusion protein or engineered immune cell of the present invention and the additional therapeutic agent may be used as separate components or admixed components in the pharmaceutical composition.

In a fourteenth aspect, the present invention provides a method for inducing an immune response against a tumor with RAS G12V mutation in a subject, and/or preventing or treating a tumor with RAS G12V mutation in a subject, wherein the method comprises administering to the subject in need thereof an effective amount of the TCR or antigen-binding fragment thereof of the second or third aspect, the conjugate of the fourth aspect, or the fusion protein of the fifth aspect, a nucleic acid molecule or vector or host cell comprising a nucleotide sequence encoding the TCR or antigen-binding fragment thereof or the conjugate or the fusion protein, or the engineered immune cell of the tenth aspect, or the pharmaceutical composition of the thirteenth aspect.

In certain embodiments, the tumor with RAS G12V mutation is selected from the group consisting of colorectal cancer, pancreatic cancer, gastric cancer, lung cancer, endometrial cancer, ovarian cancer, multiple myeloma, melanoma, thyroid cancer, bladder cancer, prostate cancer, breast cancer, head and neck cancer, or acute myeloid leukemia.

In certain embodiments, the subject is a human. In certain embodiments, the subject is a subject positive for HLA-DPB1*0301 or positive for HLA-DPB1*14:01. In certain embodiments, the subject is further positive for HLA-DPA1*02:02, positive for HLA-DPA1*02:01, or positive for HLA-DPA1*01:03. In certain embodiments, the subject has an HLA genotype of HLA-DPB1*03:01/HLA-DPA1*02:02, HLA-DPB1*03:01/HLA-DPA1*02:01, or HLA-DPB1*03:01/HLA-DPA1*01:03, HLA-DPB1*14:01/HLA-DPA1*02:02, HLA-DPB1*14:01/HLA-DPA1*02:01, or HLA-DPB1*14:01/HLA-DPA1*01:03.

In certain embodiments, the method comprises: (1) providing an immune cell required by the subject; (2) introducing a nucleotide sequence encoding the TCR or antigen-binding fragment thereof described in the second or third aspect into the immune cell described in step (1) to obtain an immune cell expressing on its surface the TCR or antigen-binding fragment thereof; and (3) administering the immune cell obtained in step (2) to the subject.

In certain embodiments, prior to step (1), the method further comprises a step of obtaining an immune cell from the subject. The immune cell may be isolated or obtained from any tissue (e.g., peripheral blood mononuclear cells, bone marrow, lymph node tissue, umbilical cord blood, thymus tissue, ascites, pleural effusion, spleen tissue, and tumor) in which such cell is found, or may be otherwise cultured and provided, for example, obtained through induction and differentiation from a precursor cell of the immune cell (e.g., a precursor of T lymphocyte).

In certain embodiments, the immune cell is selected from lymphocytes. In certain embodiments, the immune cell is selected from the group consisting of T cell (e.g., αβ T cell, γδ T cell, or iPSC-derived T cell), tumor infiltrating lymphocyte (TIL), natural killer (NK) cell, natural killer T (NKT) cell, or any combination thereof. In certain embodiments, the immune cell comprises a CD4+ T cell.

In certain exemplary embodiments, the peripheral blood mononuclear cell (PBMC) and/or TIL are obtained from the subject and directly genetically modified to express the TCR.

In certain exemplary embodiments, the T cell is obtained from the subject and genetically modified to express the TCR. T cell can be obtained from a variety of sources, for example T cell can be obtained from a blood unit collected from the subject using various techniques (e.g., sedimentation, for example Ficoll™ isolation) known to the skilled person. In one embodiment, the cells from individual circulating blood are obtained by apheresis. The product of apheresis usually contains lymphocytes, including T cells, monocytes, granulocytes, B cells, other nucleated white blood cells, red blood cells, and platelets. In one embodiment, cells collected by apheresis can be washed to remove the plasma fraction and the cells are placed in a suitable buffer or medium for subsequent processing. As understood by those of ordinary skill in the art, the washing step can be accomplished by methods known to those of skill in the art, such as by using a semiautomatic flow-through centrifuge. After washing, the cells can be resuspended in a variety of biocompatible buffers or other saline solutions with or without buffers. In certain embodiments, unwanted components of an apheresis sample can be removed from the medium in which the cells are directly resuspended. In certain embodiments, T cells are isolated from peripheral blood mononuclear cells (PBMCs) by lysing red blood cells and depleting monocytes (e.g., by Percoll™ gradient centrifugation). Specific T cell subpopulations expressing one or more of the following markers CD3, CD28, CD4, CD8, CD45RA and CD45RO, can be further isolated by positive or negative selection techniques. In one embodiment, specific T cell subpopulations expressing CD3, CD28, CD4, CD8, CD45RA and CD45RO are further isolated by positive or negative selection techniques. Enrichment of a T cell population by negative selection can be accomplished, for example, with an antibody combination against surface markers specific to the negatively selected cells. An exemplary method is cell sorting and/or selection via negative magnetic immunoadhesion or flow cytometry, in which the negative magnetic immunoadhesion or flow cytometry utilizes a mixture of monoclonal antibodies against to the cell surface markers present on the negatively selected cells. For example, to enrich for CD4+ cells by negative selection, a mixture of monoclonal antibodies typically contains antibodies against CD14, CD20, CD11b, CD16, HLA-DR, and CD8. Flow cytometry and cell sorting can also be used to isolate a cell population of interest used in the present invention.

In certain embodiments, the immune cell (e.g., T cell) can be activated and expanded (or in the case of progenitor cell, differentiated) in vitro prior to the genetic modification of the immune cell.

In some embodiments, the TCR or antigen-binding fragment thereof of the second or third aspect, the conjugate of the fourth aspect, or the fusion protein of the fifth aspect, the nucleic acid molecule or vector or host cell comprising a nucleotide sequence encoding the TCR or antigen-binding fragment thereof or the conjugate or the fusion protein, or the engineered immune cell of the tenth aspect, or the pharmaceutical composition of the thirteenth aspect, can be administrated in combination with an additional therapeutic agent (e.g., an immunopotentiator or an antitumor agent). Accordingly, in certain embodiments, the method further comprises administering to the subject an additional therapeutic agent (e.g., an immunopotentiator or an antitumor agent), for example, simultaneously, separately, or sequentially.

In some embodiments, the TCR or antigen-binding fragment thereof of the second or third aspect, the conjugate of the fourth aspect, or the fusion protein of the fifth aspect, the nucleic acid molecule or vector or host cell comprising a nucleotide sequence encoding the TCR or antigen-binding fragment thereof or the conjugate or the fusion protein, or the engineered immune cell of the tenth aspect, or the pharmaceutical composition of the thirteenth aspect can be administrated in combination with an additional therapy, for example, simultaneously, separately or sequentially. The additional therapy can be any therapy known to be used on tumors, such as surgery, chemotherapy, radiation therapy, targeted therapy, immunotherapy, hormone therapy, gene therapy or palliative therapy.

The TCR or its antigen-binding fragment, conjugate, fusion protein, nucleic acid molecule or vector or host cell comprising a nucleotide sequence encoding the TCR or antigen-binding fragment thereof or the conjugate or the fusion protein, engineered immune cell, or pharmaceutical composition comprising the same of the present invention, can be formulated into any dosage form known in the medical field, for example, tablet, pill, suspension, emulsion, solution, gel, capsule, powder, granule, elixir, lozenge, suppository, injection (including injection, sterile powder for injection and concentrated solution for injection), inhalant, spray, etc. The preferred dosage form depends on the intended mode of administration and therapeutic use. The medicament of the present invention should be sterile and stable under the conditions of manufacture and storage. A preferred dosage form is injection. Such injection can be a sterile injectable solution. For example, the sterile injectable solution can be prepared by the following method: incorporating the TCR or antigen-binding fragment thereof, conjugate, fusion protein, engineered immune cell, or pharmaceutical composition comprising the same of the present invention in an necessary dose into an appropriate solvent, and, optionally, incorporating simultaneously other desired ingredients (including but not limited to, pH adjuster, surfactant, adjuvant, ionic strength enhancer, isotonic agent, preservative, diluent, or any combination thereof), then undergoing filtration and sterilization. In addition, the sterile injectable solution can be prepared as a sterile lyophilized powder (e.g., by vacuum drying or freeze-drying) for ease of storage and use. Such sterile lyophilized powder can be dispersed in a suitable vehicle before use, such as water for injection (WFI), bacteriostatic water for injection (BWFI), sodium chloride solution (e.g., 0.9% (w/v) NaCl), dextrose solution (e.g., 5% dextrose), surfactant-containing solution (e.g., 0.01% polysorbate 20), pH buffered solution (e.g., phosphate buffered saline), Ringer's solution and any combination thereof.

Accordingly, in certain exemplary embodiments, the pharmaceutical composition of the thirteenth aspect comprises a sterile injectable liquid (e.g., aqueous or non-aqueous suspension or solution). In certain exemplary embodiments, such sterile injectable liquid is selected from the group consisting of water for injection (WFI), bacteriostatic water for injection (BWFI), sodium chloride solution (e.g. 0.9% (w/v) NaCl), dextrose solution (e.g. 5% dextrose), surfactant-containing solution (e.g. 0.01% polysorbate 20), pH buffered solution (e.g., phosphate buffered solution), Ringer's solution and any combination thereof.

The TCR or antigen-binding fragment thereof, conjugate, fusion protein, nucleic acid molecule or vector or host cell comprising a nucleotide sequence encoding the TCR or antigen-binding fragment thereof or the conjugate or the fusion protein, engineered immune cell, or pharmaceutical composition comprising the same of the present invention, can be administered by any suitable method known in the art, including, but not limited to, oral, buccal, sublingual, ocular, local, parenteral, rectal, intrathecal, intra-cisterna, inguinal, intravesical, topical (e.g., powder, ointment, or drops), or nasal route. However, for many therapeutic uses, the preferred route/mode of administration is parenteral (e.g., intravenous or bolus injection, subcutaneous injection, intraperitoneal injection, intramuscular injection). The skilled artisan will understand that the route and/or mode of administration will vary depending on the intended purpose. In certain embodiments, the TCR or antigen-binding fragment thereof, conjugate, fusion protein, nucleic acid molecule or vector or host cell comprising a nucleotide sequence encoding the TCR or antigen-binding fragment thereof or the conjugate or the fusion protein, engineered immune cell, or pharmaceutical composition comprising the same of the present invention is administrated by intravenous injection or bolus injection.

The pharmaceutical composition described in the thirteenth aspect may comprises a "therapeutically effective amount" or "prophylactically effective amount" of the TCR or antigen-binding fragment thereof, conjugate, fusion protein, nucleic acid molecule or vector or host cell comprising a nucleotide sequence encoding the TCR or antigen-binding fragment thereof or the conjugate or the fusion protein, engineered immune cell, or pharmaceutical composition comprising the same of the present invention. In this context, the "therapeutically effective amount" refers to an amount capable of generating an immune response in a treated subject, in which the immune response is capable of reducing or inhibiting proliferation of tumor cells and/or eliminating tumor cells. The "prophylactically effective amount" refers to an amount capable of generating an immune response against a target cell (e.g., a tumor cell with RAS mutation) in a treated subject, in which the immune response is capable of preventing the formation of tumors in the subject, or capable of substantially reducing the chance of developing a tumor or continuing to develop a tumor in the subject.

In another aspect, the present invention provides a use of the TCR or antigen-binding fragment thereof described in the second or third aspect, the conjugate described in the fourth aspect, the fusion protein described in the fifth aspect, the nucleic acid molecule or vector or host cell comprising a nucleotide sequence encoding the TCR or antigen-binding fragment thereof or the conjugate or the fusion protein, or the engineered immune cell described in the tenth aspect, or the pharmaceutical composition described in the thirteenth aspect, in the manufacture of a medicament for inducing an immune response against a tumor with RAS G12V mutation in a subject, and/or preventing or treating a tumor with RAS G12V mutation in a subject.

Definition of terms

In the present invention, unless otherwise specified, the scientific and technical terms used herein have the meanings commonly understood by those skilled in the art. Moreover, the procedures of immunology, molecular biology, biochemistry, nucleic acid chemistry, cell culture, etc. used herein are all routine steps widely used in the corresponding fields. Meanwhile, in order to better understand the present invention, definitions and explanations of related terms are provided below.

As used herein, the term "RAS" refers to a proto-oncogene, the RAS protein it encoded has GTPase activity and participates in many signaling pathways, such as MAPK, PI3K, STAT signaling pathway, etc., that regulate cell proliferation, differentiation and apoptosis. There are three RAS genes in human genes, namely HRAS (GeneID: 3265), NRAS (GeneID: 4893) and KRAS (GeneID: 3845), and the three RAS genes have a high degree of sequence homology (>90%). RAS gene mutation is a driving factor of cancer, and RAS gene mutation is the most frequent proto-oncogene mutation. The sequences of RAS proteins encoded by the RAS genes are well known to those skilled in the art and can be found in various public databases. For example, the sequence of KRAS protein can be found in NCBI: NP_001356715.1, the sequence of NRAS protein can be found in NCBI: NP_002515.1, and the sequence of HRAS protein can be found in NCBI: NP_001123914.1.

As used herein, the term "RAS G12V mutant" refers to a RAS mutant in which amino acid residue Gly at position 12 is mutated to Val. In some embodiments, the RAS G12V mutant refers to KRAS G12V mutant. Herein, when referring to the amino acid sequence of RAS G12V mutant, it is described with reference to the sequence set forth in SEQ ID NO: 1. For example, the expression "amino acid residues at the positions 3-13 of the RAS G12V mutant protein" refers to the amino acid residues at the positions 3-13 of the sequence set forth in SEQ ID NO: 1, or the corresponding fragment of other RAS G12V mutant amino acid sequences. "Corresponding fragment" refers to a fragment located in equivalent position of the sequences being compared when the sequences are optimally aligned, i.e., when the sequences are aligned to obtain the highest percent identity.

As used herein, the terms "major histocompatibility complex" and "MHC" are used interchangeably, which refer to a group of genes that determine whether transplanted tissues are identical, are closely related to immune responses, and are closely related, mainly including MHC-I molecules and MHC-II molecules. "MHC-I molecule" refers to a dimer of MHC type I $\alpha$-chain and $\beta$2 microglobulin chain, and "MHC-II molecule" refers to a dimer of MHC type II $\alpha$-chain and MHC type II $\beta$-chain. The human MHC is called as human leukocyte antigen (HLA) complex.

As used herein, the term "MHC-peptide complex" refers to an MHC molecule (MHC type I or MHC type II) comprising a peptide bound in the MHC peptide-binding pocket well known in the art. In some cases, the MHC molecule can be a membrane-bound protein expressed on the cell surface. In other cases, the MHC molecule can be a soluble protein that lacks a transmembrane or cytoplasmic region.

As used herein, the term "epitope" in reference to TCR refers to a localized region of an antigen (e.g., a peptide or peptide-MHC complex) to which the TCR can bind. In certain embodiments, a TCR-bound epitope can be determined by, for example, NMR spectroscopy, X-ray diffraction crystallographic study, ELISA assay, hydrogen/deuterium exchange mass spectrometry (e.g., liquid chromatography electrospray mass spectrometry), flow cytometry, mutagenesis mapping (e.g., site-directed mutagenesis mapping) and/or structural modeling. In some exemplary embodiments, the antigen epitope is determined using alanine scanning mutagenesis study. In certain embodiments, the antigen is a peptide-MHC complex or a peptide presented by an MHC molecule.

As used herein, the terms "T cell receptor" and "TCR" are used interchangeably and refer to a molecule comprising CDR or variable region from an $\alpha\beta$ or $\gamma\delta$ T cell receptor. Examples of TCR include, but are not limited to, full-length TCR, antigen-binding fragment of TCR, soluble TCR lacking transmembrane and cytoplasmic region, single-chain TCR containing TCR variable regions attached by a flexible linker, TCR chain linked via engineered disulfide bond, etc.

As used herein, the term "full-length TCR" refers to a TCR comprising a dimer of a first polypeptide chain and a second polypeptide chain, each of the polypeptide chains comprises a TCR variable region and a TCR constant region comprising a TCR transmembrane region and a TCR cytoplasmic region. In certain embodiments, the full-length TCR comprises a mature full-length TCR $\alpha$-chain and a mature full-length TCR $\beta$-chain. In certain embodiments, the full-length TCR comprises a mature full-length TCR $\gamma$ chain and a mature full-length TCR $\delta$ chain.

As used herein, the term "TCR variable region" refers to a portion of a mature TCR polypeptide chain (e.g., TCR $\alpha$-chain or $\beta$-chain), and the portion is not encoded by the TRAC gene of TCR $\alpha$-chain, the TRBC1 gene or TRBC2 gene of TCR $\beta$-chain, the TRDC gene of TCR $\delta$ chain, or the TRGC1 gene or TRGC2 gene of TCR $\gamma$ chain. In certain embodiments, the TCR variable region of TCR $\alpha$-chain encompasses all amino acids of the mature TCR $\alpha$-chain polypeptide encoded by the TRAV gene and/or TRAJ gene, and the TCR variable region of the TCR $\beta$-chain encompasses all amino acids of the mature TCR$\beta$-chain polypeptide encoded by the TRBV gene, TRBD gene and/or TRBJ gene (see, for example, "T cell receptor Facts book", (2001), LeFranc and LeFranc, Academic Press, ISBN0-12-441352-8, which is incorporated herein by reference in its entirety). The TCR variable region typically comprises framework regions (FRs) 1, 2, 3 and 4 and complementarity determining regions (CDRs) 1, 2 and 3. Herein, the terms "$\alpha$-chain variable region" and "V$\alpha$" are used interchangeably and refer to the variable region of TCR$\alpha$-chain. The terms "$\beta$-chain variable region" and "V$\beta$" are used interchangeably and refer to the variable region of TCR $\beta$-chain.

As used herein, the term "CDRs" or "complementarity determining regions" in reference to a TCR refers to the non-contiguous antigen-binding sites found within the variable regions of a TCR chain (e.g., $\alpha$-chain or $\beta$-chain). These regions have been described in Lefranc, (1999) "The Immunologist" 7:132-136; Lefranc et al., (1999) "Nucleic Acids Res" 27:209-212; LeFranc (2001) "T cell receptor facts book", Academic Press, ISBN 0-12-441352-8; Lefranc et al., (2003) Dev Comp Immunol 27(1):55-77; and Kabat et al., (1991) Sequences of proteins of immunological interest, each of which is hereby incorporated by reference in its entirety. In certain embodiments, the CDRs are defined according to the IMGT numbering system described in Lefranc (1999), supra. In certain embodiments, the CDRs are defined according to the Kabat numbering system described in Kabat, supra.

As used herein, the term "FR" or "framework region" in reference to a TCR refers to those amino acid residues in the variable region of TCR chain (e.g., $\alpha$-chain or $\beta$-chain) other than the CDRs as defined above.

As used herein, the term "constant region" in reference to a TCR refers to a portion of TCR that is encoded by the TRAC gene (for TCR $\alpha$-chain), the TRBC1 or TRBC2 gene (for TCR $\beta$-chain), the TRDC gene (for TCR $\delta$ chain), or the TRGC1 or TRGC2 gene (for TCR $\gamma$ chain), optionally lacking all or a portion of the transmembrane region and/or all or a portion of the cytoplasmic region. In certain embodiments, the TCR constant region lacks transmembrane and cytoplasmic regions. The TCR constant region does not contain amino acids encoded by the TRAV, TRAJ, TRBV, TRBD, TRBJ, TRDV, TRDD, TRDJ, TRGV, or TRGJ genes (see, for example, T cell receptor facts book, (2001), LeFranc and LeFranc, Academic Press, ISBN0-12-441352-8, which is hereby incorporated by reference in its entirety).

In the context of TCR, the term "extracellular" refers to one or more portions of a TCR chain located outside the cell, "transmembrane" refers to one or more portions of a TCR chain embedded in the membrane of the cell, and "cytoplasmic " refers to one or more portions of a TCR chain located in the cytoplasm of the cell.

As used herein, the term "antigen-binding portion" in reference to a TCR refers to any portion or fragment of a TCR which retains the biological activity of the TCR (parental TCR). The biological activity may include the ability to specifically bind to the same antigen (e.g., RAS G12V mutant) or MHC-antigen complex to which the parental TCR binds.

As used herein, the term "specific binding" refers to a non-random binding reaction between two molecules, such as the reaction between an antibody and an antigen to which it directs. The strength or affinity of a specific binding interaction can be expressed in terms of the equilibrium dissociation constant ($K_D$) for that interaction. In the present invention, the term "$K_D$" refers to a dissociation equilibrium constant of a specific antibody-antigen interaction, which is used to describe the binding affinity between the antibody and the antigen. The smaller the equilibrium dissociation constant, the tighter the antibody-antigen binding, and the higher the affinity between the antibody and the antigen. The specific binding properties between two molecules can be determined using methods well known in the art. One method involves measuring the formation and dissociation rates of the antigen binding site/antigen complex. Both "association rate constant" (ka or kon) and "dissociation rate constant" (kdis or koff) can be calculated from concentrations and actual rates of association and dissociation (see, Malmqvist M, Nature, 1993, 361:186-187). The ratio of kdis/kon is equal to the dissociation constant $K_D$ (see, Davies et al., Annual Rev Biochem, 1990; 59:439-473). $K_D$, kon and kdis values can be measured by any effective method, for example, measured by surface plasmon resonance (SPR) in Biacore, or measured by bioluminescence interferometry or Kinexa.

In the context of TCR, the term "specific binding" or "specific recognition" refers to the ability of a TCR to preferentially bind to a specific antigen (e.g., a specific peptide or a specific peptide-MHC complex). Typically, a TCR that specifically binds to an antigen does not bind or with lower affinity binds to other antigens. For example, an antigen-specific TCR binds to the target antigen with an association constant (Ka) of at least 2-fold, 5-fold, 10-fold, 50-fold, 100-fold, 500-fold, 1,000-fold, 5,000-fold, or 10,000-fold higher than that of other antigens that are not specifically bound by the TCR. In certain embodiments, the TCR or antigen-binding fragment thereof disclosed herein, specifically binds to RAS G12V mutant. In certain embodiments, the TCR or antigen-binding fragment thereof disclosed herein specifically binds to the epitope peptide or variant thereof of the first aspect. In certain embodiments, the TCR or antigen-binding fragment thereof disclosed herein specifically binds to the sequence set forth in any one of SEQ ID NOs: 2-5 (especially SEQ ID NO: 5).

As used herein, the term "antigen presenting cell" or "APC" refers to any cell capable of presenting on its cell surface a peptide fragment of a protein associated with a major histocompatibility complex (MHC) molecule. Such cell is well known to those skilled in the art, including but not limited to dendritic cell, monocyte, macrophage, B lymphocyte (e.g., B-lymphoblastoid cell B-LCL) and the like.

As used herein, the term "immune cell" refers to any cell of the immune system that has one or more effector functions. Immune cells typically include cells that play a role in the immune response, and they usually are of hematopoietic origin. The term "effector function" refers to a specialized function of an immune cell, such as a function or response that enhances or promotes an immune attack on a target cell (e.g., killing of target cell, or inhibiting its growth or proliferation). For example, an effector function of a T cell, for example, may be a cytolytic activity or an activity of helping or including secretion of cytokines. Examples of immune cell include T cell (e.g., α/β T cell and γ/δ T cell), B cell, natural killer (NK) cell, natural killer T (NKT) cell, mast cell, and bone marrow-derived macrophage, etc.

The immune cell of the present invention may be an autologous/autogenous ("self") or non-autologous ("non-self", for example, allogeneic, syngeneic or heteroallelic) cell. As used herein, "autologous" cell refers to a cell from the same subject; "allogeneic" cell refers to a cell of the same species that is genetically different from the cell being compared; "syngeneic" cell refers to a cell from a different subject that is genetically identical to the cell being compared; "heteroallelic" cell refers to a cell from a different species than the cell being compared. In certain embodiments, the immune cell of the present invention is an autologous or allogeneic cell.

As used herein, the term "cytotoxic agent" comprises any agent that is detrimental to (e.g., kills) a cell, and examples thereof include chemotherapeutic drug, bacterial toxin, plant toxin, or radioactive isotope, etc.

As used herein, the term "vector" refers to a nucleic acid delivery vehicle into which a polynucleotide can be inserted. When the vector is capable of achieving expression of the protein encoded by the inserted polynucleotide, the vector is called an expression vector. A vector can be introduced into a host cell by transformation, transduction or transfection, so that a genetic material element it carries can be expressed in the host cell. Vectors are well known to those skilled in the art, including but not limited to: plasmid; phagemid; cosmid; artificial chromosome, such as yeast artificial chromosome (YAC), bacterial artificial chromosome (BAC) or P1-derived artificial chromosome (PAC); phage, such as λ phage or M13 phage, and animal virus. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector can contain a variety of elements that control expression, including but not limited to, promoter sequence, transcription initiation sequence, enhancer sequence, selection element, and reporter gene. In addition, the vector may also contain an origin of replication.

As used herein, the term "host cell" refers to a cell into which a vector can be introduced, including but not limited to, prokaryotic cell such as *Escherichia coli* or *Bacillus subtilis*, fungal cell such as yeast cell or *Aspergillus*, insect cell such as *S2 Drosophila* cell or Sf9, or animal cell such as fibroblast, CHO cell, COS cell, NSO cell, HeLa cell, BHK cell, HEK 293 cell or human cell, immune cell (e.g., T lymphocyte, NK cell, monocyte, macrophage or dendritic cell, etc.). A host cell may comprise a single cell or a cell population.

As used herein, the term "isolated" means that it has been separated or purified from components (e.g., nucleic acid, protein, or other naturally occurring biological or organic molecules) that naturally accompany it.

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison $\times$ 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

As used herein, the terms "conservative substitution" and "conservative amino acid substitution" refer to amino acid substitutions which would not disadvantageously affect or change the expected properties of a protein/polypeptide comprising the amino acid sequence. For example, a conservative substitution may be introduced by standard techniques known in the art such as site-directed mutagenesis and PCR-mediated mutagenesis. Conservative amino acid substitutions include substitutions wherein an amino acid residue is substituted with another amino acid residue having a similar side chain, for example, a residue physically or functionally similar (such as, having similar size, shape, charge, chemical property including the capability of forming covalent bond or hydrogen bond, etc.) to the corresponding amino acid residue. The families of amino acid residues having similar side chains have been defined in the art. These families include amino acids having basic side chains (for example, lysine, arginine and histidine), amino acids having acidic side chains (for example, aspartic acid and glutamic acid), amino acids having uncharged polar side chains (for example, glycine, asparagine, glutamine, serine, threonine, tyrosine, cysteine, tryptophan), amino acids having nonpolar side chains (for example, alanine, valine, leucine, isoleucine, proline, phenylalanine, methionine), amino acids having β-branched side chains (such as threonine, valine, isoleucine) and amino acids having aromatic side chains (for example, tyrosine, phenylalanine, tryptophan, histidine). Therefore, a corresponding amino acid residue is preferably substituted with another amino acid residue from the same side-chain family. Methods for identifying amino acid conservative substitutions are well known in the art (see, for example, Brummell et al., Biochem. 32: 1180-1187 (1993); Kobayashi et al., Protein Eng. 12(10): 879-884 (1999); and Burks et al., Proc. Natl Acad. Set USA 94: 412-417 (1997), which are incorporated herein by reference).

The writing of the twenty conventional amino acids referred to herein follows conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And, in the present invention, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient pharmacologically and/or physiologically compatible with the subject and the active ingredient, which are well known in the art (see, for example, Remington's Pharmaceutical Sciences. Edited by Gennaro AR, 19th ed. Pennsylvania: Mack Publishing Company, 1995), and include, but are not limited to: pH adjusting agent, surfactant, adjuvant, ionic strength enhancer, diluent, agent for maintaining osmotic pressure, agent for delaying absorption, preservative. For example, the pH adjusting agent includes, but is not limited to, phosphate buffer. The surfactant includes but is not limited to cationic, anionic or nonionic surfactant, such as Tween-80. The ionic strength enhancer includes, but is not limited to, sodium chloride. The preservative includes, but is not limited to, various antibacterial and antifungal agents, such as paraben, chlorobutanol, phenol, sorbic acid, and the like. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The preservative includes, but is not limited to, various antibacterial and antifungal agent, such as thimerosal, 2-phenoxyethanol, paraben, chlorobutanol, phenol, sorbic acid, and the like. The stabilizer has the meaning generally understood by those skilled in the art, and it can stabilize the desired activity of the active ingredient in the medicine, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dry whey, albumin or casein) or degradation product thereof (e.g., lactalbumin hydrolyzate), etc. In certain exemplary embodiments, the pharmaceutically acceptable carrier or excipient comprises a sterile injectable liquid (e.g., aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% dextrose), surfactant-containing solution (e.g., 0.01% polysorbate 20), pH buffered solution (e.g., phosphate buffered saline), Ringer's solution, and any combination thereof.

As used herein, the term "prevention" refers to methods performed to prevent or delay the occurrence of a disease or disorder or symptom (e.g., a tumor) in a subject. As used herein, the term "treatment" refers to a method performed to obtain a beneficial or desired clinical result. For the purposes of this invention, a beneficial or desired clinical result comprises, but is not limited to, alleviation of symptom, reduction of disease extent, stabilization (i.e., no longer worsening) of disease state, delay or slowing disease progression, amelioration or palliation of disease status, and relief of symptom (whether partial or total), whether detectable or undetectable. Additionally, "treatment" can also refer to prolonging survival as compared to expected survival (if not receiving such treatment).

As used herein, the term "effective amount" refers to an amount sufficient to achieve, or at least partially achieve, the desired effect. For example, an effective amount for preventing a disease (e.g., a tumor) refers to an amount sufficient to prevent, arrest, or delay the occurrence of a disease (e.g., a tumor); an effective amount for treating a disease refers to an amount sufficient to cure or at least partially prevent an existing disease or complication thereof in a patient who has suffered from the disease. Determining such an effective amount is well within the capability of those skilled in the art. For example, an effective amount for therapeutic use will depend on the severity of the disease to be treated, the general state of the patient's own immune system, the general condition of the patient such as age, weight and sex, the mode of administration of the drug, and other therapy administered concomitantly, etc.

As used herein, the term "subject" refers to a mammal, such as a primate mammal, such as a human. In certain embodiments, the term "subject" is meant to include living organisms in which an immune response can be elicited. In certain embodiments, the subject (e.g., a human) has RAS G12V mutation-positive tumor, or is at risk of suffering from the above-mentioned diseases.

Beneficial effects of the present invention

The present invention provides an epitope peptide of RAS G12V mutant, a T cell receptor (TCR) capable of specifically recognizing the epitope peptide, a cell and pharmaceutical composition comprising the epitope peptide or TCR, and a nucleic acid encoding the epitope peptide or TCR, a vector and host cell for preparing the epitope peptide or TCR, and a method of using the epitope peptide or TCR to treat a subject. The epitope peptide and TCR provided by the present invention are capable of inducing an immune response against a tumor with RAS G12V mutation and thereby treating the above-mentioned tumor in a subject. In addition, the epitope peptide and TCR provided by the present invention are MHC-II-restricted, and the MHC-II restriction refers to an allele showing predominantly high frequency in the Asia-Pacific populations, so it is especially suitable for patients in the Asia-Pacific region. Therefore, the present invention provides a novel T cell-based immunotherapy for the treatment of RAS G12V mutation-positive tumor, and thus has great clinical value.

The embodiments of the present invention will be described in detail below with reference to the drawings and examples, but those skilled in the art will understand that the following drawings and examples are only for illustrating the present invention, rather than limiting the scope of the present invention. Various objects and advantages of the present invention will become apparent to those skilled in the art from the accompanying drawings and the following detailed description of preferred embodiments.

**Brief Description of the Drawings**

FIG. 1 shows the results of IFNγ-specific release against different LCL clones in Example 5.

FIG. 2 shows the results of IFNγ release after co-culturing the antigen-presenting cells loaded with different peptides and B13.14.1 TCR-T cells in Example 6.

FIG. 3 shows the results of IFNγ release assay of B13.14.1 TCR-T induced by different G12V peptide segments in Example 7.

FIG. 4 shows the results of IFNγ release assay of B13.14.1 TCR-T induced by mutation-containing G12V peptide segment in Example 8.

FIG. 5 shows the results of selective killing of SW620-CIITA-DPA02:02/DPB03:01 cells by B13.14.1 TCR-T in Example 9.

FIGS. 6A to 6B show the results of affinity assay of B13.14.1 TCR-T to the RAS G12V mutant in Example 10.

FIG. 7 shows the results of in vivo efficacy assay of B13.14.1 TCR-T in Example 11.

FIG. 8 shows the determination results of the binding activity of TCR mutant (CDR3 region) to KRAS-G12V antigen peptide in Example 12.

FIG. 9 shows the determination results of the binding activity of TCR mutant (MHC-II binding region) to KRAS-G12V antigen peptide in Example 12.

## Sequence information

Information on the partial sequences involved in the present invention is provided in the table below.

| SEQ ID NO | Description | Sequence information |
|---|---|---|
| 1 | RAS G12V mutant amino acid sequence | MTEYKLVVVGAVGVGKSALTIQLIQNHFVDEYDPTIEDSY RKQVVIDGETCLLDILDTAGQEEYSAMRDQYMRTGEGFLC VFAINNTKSFEDIHHYREQIKRVKDSEDVPMVLVGNKCDL PSRTVDTKQAQDLARSYGIPFIETSAKTRQGVDDAFYTLVR EIRKHKEKMSKDGKKKKKKSKTKCVIM |
| 2 | RAS G12V mutant amino acid sequence aa3-13/G12V-T9 | EYKLVVVGAVG |
| 3 | RAS G12V mutant amino acid sequence aa2-13/G12V-T3 | TEYKLVVVGAVG |
| 4 | RAS G12V mutant amino acid sequence aa3-14/G12V-T16 | EYKLVVVGAVGV |
| 5 | RAS G12V mutant amino acid sequence aa2-14/G12V-T15 | TEYKLVVVGAVGV |
| 6 | B13.14.1 TCR Vα amino acid sequence | MASAPISMLAMLFTLSGLRAQSVAQPEDQVNVAEGNPLTV KCTYSVSGNPYLFWYVQYPNRGLQFLLKYITGDNLVKGS YGFEAEFNKSQTSFHLKKPSALVSDSALYFCAVRDGRGGG NKLTFGTGTQLKVEL |
| 7 | B13.14.1 TCR Vβ amino acid sequence | MSNQVLCCVVLCLLGANTVDGGITQSPKYLFRKEGQNVT LSCEQNLNHDAMYWYRQDPGQGLRLIYYSQIVNDFQKGD IAEGYSVSREKKESFPLTVTSAQKNPTAFYLCASSPGQRDN SPLHFGNGTRLTVT |
| 8 | B13.14.1 TCR CDR1α amino acid sequence | VSGNPY |

(continued)

| SEQ ID NO | Description | Sequence information |
|---|---|---|
| 9 | B13.14.1 TCR CDR2α amino acid sequence | YITGDNLV |
| 10 | B13.14.1 TCR CDR3α amino acid sequence | AVRDGRGGGNKLT |
| 11 | B13.14.1 TCR CDR1β amino acid sequence | LNHDA |
| 12 | B13.14.1 TCR CDR2β amino acid sequence | SQIVND |
| 13 | B13.14.1 TCR CDR3β amino acid sequence | ASSPGQRDNSPLH |
| 14 | RAS G12V-mRNA nucleotide sequence | CTTGTTCTTTTTGCAGAAGCTCAGAATAAACGCTCAACT TTGGGCCACCATGCCCCGGCAGCTCAGCGCGGCGGCCG CGCTCTTCGCGTCCCTGGCCGTAATTTTGCACGATGGCA GTCAAATGAGAGCAAAAGCATTTCCAGAAACCAGAGAT TATTCTCAACCTACTGCAGCAGCAACAGTACAGGACAT AAAAAAACCTGTCCAGCAACCAGCTAAGCAAGCACCTC ACCAAACTTTAGCAGCAAGATTCATGGATGGTCATATCA CCTTTCAAACAGCGGCCACAGTAAAAATTCCAACAACT ACCCCAGCGACTACAAAAAACACTGCAACCACCAGCCC AATTACCTACACCCTGGTCACAACCCAGGCCACACCCA ACAACTCACACACAGCTCCTCCAGTTACTGAAGTTACAG TCGGCCCTAGCTTAGCCCCTTATTCACTGCCACCCACCA TCACCCCACCAGCTCATACAACTGGAACCAGTTCATCAA CCGTCAGCCACACAACTGGGAACACCACTCAACCCAGT AACCAGACCACCCTTCCAGCAACTTTATCGATAGCACTG CACAAAAGCACAACCGGTCAGAAGCCTGTTCAACCCAC CCATGCCCCAGGAACAACGGCAGCTGCCCACAATACCA CCCGCACAGCTGCACCTGCCTCCACGGTTCCTGGGCCCA CCCTTGCACCTCAGCCATCGTCAGTCAAGACTGGAATTT ATCAGGTTCTAAACGGAAGCAGACTCTGTATAAAAGCA GAGATGGGGATACAGCTGATTGTTCAAGACAAGGAGTC GGTTTTTTCACCTCGGAGATACTTCAACATCGACCCCAA CGCAACGCAAGCCTCTGGGAACTGTGGCACCCGAAAAT CCAACCTTCTGTTGAATTTTCAGGGCGGATTTGTGAATC TCACATTTACCAAGGATGAAGAATCATATTATATCAGTG AAGTGGGAGCCTATTTGACCGTCTCAGATCCAGAGACA ATTTACCAAGGAATCAAACATGCGGTGGTGATGTTCCA GACAGCAGTCGGGCATTCCTTCAAGTGCGTGAGTGAAC AGAGCCTCCAGTTGTCAGCCCACCTGCAGGTGAAAACA |

(continued)

| SEQ ID NO | Description | Sequence information |
|---|---|---|
| | | ACCGATGTCCAACTTCAAGCCTTTGATTTTGAAGATGAC CACTTTGGAAATGTGGATGAGTGCTCGTCTGACTACACA ACTGAATATAAACTTGTGGTAGTTGGAGCTGTTGGCGTA GGCAAGAGTGCCTTGACGATACAGCTAATTCAGAATCA TTTTGTGGACGAATATGATCCAACAATAGAGGATTCCTA CAGGAAGCAAGTAGTAATTGATGGAGAAACCTGTCTCT TGGATATTCTCGACACAGCAGGTCAAGAGGAGTACAGT GCAATGAGGGACCAGTACATGAGGACTGGGGAGGGCTT TCTTTGTGTATTTGCCATAAATAATACTAAATCATTTGA AGATATTCACCATTATAGAGAACAAATTAAAAGAGTTA AGGACTCTGAAGATGTACCTATGGTCCTAGTAGGAAAT AAATGTGATTTGCCTTCTAGAACAGTAGACACAAAACA GGCTCAGGACTTAGCAAGAAGTTATGGAATTCCTTTTAT TGAAACATCAGCAAAGACAAGACAGGGTGTTGATGATG CCTTCTATACATTAGTTCGAGAAATTCGAAAACATAAAG AAAAGATGAGCAAAGATGGTAAAAAGAAGAAAAAGAA GTCAAAGACAAAGTGTGTAATTATGATTGTGCTTCCTGT GATTGGGGCCATCGTGGTTGGTCTCTGCCTTATGGGTAT GGGTGTCTATAAATCCGCCTAAGGTGTCAATCATCTGG ATACCAGAGAATCTAATCCTCTAGAGGATCTGGTTACCA CTAAACCAGCCTCAAGAACACCCGAATGGAGTCTCTAA GCTACATAATACCAACTTACACTTACAAAATGTTGTCCC CCAAAATGTAGCCATTCGTATCTGCTCCTAATAAAAGA AAGTTTCTTCAC |
| 15 | B13.14.1 TCR Vα nucleotide sequence | ATGGCCTCTGCACCCATCTCGATGCTTGCGATGCTCTTC ACATTGAGTGGGCTGAGAGCTCAGTCAGTGGCTCAGCC GGAAGATCAGGTCAACGTTGCTGAAGGGAATCCTCTGA CTGTGAAATGCACCTATTCAGTCTCTGGAAACCCTTATC TTTTTTGGTATGTTCAATACCCCAACCGAGGCCTCCAGT TCCTTCTGAAATACATCACAGGGGATAACCTGGTTAAAG GCAGCTATGGCTTTGAAGCTGAATTTAACAAGAGCCAA ACCTCCTTCCACCTGAAGAAACCATCTGCCCTTGTGAGC GACTCCGCTTTGTACTTCTGTGCTGTGAGAGACGGTAGG GGAGGAGGAAACAAACTCACCTTTGGGACAGGCACTCA GCTAAAAGTGGAACTC |
| 16 | B13.14.1 TCR Vβ nucleotide sequence | ATGAGCAACCAGGTGCTCTGCTGTGTGGTCCTTTGTCTC CTGGGAGCAAACACCGTGGATGGTGGAATCACTCAGTC CCCAAAGTACCTGTTCAGAAAGGAAGGACAGAATGTGA CCCTGAGTTGTGAACAGAATTTGAACCACGATGCCATGT ACTGGTACCGACAGGACCCAGGGCAAGGGCTGAGATTG ATCTACTACTCACAGATAGTAAATGACTTTCAGAAAGG AGATATAGCTGAAGGGTACAGCGTCTCTCGGGAGAAGA AGGAATCCTTTCCTCTCACTGTGACATCGGCCCAAAAGA ACCCGACAGCTTTCTATCTCTGTGCCAGTAGCCCCGGAC AACGAGATAATTCACCCCTCCACTTTGGGAACGGGACC AGGCTCACTGTGACA |
| 17 | RAS WT-T15 | TEYKLVVVGAGGV |

(continued)

| SEQ ID NO | Description | Sequence information |
|---|---|---|
| 18 | Murine TCRα constant region amino acid sequence | DIQNPEPAVYQLKDPRSQDSTLCLFTDFDSQINVPKTMESG TFITDKTVLDMKAMDSKSNGAIAWSNQTSFTCQDIFKETN ATYPSSDVPCDATLTEKSFETDMNLNFQNLSVMGLRILLL KVAGFNLLMTLRLWSS |
| 19 | Murine TCRα constant region nucleotide sequence | GACATCCAGAACCCAGAACCTGCTGTGTACCAGTTAAA AGATCCTCGGTCTCAGGACAGCACCCTCTGCCTGTTCAC CGACTTTGACTCCCAAATCAATGTGCCGAAAACCATGG AATCTGGAACGTTCATCACTGACAAAACTGTGCTGGAC ATGAAAGCTATGGATTCCAAGAGCAATGGGGCCATTGC CTGGAGCAACCAGACAAGCTTCACCTGCCAAGATATCTT CAAAGAGACCAACGCCACCTACCCCAGTTCAGACGTTC |
| | | CCTGTGATGCCACGTTGACTGAGAAAAGCTTTGAAACA GATATGAACCTAAACTTTCAAAACCTGTCAGTTATGGGA CTCCGAATCCTCCTGCTGAAAGTAGCCGGATTTAACCTG CTCATGACGCTGAGGCTGTGGTCCAGT |
| 20 | Murine TCRβ constant region amino acid sequence | EDLRNVTPPKVSLFEPSKAEIANKQKATLVCLARGFFPDHV ELSWWVNGKEVHSGVSTDPQAYKESNYSYCLSSRLRVSA TFWHNPRNHFRCQVFHGLSEEDKWPEGSPKPVTQNISAE AWGRADCGITSASYQQGVLSATILYEILLGKATLYAVLVS TLVVMAMVKRKNS |
| 21 | Murine TCRβ constant region nucleotide sequence | GAGGACCTGCGCAACGTCACCCCACCAAAGGTCAGTTT GTTTGAGCCATCAAAGGCGGAGATCGCCAACAAACAGA AAGCTACGCTCGTGTGTTTGGCTCGGGGCTTCTTCCCAG ACCACGTAGAACTTTCCTGGTGGGTCAATGGAAAGGAG GTTCATTCCGGAGTGTCCACTGATCCCCAAGCGTACAAG GAATCCAACTATAGCTACTGTCTCTCATCTCGGCTCCGG GTGAGTGCGACATTCTGGCATAATCCTCGGAACCACTTT CGATGCCAAGTGCAGTTTCATGGGTTGAGCGAGGAAGA CAAGTGGCCCGAGGGCAGTCCTAAACCAGTCACTCAAA ACATAAGCGCCGAGGCATGGGGTAGAGCCGATTGTGGG ATTACTAGCGCTTCATACCAACAAGGGGTATTGAGCGCT ACAATTCTTTACGAAATTCTCCTCGGCAAGGCGACGCTC TACGCCGTACTGGTGTCTACTCTCGTGGTTATGGCAATG GTGAAACGGAAAAACAGC |
| 22 | P2A nucleotide sequence | GCCACCAACTTCAGCCTGCTGAAGCAGGCCGGCGACGT GGAGGAGAACCCCGGCCCC |

(continued)

| SEQ ID NO | Description | Sequence information |
|---|---|---|
| 23 | B13.14.1/TRAV+mCa nucleotide sequence | ATGGCCTCTGCACCCATCTCGATGCTTGCGATGCTCTTC ACATTGAGTGGGCTGAGAGCTCAGTCAGTGGCTCAGCC GGAAGATCAGGTCAACGTTGCTGAAGGGAATCCTCTGA CTGTGAAATGCACCTATTCAGTCTCTGGAAACCCTTATC TTTTTTGGTATGTTCAATACCCCAACCGAGGCCTCCAGT TCCTTCTGAAATACATCACAGGGGATAACCTGGTTAAAG GCAGCTATGGCTTTGAAGCTGAATTTAACAAGAGCCAA ACCTCCTTCCACCTGAAGAAACCATCTGCCCTTGTGAGC GACTCCGCTTTGTACTTCTGTGCTGTGAGAGACGGTAGG GGAGGAGGAAACAAACTCACCTTTGGGACAGGCACTCA GCTAAAAGTGGAACTCGACATCCAGAACCCAGAACCTG CTGTGTACCAGTTAAAAGATCCTCGGTCTCAGGACAGCA CCCTCTGCCTGTTCACCGACTTTGACTCCCAAATCAATG TGCCGAAAACCATGGAATCTGGAACGTTCATCACTGAC AAAACTGTGCTGGACATGAAAGCTATGGATTCCAAGAG CAATGGGGCCATTGCCTGGAGCAACCAGACAAGCTTCA CCTGCCAAGATATCTTCAAAGAGACCAACGCCACCTAC CCCAGTTCAGACGTTCCCTGTGATGCCACGTTGACTGAG AAAAGCTTTGAAACAGATATGAACCTAAACTTTCAAAA CCTGTCAGTTATGGGACTCCGAATCCTCCTGCTGAAAGT AGCCGGATTTAACCTGCTCATGACGCTGAGGCTGTGGTC CAGT |
| 24 | B13.14.1/TRBV+mCb nucleotide sequence | ATGAGCAACCAGGTGCTCTGCTGTGTGGTCCTTTGTCTC CTGGGAGCAAACACCGTGGATGGTGGAATCACTCAGTC CCCAAAGTACCTGTTCAGAAAGGAAGGACAGAATGTGA CCCTGAGTTGTGAACAGAATTTGAACCACGATGCCATGT ACTGGTACCGACAGGACCCAGGGCAAGGGCTGAGATTG ATCTACTACTCACAGATAGTAAATGACTTTCAGAAAGG AGATATAGCTGAAGGGTACAGCGTCTCTCGGGAGAAGA AGGAATCCTTTCCTCTCACTGTGACATCGGCCCAAAAGA ACCCGACAGCTTTCTATCTCTGTGCCAGTAGCCCCGGAC AACGAGATAATTCACCCCTCCACTTTGGGAACGGGACC AGGCTCACTGTGACAGAGGACCTGCGCAACGTCACCCC |
| | | ACCAAAGGTCAGTTTGTTTGAGCCATCAAAGGCGGAGA TCGCCAACAAACAGAAAGCTACGCTCGTGTGTTTGGCTC GGGGCTTCTTCCAGACCACGTAGAACTTTCCTGGTGGG TCAATGGAAAGGAGGTTCATTCCGGAGTGTCCACTGATC CCCAAGCGTACAAGGAATCCAACTATAGCTACTGTCTCT CATCTCGGCTCCGGGTGAGTGCGACATTCTGGCATAATC CTCGGAACCACTTTCGATGCCAAGTGCAGTTTCATGGGT TGAGCGAGGAAGACAAGTGGCCCGAGGGCAGTCCTAAA CCAGTCACTCAAAACATAAGCGCCGAGGCATGGGGTAG AGCCGATTGTGGGATTACTAGCGCTTCATACCAACAAG GGGTATTGAGCGCTACAATTCTTTACGAAATTCTCCTCG GCAAGGCGACGCTCTACGCCGTACTGGTGTCTACTCTCG TGGTTATGGCAATGGTGAAACGGAAAAACAGC |

(continued)

| SEQ ID NO | Description | Sequence information |
|---|---|---|
| 25 | General formula of TCR CDR3α | $AVRDX_1X_2X_3GGNKLT$ |
| 26 | General formula of TCR CDR3β | $ASSX_4GX_5RDNSPLH$ |
| 27 | General formula of TCR CDR1α | $VSGX_6PY$ |
| 28 | General formula of TCR CDR2α | $YX_7X_8GDNLV$ |
| 29 | TCR CDR2β General formula of | $SX_9X_{10}VNX_{11}$ |
| 30 | B13.14.1 TCR FR1α | MASAPISMLAMLFTLSGLRAQSVAQPEDQVNVAEGNPLTVKCTYS |
| 31 | B13.14.1 TCR FR2α | LFWYVQYPNRGLQFLLK |
| 32 | B13.14.1 TCR FR3α | KGSYGFEAEFNKSQTSFHLKKPSALVSDSALYFC |
| 33 | B13.14.1 TCR FR4α | FGTGTQLKVEL |
| 34 | B13.14.1 TCR FR1β | MSNQVLCCVVLCLLGANTVDGGITQSPKYLFRKEGQNVTLSCEQN |
| 35 | B13.14.1 TCR FR2β | MYWYRQDPGQGLRLIYY |
| 36 | B13.14.1 TCR FR3β | FQKGDIAEGYSVSREKKESFPLTVTSAQKNPTAFYLC |
| 37 | B13.14.1 TCR FR4β | FGNGTRLTVT |
| 38 | General formula of TCR FR2α | $LFWYVQYPNRGLQFLLX_{12}$ |
| 39 | General formula of TCR FR2β | $MYWYRQDPGQGLRLIYX_{13}$ |
| 40 | General formula of TCR FR3β | $FX_{14}KGDIAEGYSVSREKKESFPLTVTSAQKNPTAFYLC$ |
| 41 | G12V-T1 | TEYKLVVVGAVGVGKSALTIQLI |
| 42 | G12V-T2 | AVGVGKSALTIQLI |
| 43 | G12V-T4 | VVVGAVGVGKS |
| 44 | G12V-T5 | VGAVGVGKSALTIQ |
| 45 | G12V-T6 | YKLVVVGAVGVG |
| 46 | G12V-T7 | VVVGAVGVGKSALT |
| 47 | G12V-T8 | LVVVGAVGVGKS |
| 48 | G12V-T10 | YKLVVVGAVG |
| 49 | G12V-T11 | KLVVVGAVG |
| 50 | G12V-T12 | TEYKLVVVGAV |
| 51 | G12V-T13 | EYKLVVVGAV |
| 52 | G12V-T14 | YKLVVVGAV |
| 53 | G12V-T17 | YKLVVVGAVGV |

(continued)

| SEQ ID NO | Description | Sequence information |
|---|---|---|
| 54 | G12V-T18 | TEYKLVVVGAVGVG |
| 55 | G12V-T19 | TEYKLVVVGAVGVGK |
| 56 | G12V-T20 | TEYKLVVVGAVGVGKS |
| 57 | G12V-T21 | TEYKLVVVGAVGVGKSA |
| 58 | G12V-T15-A1 | AEYKLVVVGAVGV |
| 59 | G12V-T15-A2 | TAYKLVVVGAVGV |
| 60 | G12V-T15-A3 | TEAKLVVVGAVGV |
| 61 | G12V-T15-A4 | TEYALVVVGAVGV |
| 62 | G12V-T15-A5 | TEYKAVVVGAVGV |
| 63 | G12V-T15-A6 | TEYKLAVVGAVGV |
| 64 | G12V-T15-A7 | TEYKLVAVGAVGV |
| 65 | G12V-T15-A8 | TEYKLVVAGAVGV |
| 66 | G12V-T15-A9 | TEYKLVVVAAVGV |
| 67 | G12V-T15-G10 | TEYKLVVVAGVGV |
| 68 | G12V-T15-A12 | TEYKLVVVGAVAV |
| 69 | G12V-T15-A13 | TEYKLVVVGAVGA |

## EXAMPLES

The present invention will now be described with reference to the following examples, which are intended to illustrate the present invention, but not to limit it.

Unless otherwise specified, the molecular biology experiment methods and immunoassay methods used in the present invention were basically referred to the methods described by J. Sambrook et al., Molecular Cloning: A Laboratory Manual, 2nd Edition, Cold Spring Harbor Laboratory Press, 1989, and F. M. Ausubel et al., Short protocols in Molecular Biology, 3rd Edition, John Wiley & Sons, Inc., 1995; the restriction enzymes were used in accordance with the conditions recommended by the product manufacturers. Those skilled in the art understand that the examples describe the present invention by way of example and are not intended to limit the scope of the claimed invention.

Example 1: Culture and expansion of tumor-infiltrating T cell

The tumor samples obtained by resection surgery from patients with colorectal cancer with RAS mutation G12V were minced with scalpel to tumor pieces of 2mm to 4mm, washed twice with DPBS solution, and cultured in a TIL medium containing IL2 (6000IU/ml), human AB serum (2%), Hepes (25mM), and Xvivo15 in a 24-well plate. Half of the medium was replaced every 2 to 3 days, and when the tumor-infiltrating T lymphocytes (TIL) grew to 60-80% confluence (containing about $0.5\text{-}3.0\times10^6$ TILs), the TILs were harvested and stored in CS10 cryoprotectant solution.

The TIL cells can be expanded by co-cultured with peripheral blood mononuclear cells derived from different donors (pooled PBMC, donor>3) irradiated with γ-rays (4000cGy) at a ratio of 1:30 to 1:200, and cultured in a T175 culture flask with no more than $1\times10^8$ cells per flask (the culture medium was TIL medium supplemented with 10ng/ml OKT3). After 3 days of culture, half of the medium (X vivo 15 medium containing 3000IU/ml IL2, 2% human AB serum) was replaced; after 7 days of culture, the cells were washed once, the medium was replaced and passaged at $1.0\times10^6$/ml; the cells were harvested and cryopreserved after 10-14 days of culture.

Example 2: Preparation of RAS G12V antigen mRNA

The vector UTR-LAMP3 Lumenal-KRAS$^{G12V}$-LMP3 Sorting-UTR was constructed according to the following design for sequence containing RAS mutation G12V, the sequence was set forth in SEQ ID NO: 14 in Table 1, in which KRAS was marked with a single underline, and the G12V mutation was marked with a double underline. The above sequence was synthesized and cloned into the pcDNA3.1 vector, and the mRNA transcription was prepared in vitro using T7 promoter (mMESSAGE mMACHINE T7 Transcription Kit, Thermofisher), and the mRNA after transcription was sub-packaged and stored at -80°C.

Example 3: Screening by antigen-presenting cell

Maturation of DC cell (Dendritic cell): The patient's autologous peripheral blood CD14-positive cells were isolated with MACS CD14 Isolation Kit, and cultured in AIM-V medium containing IL4 (1000IU/ml), GM-CSF (1000IU/ml) and 1% human AB serum. The culture medium was replaced with fresh medium on the third day, and the cells were cryopreserved in CS10 cryoprotectant solution after culturing for 5-6 days.

Induction and generation of LCL cell (Lymphoblastoid cell line): The patient's peripheral blood mononuclear cells in an amount of $5 \times 10^6$ were resuspended in RPMI1640 medium containing 10% fetal bovine serum, and added with B95.8 supernatant of cell culture which contains EBV for induction. The induction was generally completed within 14 to 30 days, and half of the medium was replaced every 7 days during the induction period, and the established LCL cell line after induction was expanded and cryopreserved.

After recovery, the TIL cells were cultured for at least 48 hours for screening. The DC cells or LCL cells, referred to as APC (antigen-presenting cell), were subjected to transfection of KRAS G12V mRNA using a Neon electroporator, in which APC cells were resuspended in the electroporation solution to $1 \times 10^7$/ml, 100 $\mu$l of the cells was added with 5-8$\mu$g mRNA for each electroporation (1500V, 30ms, 1 pulse). Transfected APCs can be used next day after culturing. 1 to $2 \times 10^5$ of TIL cells and $0.5 \times 10^5$ of electroporated DC cells or $4 \times 10^5$ of electroporated LCL cells were cultured in Xvivo15 medium in a 96-well U-bottom plate; after 16 hours of culture, the cell culture supernatant was collected, and the IFN$\gamma$ release in the supernatant was determined using Human IFN$\gamma$ Flex Set. The patient numbered as B13 had metastatic colorectal cancer which had KRAS$^{G12V}$ mutation. The TIL screening results were as follows:

Table 1. TIL screening results

| TIL Fraction | IFN$\gamma$-Ctrl | IFN$\gamma$-G12V | Relative change |
|---|---|---|---|
| B13.1 | 579 | 1937 | 3.3 |
| B13.2 | 1115 | 1106 | 1.0 |
| B13.3 | 2340 | 3551 | 1.5 |
| B13.4 | 3189 | 20387 | 6.39 |
| B13.5 | 588 | 744 | 1.3 |
| B13.8 | 20304 | 34676 | 1.7 |
| B13.10 | 815 | 812 | 1.0 |
| B13.11 | 1846 | 3403 | 1.8 |
| B13.12 | 1313 | 2452 | 1.8 |
| B13.13 | 676 | 980 | 1.4 |
| B13.14 | 811 | 3899 | 4.8 |
| B13.15 | 7369 | 18518 | 2.5 |
| B13.16 | 8181 | 788 | 10.3 |
| Ctrl: TIL co-cultured with non-electroporated APC<br>G12V: TIL co-cultured with APC electroporated with G12V mRNA | | | |

Example 4: Sorting and expansion of TIL

The TIL cells stimulated by APC cells were sorted by flow cytometry. $1 \times 10^6$ TIL cells were resuspended in flow buffer (DPBS solution containing 1% human AB serum, 2mM EDTA), added with CD3/CD137 antibody and PI (propidium iodide solution), incubated at 4°C for 1 hour, washed twice with flow buffer, and sorted with BD FACSAiraII flow sorter. The sorted population was a cell population negative for PI, positive for CD3 and positive for CD137. The sorted cells were preserved in RPMI1640 medium containing 10% human AB serum, and placed on ice. The collected sorted cells (CD3 and CD137+) were centrifuged at 300g for 10 minutes at 4°C to remove 80% of the preservation solution, washed twice with DPBS solution, resuspended in DPBS, and subjected to 10×Genomics single-cell sequencing.

Example 5: Screening for TCR recognizing KRAS[G12V]

The TCR clone obtained by the single-cell sequencing was subjected to gene-synthesis according to the sequence of TRAVmCa-P2A-TRBVmCb, wherein TRAV was the α-chain variable region of TCR, mCa was the murine TCRα constant region (its amino acid sequence and nucleotide sequence were set forth in SEQ ID NO: 18 and 19, respectively), TRBV was the β-chain variable region of TCR, mCb was the murine TCRβ constant region (its amino acid sequence and nucleotide sequence were set forth in SEQ ID NO: 20 and 21, respectively), and P2A was the self-cleaving peptide (its nucleotide sequence was set forth in SEQ ID NO: 22); the above sequence was cloned into a lentiviral transfer vector (GV401); the transfer and package vectors were transiently transfected into 293T cells according to the standard lentiviral vector packaging method, the culture supernatant was collected which contained the lentiviral vector expressing TCR. T cells from a healthy donor were activated in OKT3/15E8 antibody-coated 6-well plate for 24 hours, then transduced with the TCR-containing lentiviral vector and cultured for 6 to 8 days for TCR screening (the transduced T cells were collected, washed with FACS buffer solution, and $1\times10^6$ modified T cells were stained by using an antibody recognizing murine TCRβ constant region to detect the expression of recombinant TCR), thereby obtaining T cells modified by recombinant TCR.

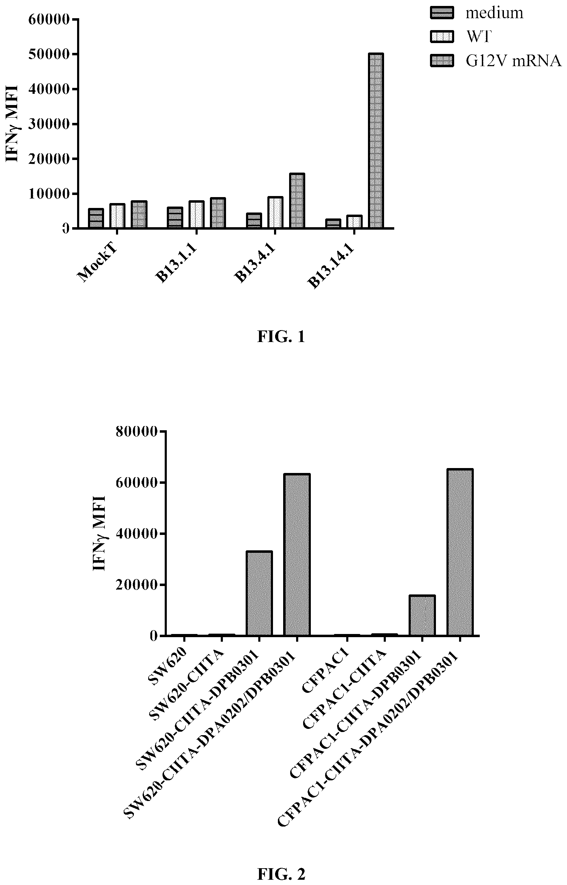

The autologous LCL cells of Patient No. B13 were transiently transfected with KRAS[G12V] mRNA and cultured overnight according to the electroporation method in "Example 3"; the T cells modified by recombinant TCR and the electroporated LCL cells at a ratio of $1\times10^5 : 1\times10^5$ were inoculated and co-cultured in a 96-well U-bottom plate, the IFNγ specific release in the supernatant was detected, and the results were shown in FIG. 1. The screening results showed that the TCR with clone number B13.14.1 could specifically recognize the KRAS G12V point mutation but not wild-type RAS.

The sequence of B13.14.1 TCR was shown in the table below:

Table 2. TCR sequences

|  | B13.14.1-TCR Vα region TRAV3*01-J10*01 | B13.14.1-TCR Vβ region TRBV19*01-D1*01-J1-6*02 |
|---|---|---|
| Variable region | SEQ ID NO: 6 | SEQ ID NO: 7 |
| CDR1 | SEQ ID NO: 8 | SEQ ID NO: 11 |
| CDR2 | SEQ ID NO: 9 | SEQ ID NO: 12 |
| CDR3 | SEQ ID NO: 10 | SEQ ID NO: 13 |

Example 6: HLA restriction determination of B13.14.1 TCR

6.1 HLA restriction determination 1

T cells expressing recombinant B13.14.1 TCR (hereinafter referred to as B13.14.1 TCR-T) were prepared according to the method described in Example 5, which comprises transducing T cells from a healthy donor with a lentiviral vector containing a nucleotide sequence encoding the recombinant TCR, wherein the nucleotide sequences of the α-chain (TRAVmCa) and β-chain (TRBVmCb) of the recombinant TCR were set forth in SEQ ID NO: 23 and 24, respectively. The HLA restriction of the B13.14.1 TCR was determined by the method described below.

SW620 cell line (colorectal cancer lymph-node metastasis) contained KRAS G12V homozygous mutation; CFPAC-1 cell line (pancreatic cancer) contained KRAS G12V heterozygous mutation; and their HLA-DP matched-types were shown in the following table:

Table 3. HLA-DP matched-type of SW620 cell

| Cell line | HLA-DP gene | Allelic gene |
|---|---|---|
| SW620 | DPA1 | 01:03/02:01 |
|  | DPB1 | 01:01/04:01 |

(continued)

| Cell line | HLA-DP gene | Allelic gene |
|-----------|-------------|--------------|
| CFPAC-1 | DPA1 | 01:03/01:03 |
| | DPB1 | 04:01/04:01 |

The SW620-CIITA or CFPAC1-CIITA cells, SW620/CFPAC1-CIITA-DPB1*03:01 cells and SW620/CFPAC1-CIITA-DPA1*02:02-DPB1*03:01 cells were collected and resuspended in RPMI1640 medium, incubated at 37°C for 2 hours, and washed twice with DPBS solution. The antigen-presenting cells (the above-mentioned modified SW620 or CFPAC-1 cells) were co-cultured overnight with B13.14.1 TCR-T cells in a ratio of $2 \times 10^4 : 2 \times 10^4$ in RPMI1640 medium containing 2% fetal bovine serum, the IFN$\gamma$ release in the supernatant was measured, and the results were shown in FIG. 2.

The results showed that B13.14.1 TCR could recognize the G12V mutation restricted and presented by HLA-DPB1*03:01, in which all three alleles of HLA-DPA1 in combination with DPB1*03:01 could be used for presentation.

6.2 HLA restriction determination 2

The LCL cell lines prepared from different donor sources were loaded with KRAS-G12V T15 antigen peptide (10 $\mu$g/ml), then were co-incubated with Mock T or B13.14.1 TCR-T in RPMI1640 medium containing 2% FBS, and the IFN$\gamma$ content in supernatant was determined. The ability of B13.14.1 TCR to recognize KRAS-G12V presented by human high-frequency HLA-DPB1 genes was determined, and the results were shown in the table below.

Table 4. Results of HLA restriction determination

| HLA-DPB1 | LCL cell | IFN$\gamma$ release Mock T | IFN$\gamma$ release B13.14.1 TCR |
|----------|----------|---------------------------|-------------------------------|
| DPB1*02:01 | D5-LCL | 373 | 212 |
| DPB1*02:02 | D12-LCL | 620 | 1156 |
| DPB1*03:01 | B13-LCL | 398 | 38765 |
| DPB1*04:01 | B8-LCL | 543 | 874 |
| DPB1*04:02 | B11-LCL | 607 | 923 |
| DPB1*05:01 | B4-LCL | 1284 | 1021 |
| DPB1*09:01 | YY-LCL | 621 | 907 |
| DPB1*13:01 | B10-LCL | 507 | 444 |
| DPB1*14:01 | B1-LCL | 473 | 28375 |
| DPB1*17:01 | D350-LCL | 418 | 295 |
| DPB1*38:01 | D2-LCL | 725 | 251 |
| DPB1*135:01 | WY-LCL | 413 | 324 |

The results in Table 4 showed that B13.14.1 TCR could not only recognize the KRAS-G12V antigen peptide presented by HLA-DPB1*03:01, but also recognize the KRAS-G12V antigen peptide presented by HLA-DPB1*14:01.

Example 7: Determination of presenting epitope of RAS G12V

Peptides with length of 9-23 (SEQ ID NOs: 2-5, 18-34) containing G12V mutation site were synthesized according to the following table and subjected to antigen presentation by autologous LCL cells, then the IFN release was determined to screen the RAS G12V epitope recognized by B13.14.1 TCR.

Table 5. Ras G12V epitope screening table

| Peptide No. | Peptide length | Amino acid sequence | SEQ ID NO: |
|-------------|----------------|---------------------|------------|
| G12V-T1 | 23 | TEYKLVVVGAVGVGKSALTIQLI | 41 |
| G12V-T2 | 15 | AVGVGKSALTIQLI | 42 |

(continued)

| Peptide No. | Peptide length | Amino acid sequence | SEQ ID NO: |
|---|---|---|---|
| G12V-T3 | 12 | TEYKLVVVGAVG | 3 |
| G12V-T4 | 11 | VVVGAVGVGKS | 43 |
| G12V-T5 | 15 | VGAVGVGKSALTIQ | 44 |
| G12V-T6 | 12 | YKLVVVGAVGVG | 45 |
| G12V-T7 | 15 | VVVGAVGVGKSALT | 46 |
| G12V-T8 | 12 | LVVVGAVGVGKS | 47 |
| G12V-T9 | 11 | EYKLVVVGAVG | 2 |
| G12V-T10 | 10 | YKLVVVGAVG | 48 |
| G12V-T11 | 9 | KLVVVGAVG | 49 |
| G12V-T12 | 11 | TEYKLVVVGAV | 50 |
| G12V-T13 | 10 | EYKLVVVGAV | 51 |
| G12V-T14 | 9 | YKLVVVGAV | 52 |
| G12V-T15 | 13 | TEYKLVVVGAVGV | 5 |
| G12V-T16 | 12 | EYKLVVVGAVGV | 4 |
| G12V-T17 | 11 | YKLVVVGAVGV | 53 |
| G12V-T18 | 14 | TEYKLVVVGAVGVG | 54 |
| G12V-T19 | 16 | TEYKLVVVGAVGVGK | 55 |
| G12V-T20 | 16 | TEYKLVVVGAVGVGKS | 56 |
| G12V-T21 | 17 | TEYKLVVVGAVGVGKS A | 57 |

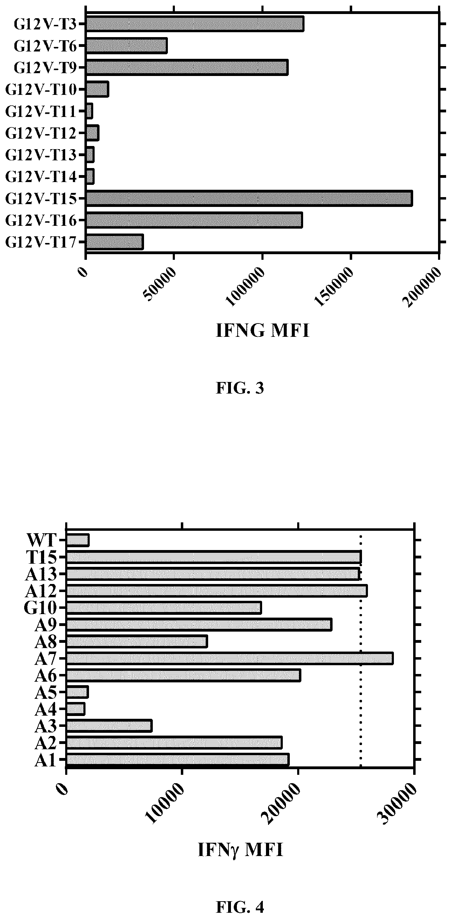

The above peptides were synthesized and then dissolved by adding DMSO; the autologous LCL cells of Patient B13 were resuspended in RPMI1640 medium, added with the above peptides to a final concentration of 1 $\mu$g/ml, incubated for 2 hours, washed twice with DPBS solution, and resuspended in RPMI1640 medium with % fetal bovine serum to reach $2\times10^5$/ml. The antigen-presenting cells loaded with the peptides were co-cultured with B13.14.1 TCR-T cells overnight according to a ratio of $2\times10^4$: $2\times10^4$, the IFN$\gamma$ release in supernatant was measured, and the results were shown in FIG. 3.

The results showed that all G12V-T3, G12V-T9, G12V-T15, and G12V-T16 peptides could effectively induce the IFN$\gamma$ release of B13.14.1 TCR-T, indicating that HLA-DPB1*03:01 could present the above peptides. The above results also indicated that the peptides comprising amino acid residues at positions 3-13 of the RAS G12V mutant could effectively activate T lymphocytes, thereby inducing an immune response against tumors with RAS G12V mutation.

Example 8: Determination of key amino acids presented by RAS G12V epitope by alanine scanning

By performing one-by-one alanine replacement on RAS G12V epitope peptides, the key amino acids involved in antigen presentation in the RAS G12V epitopes could be screened out. The peptide segments after alanine mutation (the mutated amino acid was underlined) were shown in the following table:

Table 6. Alanine scanning of RAS G12V epitope

| Peptide number | Mutated amino acid | Amino acid sequence | SEQ ID NO: |
|---|---|---|---|
| G12V-T15-A1 | T1A | AEYKLVVVGAVGV | 58 |
| G12V-T15-A2 | E2A | TAYKLVVVGAVGV | 59 |
| G12V-T15-A3 | Y3A | TEAKLVVVGAVGV | 60 |
| G12V-T15-A4 | K4A | TEYALVVVGAVGV | 61 |

(continued)

| Peptide number | Mutated amino acid | Amino acid sequence | SEQ ID NO: |
|---|---|---|---|
| G12V-T15-A5 | L5A | TEYKAVVVGAVGV | 62 |
| G12V-T15-A6 | V6A | TEYKLAVVGAVGV | 63 |
| G12V-T15-A7 | V7A | TEYKLVAVGAVGV | 64 |
| G12V-T15-A8 | V8A | TEYKLVVAGAVGV | 65 |
| G12V-T15-A9 | G9A | TEYKLVVVAAVGV | 66 |
| G12V-T15-G10 | A10G | TEYKLVVVAGVGV | 67 |
| G12V-T15-A12 | G12A | TEYKLVVVGAVAV | 68 |
| G12V-T15-A13 | V13A | TEYKLVVVGAVGA | 69 |

After the above peptides were synthesized, IFNγ or IL2 in the supernatant after co-culture of antigen-presenting cells with B13.14.1 TCR-T was measured according to the method in Example 7, and the results were shown in FIG. 4. The above results showed that in the TEYKLVVVGAVGV epitope, the key amino acids involved in antigen presentation were p3Y, p4K, p5L, and p8V, which was manifested in that after these four amino acids were mutated to alanine, the ability of B13.14.1 TCR-T to recognize the RAS G12V epitope was reduced, the most important of these are p3Y, p4K and p5L.

Example 9: Tumor lysis determination of B13.14.1 TCR-T

SW620-CIITA-DPA02:02/DPB03:01 cells were resuspended in RPMI1640 medium containing 2% FBS, inoculated in a 96-well plate according to $10^4$/well, and added with Mock-T, B13.14.1 TCR-CD4$^+$T and B13.14.1 TCR-CD8$^+$T according to an E:T ratio of 10, 3, 1, 0.3, 0.1, and a control without T cell was set; after 48 hours of co-incubation, the medium was sucked out and 200 μl of DPBS solution was added to each well to perform washing once, then 100 μl of RPMI1640 medium containing 2% FBS and 10 μl of CCK8 detection reagent (Cell Counting Kit-8) were added to each well, incubated at 37°C for 1 hour, and the light absorption at 450 nm was read.

$$\text{Killing rate} = 1 - (\text{light absorption value/light absorption value of control well})$$

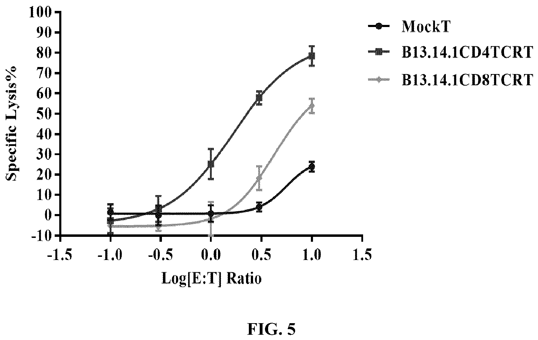

The results were shown in FIG. 5. The results showed that B13.14.1 TCR-T could kill tumor cells in a dose-dependent manner, and showed a dependency on CD4.

Example 10: Functional avidity determination of B13.14.1 TCR-T

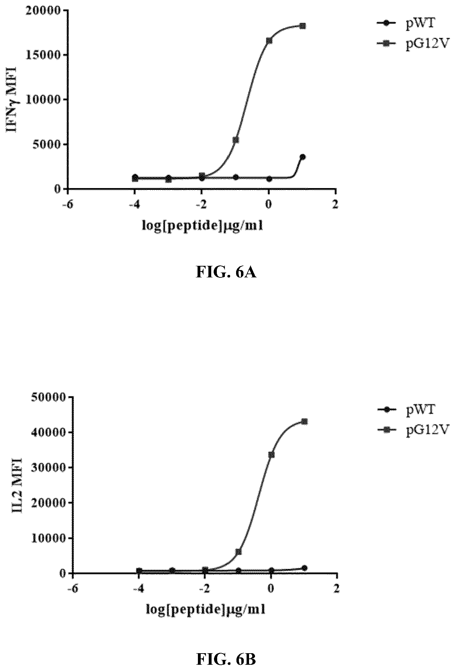

B13 autologous LCL cells were subjected to antigen loading with different concentrations (10 μg/ml, 1 μg/ml, 0.1 μg/ml, 0.01 μg/ml, 0.001 μg/ml) of RAS G12V-T15 peptide (SEQ ID NO: 5) and the corresponding wild-type peptide (SEQ ID NO: 17), respectively, cultured at 37°C for 2 hours, then washed twice with DPBS solution; the antigen-presenting cells were co-cultured overnight with B13.14.1 TCR-T cells according to a ratio of $2 \times 10^4 : 2 \times 10^4$ in RPMI1640 medium containing 2% fetal bovine serum, and the release of IL2 and IFNγ in the supernatant was measured. The results were shown in FIGS. 6A to 6B. The results showed that B13.14.1 TCR-T could specifically recognize the RAS G12V mutant peptide within the tested concentration range, and could release IFNγ and IL2, and it could still efficiently recognize the RAS G12V mutant peptide when the antigen peptide concentration was 0.1 μg/ml, indicating that the T cells expressing this TCR had high affinity and high specificity for the RAS G12V mutant.

Example 11: Determination of in vivo efficacy of B13.14.1 TCR-T

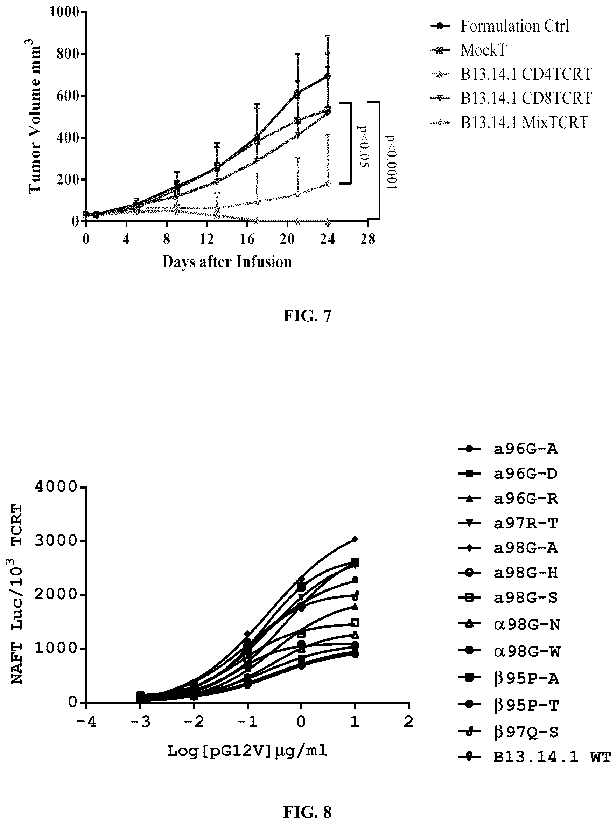

The SW620-DPAB tumor cell line (overexpressing HLA-DPB1*03:01) was inoculated subcutaneously in NSG mice ($1 \times 10^7$/mouse), and when the tumor volume reached about 50 mm$^3$, the blank preparation, MockT (non-transduced T cell control), B13.14.1CD4TCRT (CD4 T cells genetically modified with B13.14.1TCR), B13.14.1CD8TCRT (CD8 T cells genetically modified with B13.14.1TCR), or B13.14.1 MixTCRT (CD4 T cells and CD8 T cells modified with B13.14.1TCR, which were mixed at a ratio of 1:1) were administered via tail vein, respectively, the dose was $1 \times 10^7$ mTCRβ+ cells/animal, and the tumor growth was measured. The results were shown in FIG. 7.

The results showed that B13.14.1TCRT could eliminate the tumor of SW620-DPAB cell line, and showed dependence on CD4 co-receptor.

Example 12: Affinity maturation of B13.14.1 TCR

According to the table below, point mutations were performed on the Vα and Vβ of B13.14.1 TCR (including CDR3 region and MHC II binding region), and a lentiviral vector shuttle plasmid encoding the TCR mutant was constructed, packaged with the standard lentiviral vector in 293T cells, and B13.14.1TCR mutant were subjected to function screening.

Table 7.

| Mutation region | Amino acid at the mutated position |
|---|---|
| Vα-CDR3 | 95D, 96G, 97R, 98G |
| Vβ-CDR3 | 95P, 96G, 97Q, 98R, 99D |
| Vα-MHCIIβ binding region | 30N, 32Y, 49K, 511, 52T |
| Vβ-MHCIIα binding region | 48Y, 50Q, 511, 54D, 56Q |
| Note: The amino acid positions were determined according to the IMGT TCR numbering system. | |

12.1 TCR CDR3 mutant screening

The TCR mutant (CDR3 region) lentiviral vector was transduced into Jurkat-NFAT-Luc cell line, and B13 LCL cells loaded with KRAS-G12V antigen (RAS G12V-T15 peptide, SEQ ID NO: 5) were used as antigen-presenting cells. $2\times10^4$ TCR-T and $2\times10^4$ antigen-loaded B13 LCL cells were co-cultured for 16-24 hours, and added with ONE Glo Luciferase to detect the expression of fluorescent signal, and the TCR Mut RLU/WT RLU (ratio of RLU signal value of TCR mutant to RLU signal value of wild-type B13.14.1 TCR) was calculated. The results were as follows.

Table 8. Screening results of TCR CDR3 mutants

| TCR mutant | TCR Mut RLU/WT RLU | mTCRβ% |
|---|---|---|
| α95D-A | 0.5% | 89.90% |
| α95D-E | 0.9% | 85.60% |
| α95D-F | 1.0% | 59.10% |
| α95D-G | 1.5% | 79.20% |
| α95D-H | 0.8% | 92.80% |
| α95D-I | 1.6% | 83.70% |
| α95D-K | 1.4% | 57.50% |
| α95D-L | 1.4% | 84.50% |
| α95D-M | 0.7% | 90.20% |
| α95D-N | 11.9% | 85.40% |
| α95D-P | 1.2% | 80.70% |
| α95D-Q | 1.3% | 88.70% |
| α95D-R | 1.8% | 65.50% |
| α95D-S | 2.2% | 87.80% |
| α95D-T | 4.7% | 84.90% |
| α95D-V | 2.0% | 87.80% |
| α95D-W | 2.3% | 84.80% |
| α95D-Y | 2.4% | 88.20% |
| α96G-A | 45.5% | 83.90% |

(continued)

| TCR mutant | TCR Mut RLU/WT RLU | mTCRβ% |
|---|---|---|
| α96G-D | 30.7% | 56.30% |
| α96G-E | 1.7% | 73.80% |
| α96G-F | 2.7% | 73.80% |
| α96G-H | 2.2% | 80.30% |
| α96G-I | 2.6% | 75.40% |
| α96G-K | 5.7% | 87.70% |
| α96G-L | 123.2% | 84.80% |
| α96G-M | 126.2% | 86.90% |
| α96G-N | 19.3% | 71.30% |
| α96G-P | 9.4% | 70.90% |
| α96G-Q | 90.0% | 81.20% |
| α96G-R | 122.9% | 89.50% |
| α96G-S | 76.1% | 78.00% |
| α96G-T | 4.5% | 84.00% |
| α96G-V | 48.1% | 54.80% |
| α96G-W | 58.8% | 49.50% |
| α96G-Y | 90.0% | 47.60% |
| α97R-A | 68.6% | 70.90% |
| α97R-D | 46.2% | 41.40% |
| α97R-E | 6.5% | 40.00% |
| α97R-F | 21.0% | 62.10% |
| α97R-G | 45.4% | 51.90% |
| α97R-H | 50.1% | 48.40% |
| α97R-I | 71.9% | 52.80% |
| α97R-K | 113.0% | 59.00% |
| α97R-L | 33.8% | 33.20% |
| α97R-M | 36.5% | 35.10% |
| α97R-N | 79.4% | 38.70% |
| α97R-P | 38.9% | 36.60% |
| α97R-Q | 37.5% | 49.30% |
| α97R-S | 44.4% | 7.06% |
| α97R-T | 245.3% | 49.50% |
| α97R-V | 40.3% | 26.50% |
| α97R-W | 35.5% | 56.40% |
| α97R-Y | 46.9% | 42.90% |
| α98G-A | 467.6% | 48.20% |
| α98G-D | 113.1% | 32.90% |
| α98G-E | 144.2% | 38.20% |

(continued)

| TCR mutant | TCR Mut RLU/WT RLU | mTCRβ% |
|---|---|---|
| α98G-F | 110.3% | 17.10% |
| α98G-H | 162.2% | 32.50% |
| α98G-I | 216.3% | 58.40% |
| α98G-K | 65.4% | 31.70% |
| α98G-L | 89.5% | 23.60% |
| α98G-M | 115.8% | 48.60% |
| α98G-N | 235.3% | 42.50% |
| α98G-P | 46.4% | 62.60% |
| α98G-Q | 478.8% | 51.10% |
| α98G-R | 59.6% | 30.60% |
| α98G-S | 321.2% | 39.30% |
| α98G-T | 155.2% | 36.20% |
| α98G-V | 99.0% | 44.80% |
| α98G-W | 75.8% | 18.60% |
| α98G-Y | 58.5% | 29.60% |
| β95P-A | 226.0% | 44.30% |
| β95P-D | 11.8% | 25.20% |
| β95P-E | 12.4% | 8.98% |
| β95P-F | 20.1% | 33.50% |
| β95P-G | 12.4% | 18.00% |
| β95P-H | 31.4% | 18.00% |
| β95P-I | 18.2% | 48.60% |
| β95P-K | 11.9% | 30.90% |
| β95P-E | 4.6% | 61.80% |
| β95P-M | 72.8% | 59.00% |
| β95P-N | 25.2% | 35.50% |
| β95P-Q | 9.0% | 21.50% |
| β95P-R | 8.7% | 30.70% |
| β95P-S | 58.5% | 25.30% |
| β95P-T | 175.3% | 40.20% |
| β95P-V | 77.8% | 42.00% |
| β95P-W | 5.4% | 0.36% |
| β95P-Y | 38.9% | 24.40% |
| β96G-A | 23.3% | 17.40% |
| β96G-D | 5.6% | 42.80% |
| β96G-E | 7.3% | 28.80% |
| β96G-F | 9.4% | 56.40% |
| β96G-H | 8.1% | 18.20% |

(continued)

| TCR mutant | TCR Mut RLU/WT RLU | mTCRβ% |
|---|---|---|
| β96G-I | 8.2% | 34.10% |
| β96G-K | 24.0% | 39.10% |
| β96G-L | 9.2% | 21.40% |
| β96G-M | 7.6% | 10.60% |
| β96G-N | 8.3% | 14.90% |
| β96G-P | 8.8% | 20.20% |
| β96G-Q | 7.6% | 6.67% |
| β96G-R | 5.9% | 41.90% |
| β96G-S | 17.4% | 24.30% |
| β96G-T | 10.2% | 12.70% |
| β96G-V | 6.3% | 28.00% |
| β96G-W | 8.7% | 28.80% |
| β96G-Y | 8.8% | 20.90% |
| β97Q-A | 12.6% | 31.50% |
| β97Q-D | 10.4% | 54.50% |
| β97Q-E | 5.9% | 55.70% |
| β97Q-F | 15.0% | 15.10% |
| β97Q-G | 6.2% | 28.40% |
| β97Q-H | 11.9% | 29.80% |
| P97Q-I | 6.7% | 21.10% |
| β97Q-K | 7.4% | 33.90% |
| β97Q-L | 6.4% | 40.90% |
| β97Q-M | 5.0% | 11.00% |
| β97Q-N | 5.8% | 19.50% |
| P97Q-P | 10.3% | 20.50% |
| β97Q-R | 12.4% | 59.80% |
| β97Q-S | 175.5% | 42.70% |
| β97Q-T | 11.6% | 22.10% |
| β97Q-V | 2.7% | 47.40% |
| β97Q-W | 3.4% | 33.90% |
| P97Q-Y | 4.1% | 75.70% |
| β98K-A | 3.5% | 57.40% |
| β98R-D | 2.5% | 45.50% |
| β98K-E | 6.2% | 39.90% |
| β98K-F | 3.2% | 72.40% |
| β98R-G | 4.0% | 45.30% |
| β98K-H | 5.4% | 48.40% |
| β98R-I | 4.1% | 47.80% |

(continued)

| TCR mutant | TCR Mut RLU/WT RLU | mTCRβ% |
|---|---|---|
| β98K-K | 7.1% | 57.60% |
| β98R-L | 10.9% | 21.30% |
| β98K-M | 6.8% | 35.50% |
| β98K-N | 4.0% | 75.50% |
| β98R-P | 4.2% | 71.20% |
| β98R-Q | 5.8% | 51.90% |
| β98R-S | 1.5% | 9.96% |
| β98R-T | 3.2% | 51.70% |
| β98R-V | 3.4% | 55.00% |
| β98R-W | 6.1% | 34.40% |
| β98K-Y | 4.3% | 53.50% |
| β99D-A | 5.0% | 31.30% |
| β99D-E | 5.8% | 28.50% |
| β99D-F | 6.3% | 50.10% |
| β99D-G | 10.3% | 29.10% |
| β99D-H | 5.6% | 44.90% |
| β99D-I | 2.2% | 65.10% |
| β99D-K | 2.6% | 74.70% |
| β99D-L | 6.4% | 36.60% |
| β99D-M | 6.2% | 51.40% |
| β99D-N | 4.6% | 57.20% |
| β99D-P | 6.3% | 53.30% |
| β99D-Q | 6.8% | 33.90% |
| β99D-R | 19.7% | 19.30% |
| β99D-S | 6.3% | 47.40% |
| β99D-T | 8.8% | 41.30% |
| β99D-V | 6.9% | 45.50% |
| β99D-W | 5.6% | 30.20% |
| β99D-Y | 6.5% | 32.30% |

The above results showed that:

1) The results of mTCRβ expression detection showed that all TCR mutants could be expressed on the surface of Jurkat-NFAT-Luc cells;

2) In the mutations in CDRα3 and CDRβ3 regions of B13.14.1 TCR, the amino acid positions that had an important impact on TCR specificity were CDRα3-95D, CDRβ3-96G, CDRβ3-98R and CDRβ3-99D, and after the amino acid were mutated at these positions, all mutants could not effectively recognize KRAS$^{G12V}$ antigen peptide presented by the antigen-presenting cells;

The TCR CDRα3 and CDRβ3 mutants with activity (maintaining ≥30% wild-type B13.14.1 TCR RLU signal value) were shown in the table below.

Table 9. Activity-maintaining mutation sites

| TCR region | Wild-type amino acid | TCR mutant with activity |
|---|---|---|
| CDRα3 | 95D | None |
| | 96G | A, D, L, M, Q, R, S, V, W, Y |
| | 97R | A, D, G, H, I, K, L, M, N, P, Q, S, T, V, W, Y |
| | 98G | A, D, E, F, H, I, K, L, M, N, P, Q, R, S, T, V, W, Y |
| CDRβ3 | 95P | A, H, M, S, T, V, Y |
| | 96G | None |
| | 97Q | S |
| | 98R | None |
| | 99D | None |

In the above table, those with a higher ratio of TCR Mut RLU/WT RLU were underlined, which were preferred TCR mutants. The Emax and EC50 (Functional Avidity) of these TCR mutants in recognition of the KRAS-G12V antigen peptide were further determined. B13 LCL cells were loaded with different concentrations of G12V antigen peptide (10 μg/ml, 10-fold dilution, 5 concentrations), and co-incubated with Jurkat-NFAT-Luc cells expressing the TCR mutants for 16-24 hours, the fluorescence signal expression was detected by adding ONE Glo Luciferase, and the results were shown in FIG. 8 and the table below. The results showed that the preferred TCR mutants could recognize the KRAS-G12V mutant antigen peptide in a concentration-dependent manner, and had stronger antigen recognition ability than the wild-type B13.14.1WT.

Table 10. Recognition activity of TCR mutants to KRAS-G12V antigen peptide

| TCR mutant | Emax relative to WT | EC50 μg/ml (Functional Avidity) |
|---|---|---|
| B13.14.1WT | 100% | 0.288 |
| α96G-A | 232% | 0.142 |
| α96G-D | 104% | 0.212 |
| α96G-R | 183% | 0.312 |
| α97R-T | 263% | 0.272 |
| α98G-A | 319% | 0.245 |
| α98G-H | 275% | 0.520 |
| α98G-S | 140% | 0.075 |
| α98G-N | 125% | 0.255 |
| α98G-W | 104% | 0.064 |
| β95P-A | 256% | 0.204 |
| β95P-T | 94% | 0.325 |
| β97Q-S | 192% | 0.123 |

12.2 Screening of TCR MHC-II binding region mutants

The TCR mutant (MHC-II binding region) lentiviral vector was transduced into the Jurkat-NFAT-Luc cell line, and B13 LCL cells loaded with KRAS-G12V antigen (RAS G12V-T15 peptide, SEQ ID NO: 5) were used as the antigen-presenting cells. $2\times10^4$ TCR-T and $2\times10^4$ antigen-loaded B13 LCL cells were co-cultured for 16-24 hours, the fluorescence signal expression was detected by adding ONE Glo Luciferase, and TCR Mut RLU/WT RLU (ratio of RLU signal value of TCR mutant to RLU signal value of wild-type B13.14.1 TCR) was calculated. The results were shown in the table below.

Table 11. Screening results of TCR MHC-II binding region mutants

| TCR mutant | TCR Mut RLU/WT RLU | mTCRβ% |
|---|---|---|
| α30N-A | 46.85% | 97.20% |
| α30N-D | 62.37% | 96.70% |
| α30N-E | 100.86% | 96.10% |
| α30N-F | 48.29% | 98.10% |
| α30N-G | 41.51% | 95.30% |
| α30N-H | 65.37% | 95.50% |
| α30N-1 | 30.71% | 97.60% |
| α30N-K | 67.81% | 94.30% |
| α30N-L | 65.66% | 97.30% |
| α30N-M | 42.72% | 92.70% |
| α30N-P | 90.36% | 94.70% |
| α30N-Q | 90.70% | 96.00% |
| α30N-R | 88.21% | 95.20% |
| α30N-S | 80.22% | 93.40% |
| α30N-T | 76.98% | 95.60% |
| α30N-V | 63.25% | 95.40% |
| α30N-W | 65.55% | 97.80% |
| α30N-Y | 73.45% | 94.90% |
| α32Y-A | 4.68% | 91.30% |
| α32Y-D | 4.13% | 95.20% |
| α32Y-E | 4.59% | 93.10% |
| α32Y-F | 3.81% | 95.70% |
| α32Y-G | 4.03% | 97.40% |
| α32Y-H | 5.60% | 94.00% |
| α32Y-I | 4.64% | 83.80% |
| α32Y-K | 4.00% | 94.40% |
| α32Y-L | 6.35% | 89.60% |
| α32Y-M | 4.94% | 90.20% |
| α32Y-N | 4.17% | 94.90% |
| α32Y-P | 3.98% | 96.90% |
| α32Y-Q | 3.12% | 89.00% |
| α32Y-R | 5.60% | 82.10% |
| α32Y-S | 5.77% | 83.70% |
| α32Y-T | 3.77% | 91.70% |
| α32Y-V | 4.71% | 88.80% |
| α32Y-W | 3.81% | 90.30% |
| α49K-A | 52.40% | 96.30% |

(continued)

| TCR mutant | TCR Mut RLU/WT RLU | mTCRβ% |
|---|---|---|
| α49K-D | 33.44% | 98.70% |
| α49K-E | 46.49% | 96.10% |
| α49K-F | 64.93% | 93.70% |
| α49K-G | 132.39% | 96.30% |
| α49K-H | 85.38% | 97.70% |
| α49K-I | 77.25% | 91.90% |
| α49K-L | 65.99% | 95.50% |
| α49K-M | 44.35% | 96.10% |
| α49K-N | 90.90% | 93.00% |
| α49K-P | 93.63% | 94.70% |
| α49K-Q | 66.90% | 97.10% |
| α49K-R | 91.14% | 96.10% |
| α49K-S | 112.11% | 95.80% |
| α49K-T | 63.63% | 95.50% |
| α49K-V | 100.99% | 93.90% |
| α49K-W | 94.30% | 93.10% |
| α49K-Y | 119.44% | 94.90% |
| α51I-A | 56.93% | 95.90% |
| α51I-D | 93.65% | 93.20% |
| α51I-E | 160.66% | 93.70% |
| α51I-F | 107.59% | 94.40% |
| α51I-G | 90.09% | 90.10% |
| α51I-H | 85.85% | 94.60% |
| α51I-K | 61.76% | 94.40% |
| α51I-L | 75.40% | 95.20% |
| α51I-M | 68.25% | 92.40% |
| α51I-N | 84.17% | 91.00% |
| α51I-P | 82.47% | 86.10% |
| α51I-Q | 71.70% | 93.10% |
| α51I-R | 87.31% | 86.70% |
| α51I-S | 78.65% | 86.40% |
| α51I-T | 68.06% | 93.20% |
| α51I-V | 48.02% | 94.40% |
| α51I-W | 128.73% | 95.20% |
| α51I-Y | 161.75% | 92.60% |
| α52T-A | 139.10% | 87.70% |
| α52T-D | 101.16% | 63.20% |
| α52T-E | 43.18% | 49.60% |

(continued)

| TCR mutant | TCR Mut RLU/WT RLU | mTCRβ% |
|---|---|---|
| α52T-F | 14.98% | 72.90% |
| α52T-G | 9.36% | 80.50% |
| α52T-H | 114.90% | 71.60% |
| α52T-I | 113.54% | 80.70% |
| α52T-K | 11.05% | 78.90% |
| α52T-L | 79.97% | 78.70% |
| α52T-M | 16.65% | 78.70% |
| α52T-N | 72.41% | 81.80% |
| α52T-P | 12.69% | 76.30% |
| α52T-Q | 102.55% | 75.60% |
| α52T-R | 106.98% | 73.20% |
| α52T-S | 76.99% | 77.00% |
| α52T-V | 19.70% | 74.80% |
| α52T-W | 37.02% | 78.40% |
| α52T-Y | 68.60% | 86.30% |
| β48Y-A | 110.29% | 67.20% |
| β48Y-D | 15.07% | 61.90% |
| β48Y-E | 90.23% | 73.80% |
| β48Y-F | 125.84% | 34.60% |
| β48Y-G | 8.49% | 76.60% |
| β48Y-H | 11.40% | 75.40% |
| β48Y-I | 34.66% | 71.00% |
| β48Y-K | 83.49% | 75.40% |
| β48Y-L | 37.97% | 54.30% |
| β48Y-M | 66.36% | 96.10% |
| β48Y-N | 42.82% | 50.10% |
| β48Y-P | 36.89% | 20.80% |
| β48Y-Q | 42.56% | 74.30% |
| β48Y-K | 62.60% | 61.70% |
| β48Y-8 | 34.02% | 54.30% |
| β48Y-T | 40.26% | 46.40% |
| β48Y-V | 63.15% | 67.10% |
| β48Y-W | 32.62% | 54.90% |
| β50Q-A | 81.49% | 84.80% |
| β50Q-D | 19.13% | 95.10% |
| β50Q-E | 2.80% | 89.00% |
| β50Q-F | 42.98% | 73.40% |
| β50Q-G | 34.63% | 58.60% |

(continued)

| TCR mutant | TCR Mut RLU/WT RLU | mTCRβ% |
|---|---|---|
| β50Q-H | 64.73% | 90.20% |
| β50Q-I | 94.65% | 95.60% |
| β50Q-K | 67.50% | 88.60% |
| β50Q-L | 62.99% | 92.90% |
| β50Q-M | 85.81% | 77.80% |
| β50Q-N | 109.90% | 95.10% |
| β50Q-P | 78.46% | 87.50% |
| β50Q-R | 72.99% | 73.20% |
| β50Q-S | 88.72% | 78.10% |
| β50Q-T | 101.44% | 89.00% |
| β50Q-V | 35.26% | 86.10% |
| β50Q-W | 72.66% | 74.20% |
| β50Q-Y | 89.49% | 84.20% |
| β51I-A | 27.57% | 72.70% |
| β51I-D | 4.56% | 81.60% |
| β51I-E | 6.28% | 80.10% |
| β51I-F | 32.79% | 81.20% |
| β51I-G | 5.49% | 80.60% |
| β51I-H | 34.95% | 82.80% |
| β51I-K | 3.82% | 91.90% |
| β51I-L | 25.46% | 78.60% |
| β51I-M | 26.84% | 85.80% |
| β51I-N | 7.61% | 89.00% |
| β51I-P | 19.95% | 39.10% |
| β51I-Q | 12.98% | 86.60% |
| β51I-R | 9.90% | 84.20% |
| β51I-S | 21.00% | 80.30% |
| β51I-T | 88.58% | 86.00% |
| β51I-V | 35.46% | 80.30% |
| β51I-W | 22.56% | 61.00% |
| β51I-Y | 19.64% | 86.20% |
| β54D-A | 63.81% | 92.20% |
| β54D-E | 53.62% | 40.70% |
| β54D-F | 49.69% | 85.80% |
| β54D-G | 92.37% | 89.50% |
| β540-H | 71.90% | 91.40% |
| β54D-I | 67.27% | 94.40% |
| β54D-K | 61.65% | 86.70% |

(continued)

| TCR mutant | TCR Mut RLU/WT RLU | mTCRβ% |
|---|---|---|
| β54D-L | 85.12% | 80.80% |
| β540-M | 91.57% | 91.80% |
| β540-N | 82.50% | 85.60% |
| β540-P | 40.29% | 65.40% |
| β54D-Q | 60.43% | 90.10% |
| β54D-R | 79.21% | 72.40% |
| β54D-S | 57.33% | 26.70% |
| β54D-T | 89.58% | 85.40% |
| β54D-V | 52.11% | 79.90% |
| β54D-W | 62.51% | 85.70% |
| β54D-Y | 84.41% | 87.90% |
| β56Q-A | 100.38% | 84.90% |
| β56Q-D | 92.36% | 77.60% |
| β56Q-E | 104.57% | 84.90% |
| β56Q-F | 82.86% | 79.10% |
| β56Q-G | 58.76% | 61.80% |
| β56Q-H | 96.49% | 88.50% |
| β56Q-I | 57.81% | 15.20% |
| β56Q-K | 71.30% | 87.30% |
| β56Q-L | 100.05% | 88.10% |
| β56Q-M | 72.61% | 74.40% |
| β56Q-N | 75.67% | 73.10% |
| β56Q-P | 78.34% | 55.00% |
| β56Q-R | 96.16% | 85.60% |
| β56Q-S | 58.09% | 77.00% |
| β56Q-T | 118.84% | 73.60% |
| β56Q-V | 56.07% | 66.80% |
| β56Q-W | 99.67% | 83.50% |
| β56Q-Y | 66.39% | 44.10% |

The above results showed that:

1) The results of mTCRβ expression detection showed that all TCR mutants could be expressed on the surface of Jurkat-NFAT-Luc cells;

2) In the mutations in MHCII binding region of B13.14.1 TCR, the amino acid position that had an important impact on TCR specificity was Vα-32Y. After amino acid was mutated at the above position, the mutant could not effectively recognize the KRAS$^{G12V}$ antigen peptide presented by the antigen-presenting cells;

3) The TCR mutants with activity (maintaining ≥30% wild-type B13.14.1 TCR RLU signal value) were showed in the table below.

Table 12. Activity-maintaining mutation sites

| TCR region | Wild-type amino acid | TCR mutant with activity |
|---|---|---|
| Vα | α30N | A, D, E, F, G, H, I, K, L, M, P, Q, R, S, T, V, W, Y |
| | α32Y | None |
| | α49K | A, D, E, F, G, H, I, L, M, N, P, Q, R, S, T, V, W, Y |
| | α51I | A, D, E, F, G, H, K, L, M, N, P, Q, R, S, T, V, W, Y |
| | α52T | A, D, E, H, I, L, N, Q, R, S, W, Y |
| Vβ | β48Y | A, E, F, I, K, L, M, N, P, Q, R, S, T, V, W |
| | β50Q | A, F, G, H, I, K, L, M, N, P, R, S, T, V, W, Y |
| | β51I | F, H, T, V |
| | β54D | A, E, F, G, H, I, K, L, M, N, P, Q, R, S, T, V, W, Y |
| | β56Q | A, D, E, F, G, H, I, K, L, M, N, P, R, S, T, V, W, Y |

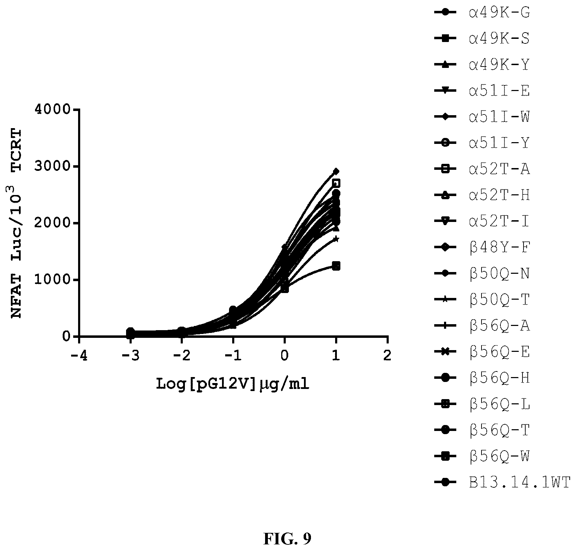

In the above table, those with TCR Mut RLU/WT RLU ratio ≥ 80% were underlined, which were the preferred TCR mutants. The Emax and EC50 (Functional Avidity) of the TCR mutants to recognize the KRAS-G12V antigen peptide were further determined. SW620-DPB cells (SW620 expressing HLA-DPB1*03:01) were loaded with different concentrations of G12V antigen peptide (10 μg/ml, 10-fold dilution, 5 concentrations), and co-incubated with Jurkat-NFAT-Luc cells expressing the TCR mutants for 16-24 hours, and the fluorescence signal expression was detected by adding ONE Glo Luciferase. The results were shown in FIG. 9 and the table below. The results showed that the preferred TCR mutants could recognize the KRAS-G12V mutant antigen peptide in a concentration-dependent manner, and had stronger antigen recognition ability than the wild-type B13.14.1WT.

Table 13. Recognition activity of TCR mutants to KRAS-G12V antigen peptide

| TCR mutant | Emax relative to WT | EC50 μg/ml (Functional Avidity) |
|---|---|---|
| B13.14.1WT | 100% | 0.868 |
| α49K-G | 104% | 0.809 |
| α49K-S | 106% | 1.208 |
| α49K-Y | 82% | 0.960 |
| α51I-E | 95% | 0.927 |
| α51I-W | 134% | 1.235 |
| α51I-Y | 94% | 0.894 |
| α52T-A | 131% | 1.565 |
| α52T-H | 111% | 1.028 |
| α52T-I | 95% | 1.350 |
| β48Y-F | 180% | 9.508 |
| β50Q-N | 97% | 1.189 |
| β50Q-T | 78% | 1.340 |
| β56Q-A | 103% | 0.859 |
| β56Q-E | 100% | 1.052 |
| β56Q-H | 97% | 1.263 |
| β56Q-L | 108% | 1.412 |
| β56Q-T | 131% | 1.762 |

(continued)

| TCR mutant | Emax relative to WT | EC50 μg/ml (Functional Avidity) |
|---|---|---|
| β56Q-W | 53% | 0.556 |

Although the specific models for carrying out the present invention have been described in detail, those skilled in the art will understand that: according to all the teachings that have been disclosed, various modifications and changes can be made to the details, and these changes are all within the protection scope of the present invention. The full scope of the present invention is given by the claims appended hereto and any equivalents thereof.

## Claims

1.  An isolated epitope peptide or variant thereof, wherein the epitope peptide consists of 11-30 (e.g., 30, 29, 28, 27, 26, 25, 24, 23, 22, 21, 20, 19, 18, 17, 16, 15, 14, 13, 12, or 11) consecutive amino acid residues of RAS G12V mutant, and comprises amino acid residues at positions 3-13 of the RAS G12V mutant;

    the variant differs from the epitope peptide from which it is derived only in a substitution of one or several (e.g., 1, 2 or 3) amino acid residues, and does not comprise an amino acid substitution at positions corresponding to amino acid positions 4, 5, 6, 9 and 12 of the RAS G12V mutant, and retains a biological function of the epitope peptide from which it is derived;
    preferably, the epitope peptide consists of 11-25 (e.g., 11-23, 11-13) consecutive amino acid residues of the RAS G12V mutant.

2.  The epitope peptide or variant thereof according to claim 1, wherein the epitope peptide or variant thereof is capable of being presented by a MHC-II molecule, and the epitope peptide or variant thereof associated with the MHC-II molecule is capable of being recognized by a T cell, for example recognized by an antigen-specific T cell receptor on the T cell;

    preferably, the MHC-II molecule is HLA-DP;
    preferably, the HLA-DP comprises HLA-DPB1 *03:01 and/or HLA-DPB1*14:01; preferably, the HLA-DP further comprises one selected from the group consisting of HLA-DPA1*02:02, HLA-DPA1*02:01, HLA-DPA1*01:03.

3.  The epitope peptide or variant thereof according to claim 1 or 2, wherein the amino acid residues at positions 3-13 of the RAS G12V mutant have a sequence as set forth in SEQ ID NO: 2.

4.  The epitope peptide or variant thereof according to any one of claims 1-3, wherein the epitope peptide comprises amino acid residues at positions 3-13, amino acid residues at positions 2-13, amino acid residues at positions 3-14 or amino acid residues at positions 2-14 of the RAS G12V mutant;
    preferably, the amino acid residues at positions 3-13, the amino acid residues at positions 2-13, the amino acid residues at positions 3-14 or the amino acid residues at positions 2-14 of the RAS G12V mutant have a sequence as set forth in SEQ ID NOs: 2-5, respectively.

5.  The epitope peptide or variant thereof according to claims 1-4, wherein the RAS G12V mutant has a sequence as set forth in SEQ ID NO: 1.

6.  The epitope peptide or variant thereof according to any one of claims 1-5, wherein the epitope peptide comprises a sequence set forth in any one of SEQ ID NOs: 2-5; the variant comprises a sequence selected from the group consisting of: (i) a sequence having a substitution, deletion or addition of one or several amino acids (e.g., a substitution, deletion or addition of 1, 2, 3, 4 or 5 amino acids) compared to the sequence set forth in any one of SEQ ID NOs: 2-5; (ii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in any one of SEQ ID NOs: 2-5.

7.  An isolated T cell receptor or antigen-binding fragment thereof, which is capable of specifically recognizing the epitope peptide or variant thereof according to any one of claims 1-6;

preferably, the epitope peptide or variant thereof is presented by a MHC-II molecule;
preferably, the MHC-II molecule is HLA-DP;
preferably, the HLA-DP comprises HLA-DPB1 *03:01 and/or HLA-DPB1*14:01; preferably, the HLA-DP further comprises one selected from the group consisting of HLA-DPA1*02:02, HLA-DPA1*02:01, HLA-DPA1*01:03; preferably, the TCR is soluble or membrane-bound;
preferably, the TCR is a full-length TCR, a soluble TCR or a single-chain TCR.

8. An isolated T cell receptor (TCR) or antigen-binding fragment thereof, which is capable of specifically recognizing RAS G12V mutant, wherein the TCR or antigen-binding fragment thereof comprises an α-chain variable region (Vα) and/or a β-chain variable region (Yβ), wherein,

(a) the Vα comprises CDR1α, CDR2α and CDR3α, wherein the CDR3α comprises a sequence as set forth in AVRDX$_1$X$_2$X$_3$GGNKLT (SEQ ID NO: 25); wherein:

X$_1$ is selected from the group consisting of G, A, D, L, M, Q, R, S, V, W, Y; preferably G, A, D or R;
X$_2$ is selected from the group consisting of R, A, D, G, H, I, K, L, M, N, P, Q, S, T, V, W, Y; preferably R or T;
X$_3$ is selected from the group consisting of G, A, D, E, F, H, I, K, L, M, N, P, Q, R, S, T, V, W, Y; preferably G, A, H, N, S or W;
and/or,

(b) the Vβ comprises CDR1β, CDR2β and CDR30, wherein the CDR3β comprises a sequence as set forth in ASSX$_4$GX$_5$RDNSPLH (SEQ ID NO: 26); wherein:

X$_4$ is selected from the group consisting of P, A, H, M, S, T, V, Y; preferably P, A or T;
X$_5$ is selected from the group consisting of Q or S;
preferably, the TCR is soluble or membrane-bound;
preferably, the TCR is a full-length TCR, a soluble TCR or a single-chain TCR.

9. The TCR or antigen-binding fragment thereof according to claim 8, which has one or more of the following characteristics:

(i) the CDR1α comprises a sequence as set forth in VSGX$_6$PY (SEQ ID NO: 27); wherein, X$_6$ is selected from the group consisting of N, A, D, E, F, G, H, I, K, L, M, P, Q, R, S, T, V, W, Y (preferably N, E, P, Q, R or S);
(ii) the CDR2α comprises a sequence as set forth in YX$_7$X$_8$GDNLV (SEQ ID NO: 28); wherein, X$_7$ is selected from the group consisting of I, A, D, E, F, G, H, K, L, M, N, P, Q, R, S, T, V, W, Y (preferably I, D, E, F, G, H, N, P, R, W or Y); Xs is selected from the group consisting of T, A, D, E, H, I, L, N, Q, R, S, W, Y (preferably T, A, D, H, I, Q or R);
(iii) the CDR1β comprises a sequence set forth in SEQ ID NO: 11;
(iv) the CDR2β comprises a sequence as set forth in SX$_9$X$_{10}$VNX$_{11}$ (SEQ ID NO: 29); wherein, X$_9$ is selected from the group consisting of Q, A, F, G, H, I, K, L, M, N, P, R, S, T, V, W, Y (preferably Q, A, I, M, N, S, T or Y); X$_{10}$ is selected from the group consisting of I, F, H, T, V (preferably I or T); X$_{11}$ selected from the group consisting of D, A, E, F, G, H, I, K, L, M, N, P, Q, R, S, T, V, W, Y (preferably D, G, L, M, N, T or Y).

10. The TCR or antigen-binding fragment thereof according to claim 8 or 9, wherein,

(a) the Vα comprises FR1α, FR2α, FR3α and FR4α, wherein:

the FR1α comprises a sequence set forth in SEQ ID NO: 30;
the FR2α comprises a sequence as set forth in LFWYVQYPNRGLQFLLX$_{12}$ (SEQ ID NO: 38); wherein, X$_{12}$ is selected from the group consisting of K, A, D, E, F, G, H, I, L, M, N, P, Q, R, S, T, V, W, Y (preferably K, G, H, N, P, R, S, V, W or Y);
the FR3α comprises a sequence set forth in SEQ ID NO: 32;
the FR4α comprises a sequence set forth in SEQ ID NO: 33;
and/or,

(b) the Vβ comprises FR1β, FR2β, FR3β and FR4β, wherein:

the FR1β comprises a sequence set forth in SEQ ID NO: 34;

the FR2β comprises a sequence as set forth in MYWYRQDPGQGLRLIYX$_{13}$ (SEQ ID NO: 39); wherein, X$_{13}$ is selected from the group consisting of Y, A, E, F, I, K, L, M, N, P, Q, R, S, T, V, W (preferably Y, A, E, F or K);

the FR3β comprises a sequence as set forth in FX$_{14}$KGDIAEGYSVSREKKESFPLTVTSAQKNPTAFYLC (SEQ ID NO: 40); wherein, X$_{14}$ is selected from the group consisting of Q, A, D, E, F, G, H, I, K, L, M, N, P, R, S, T, V, W, Y (preferably Q, A, D, E, F, H, L, R, T or W);

the PP4β comprises a sequence set forth in SEQ ID NO: 37.

**11.** The TCR or antigen-binding fragment thereof according to any one of claims 8-10, wherein,

(a) the Vα comprises a sequence set forth in SEQ ID NO: 6 or variant thereof, wherein the variant comprises an amino acid substitution at one or more (e.g., 1, 2, 3 or 4) amino acid positions selected from the group consisting of amino acid positions 30, 49, 51, 52, 96, 97, 98 as determined according to the IMGT TCR numbering system;

and/or,

(b) the Vβ comprises a sequence set forth in SEQ ID NO: 7 or variant thereof, and the variant comprises an amino acid substitution at one or more (e.g., 1, 2, 3 or 4) amino acid positions selected from the group consisting of amino acid positions 48, 50, 51, 54, 56, 95, 97 as determined according to the IMGT TCR numbering system.

**12.** The TCR or antigen-binding fragment thereof according to claim 11, wherein the Vα comprises a variant of the sequence set forth in SEQ ID NO: 6, the variant comprises one or more (e.g., 1, 2, 3 or 4) amino acid substitutions selected from the followings, in which the amino acid position is determined according to the IMGT TCR numbering system: substitution of the amino acid at position 30 with A, D, E, F, G, H, I, K, L, M, P, Q, R, S, T, V, W or Y (preferably E, P, Q, R or S); substitution of the amino acid at position 49 with A, D, E, F, G, H, I, L, M, N, P, Q, R, S, T, V, W or Y (preferably G, H, N, P, R, S, V, W or Y); substitution of the amino acid at position 51 with A, D, E, F, G, H, K, L, M, N, P, Q, R, S, T, V, W or Y (preferably D, E, F, G, H, N, P, R, W or Y); substitution of the amino acid at position 52 with A, D, E, H, I, L, N, Q, R, S, W or Y (preferably A, D, H, I, Q or R); substitution of the amino acid at position 96 with A, D, L, M, Q, R, S, V, W or Y (preferably A, D or R); substitution of the amino acid at position 97 with A, D, G, H, I, K, L, M, N, P, Q, S, T, V, W or Y (preferably T); substitution of the amino acid at position 98 with A, D, E, F, H, I, K, L,M, N, P, Q, R, S, T, V, W or Y (preferably A, H, N, S or W);

preferably, the Vβ comprises a sequence set forth in SEQ ID NO:7.

**13.** The TCR or antigen-binding fragment thereof according to claim 11 or 12, wherein the Vβ comprises a variant of the sequence set forth in SEQ ID NO: 7, the variant comprises one or more (e.g., 1, 2, 3 or 4) amino acid substitutions selected from the followings, in which the amino acid position is determined according to the IMGT TCR numbering system: substitution of the amino acid at position 48 with A, E, F, I, K, L, M, N, P, Q, R, S, T, V or W (preferably A, E, F or K); substitution of the amino acid at position 50 with A, F, G, H, I, K, L, M, N, P, R, S, T, V, W or Y (preferably A, I, M, N, S, T or Y); substitution of the amino acid at position 51 with F, H, T or V (preferably T); substitution of the amino acid at position 54 with A, E, F, G, H, I, K, L, M, N, P, Q, R, S, T, V, W or Y (preferably G, L, M, N, T or Y); substitution of the amino acid at position 56 with A, D, E, F, G, H, I, K, L, M, N, P, R, S, T, V, W or Y (preferably A, D, E, F, H, L, R, T or W); substitution of the amino acid at position 95 with A, H, M, S, T, V or Y (preferably A or T); substitution of the amino acid at position 97 with S;

preferably, the Vα comprises a sequence set forth in SEQ ID NO:6.

**14.** The TCR or antigen-binding fragment thereof according to any one of claims 8-13, wherein the TCR or antigen-binding fragment thereof is capable of specifically recognizing the epitope peptide or variant thereof according to any one of claims 1-6;

preferably, the epitope peptide or variant thereof is presented by a MHC-II molecule;
preferably, the MHC-II molecule is HLA-DP;
preferably, the HLA-DP comprises HLA-DPB1 *03:01 and/or HLA-DPB 1*14:01; preferably, the HLA-DP further comprises one selected from the group consisting of HLA-DPA1*02:02, HLA-DPA1*02:01, HLA-DPA1*01:03;
preferably, a T cell expressing on its surface the TCR or antigen-binding fragment thereof is activated under co-cultivation with a second cell (e.g., APC) displaying the epitope peptide or variant thereof according to any one of claims 1-6.

**15.** A conjugate, which comprises the TCR or antigen-binding fragment thereof according to any one of claims 7-14 and an effector moiety conjugated thereto;

preferably, the TCR or antigen-binding fragment thereof is soluble.

16. A fusion protein, which comprises the TCR or antigen-binding fragment thereof according to any one of claims 7-14 and an additional peptide or protein;

preferably, the TCR or antigen-binding fragment thereof is soluble.

17. An isolated nucleic acid molecule, which comprises a nucleotide sequence encoding the epitope peptide or variant thereof according to any one of claims 1-6, or comprises a nucleotide sequence encoding the TCR or antigen-binding fragment thereof according to any one of claims 7-14 or its $\alpha$-chain variable region and/or $\beta$-chain variable region, or comprises a nucleotide sequence encoding the fusion protein according to claim 16.

18. A vector, which comprises the isolated nucleic acid molecule according to claim 17; preferably, the vector comprises a nucleotide sequence encoding the epitope peptide or variant thereof according to any one of claims 1-6;

preferably, the vector comprises a nucleotide sequence encoding the TCR or antigen-binding fragment thereof according to any one of claims 7-14 or its $\alpha$-chain variable region and/or $\beta$-chain variable region;
preferably, the vector comprises a nucleotide sequence encoding the fusion protein according to claim 16;
preferably, the vector is a viral vector, such as a lentiviral vector, a retroviral vector, an adenoviral vector, an adeno-associated viral vector or a baculoviral vector.

19. A host cell, which comprises the isolated nucleic acid molecule according to claim 17, or the vector according to claim 18;

preferably, the host cell comprises a nucleotide sequence encoding the epitope peptide or variant thereof according to any one of claims 1-6;
preferably, the host cell comprises a nucleotide sequence encoding the TCR or antigen-binding fragment thereof according to any one of claims 7-14 or its $\alpha$-chain variable region and/or $\beta$-chain variable region;
preferably, the host cell comprises a nucleotide sequence encoding the fusion protein according to claim 16;
preferably, the host cell comprises *Escherichia coli,* yeast, insect cell, or mammalian cell.

20. A method for preparing the epitope peptide or variant thereof according to any one of claims 1-6, or the TCR or antigen-binding fragment thereof according to any one of claims 7-14, or the fusion protein according to claim 16, which comprises culturing the host cell according to claim 19 under conditions that allow protein expression, and recovering the epitope peptide or variant thereof, or the TCR or antigen-binding fragment thereof, or the fusion protein from a culture of the cultured host cell.

21. An engineered antigen-presenting cell (APC), presenting on its surface the epitope peptide or variant thereof according to any one of claims 1-6;

preferably, the epitope peptide or variant thereof is presented by a MHC-II molecule;
preferably, the MHC-II molecule is HLA-DP;
preferably, the HLA-DP comprises HLA-DPB1 *03:01 and/or HLA-DPB1*14:01; preferably, the HLA-DP further comprises one selected from the group consisting of HLA-DPA1*02:02, HLA-DPA1*02:01, HLA-DPA1*01:03;
preferably, the APC is selected from the group consisting of dendritic cell, monocyte, macrophage, B lymphocyte (e.g., B-lymphoblastoid cell B-LCL), or any combination thereof;
preferably, the APC is positive for HLA-DPB1 *03:01 or positive for HLA-DPB1*14:01; preferably, the APC is further positive for HLA-DPA1*02:02, positive for HLA-DPA1*02:01, or positive for HLA-DPA1*01:03;
preferably, the APC is isolated from a subject positive for HLA-DPB1*03:01 or a subject positive for HLA-DPB1*14:01; preferably, the subject is further positive for HLA-DPA1*02:02, positive for HLA-DPA1*02:01, or positive for HLA-DPA1*01:03.

22. A method for preparing the engineered APC according to claim 21, which comprises: (1) providing an APC from a subject; (2) contacting the APC with the epitope peptide or variant thereof according to any one of claims 1-6 *in vitro* or introducing an expression vector comprising a nucleotide sequence encoding the epitope peptide or variant thereof according to any one of claims 1-6 into the APC, thereby obtaining an APC presenting on its surface the epitope peptide or variant thereof.

23. An engineered immune cell, expressing on its surface the TCR or antigen-binding fragment thereof according to any one of claims 7-14;

preferably, the engineered immune cell comprises a nucleotide sequence encoding the TCR or antigen-binding fragment thereof according to any one of claims 7-14;
preferably, the immune cell is a lymphocyte;
preferably, the immune cell is selected from the group consisting of T cell (e.g., αβT cell, γδT cell or iPSC-derived T cell), tumor infiltrating lymphocyte (TIL), natural killer (NK) cell, natural killer T (NKT) cell, or any combination thereof.

24. A method for preparing the engineered immune cell according to claim 23, which comprises: (1) providing an immune cell from a subject; (2) introducing the isolated nucleic acid molecule according to claim 17 or the vector according to claim 18 into the immune cell of step (1), the nucleic acid molecule or vector comprising a nucleotide sequence encoding the TCR or antigen-binding fragment thereof according to any one of claims 7-14, thereby obtaining an immune cell expressing the TCR or antigen-binding fragment thereof;

preferably, in step (1), the immune cell undergoes pretreatment; the pretreatment comprises sorting, activation and/or proliferation of the immune cell;
preferably, the pretreatment comprises contacting the immune cell with one or more selected from the group consisting of anti-CD3 antibody, anti-CD28 antibody, IL-2 and IL-15, to stimulate the immune cell and induce its proliferation, thereby producing a pretreated immune cell.

25. A pharmaceutical composition, which comprises the epitope peptide or variant thereof according to any one of claims 1-6, a nucleic acid molecule or carrier or host cell comprising a nucleotide sequence encoding the epitope peptide or variant thereof, or the engineered antigen-presenting cell (APC) according to claim 21; and a pharmaceutically acceptable carrier and/or excipient;

preferably, the pharmaceutical composition is a tumor vaccine;
preferably, the pharmaceutical composition comprises an adjuvant;
preferably, the pharmaceutical composition further comprises an additional therapeutic agent, such as an antitumor agent or an immunopotentiator;
preferably, the antitumor agent is selected from the group consisting of alkylating agent, mitotic inhibitor, antitumor antibiotic, antimetabolite, topoisomerase inhibitor, tyrosine kinase inhibitor, radionuclide agent, radiosensitizer, anti-angiogenic agent, cytokine, immune checkpoint inhibitor (e.g., PD-1 antibody, PD-L1 antibody, CTLA-4 antibody, LAG-3 antibody, or TIM3 antibody);
preferably, the immunopotentiator is selected from the group consisting of immunostimulatory antibody (e.g., anti-CD3 antibody, anti-CD28 antibody, anti-CD40L (CD154) antibody, anti-41BB (CD137) antibody, anti-OX40 antibody, anti-GITR antibody or any combination thereof), or immunostimulatory cytokine (e.g., IL-2, IL-3, IL-12, IL-15, IL-18, IFN-γ, IL-10, TGF-β, GM-CSF, or any combination thereof).

26. A pharmaceutical composition, which comprises the TCR or antigen-binding fragment thereof according to any one of claims 7-14, the conjugate according to claim 15, the fusion protein according to claim 16, a nucleic acid molecule or vector or host cell comprising a nucleotide sequence encoding the TCR or antigen-binding fragment thereof or the fusion protein, or the engineered immune cell according to claim 23; and a pharmaceutically acceptable carrier and/or excipient;

preferably, the pharmaceutical composition further comprises an additional therapeutic agent, such as an antitumor agent or an immunopotentiator;
preferably, the antitumor agent is selected from the group consisting of alkylating agent, mitotic inhibitor, antitumor antibiotic, antimetabolite, topoisomerase inhibitor, tyrosine kinase inhibitor, radionuclide agent, radiosensitizer, anti-angiogenic agent, cytokine, immune checkpoint inhibitor (e.g., PD-1 antibody, PD-L1 antibody, CTLA-4 antibody, LAG-3 antibody, or TIM3 antibody);
preferably, the immunopotentiator is selected from the group consisting of immunostimulatory antibody (e.g., anti-CD3 antibody, anti-CD28 antibody, anti-CD40L (CD154) antibody, anti-41BB (CD137) antibody, anti-OX40 antibody, anti-GITR antibody or any combination thereof) or immunostimulatory cytokine (e.g., IL-2, IL-3, IL-12, IL-15, IL-18, IFN-γ, IL-10, TGF-β, GM-CSF, or any combination thereof).

27. Use of the epitope peptide or variant thereof according to any one of claims 1-6, a nucleic acid molecule or vector or host cell comprising a nucleotide sequence encoding the epitope peptide or variant thereof, or the engineered antigen-presenting cell (APC) according to claim 21, or the pharmaceutical composition according to claim 25, in the manufacture of a medicament, wherein the medicament is used for inducing an immune response against a tumor with RAS G12V mutation in a subject, and/or preventing or treating a tumor with RAS G12V mutation in a subject;

> preferably, the tumor with RAS G12V mutation is selected from the group consisting of colorectal cancer, pancreatic cancer, gastric cancer, lung cancer, endometrial cancer, ovarian cancer, multiple myeloma, melanoma, thyroid cancer, bladder cancer, prostate cancer, breast cancer, head and neck cancer, or acute myeloid leukemia;
> preferably, the subject is a human;
> preferably, the subject is positive for HLA-DPB1 *03:01 or positive for HLA-DPB1 *14:01; preferably, the subject is further positive for HLA-DPA1*02:02, positive for HLA-DPA1*02:01, or positive for HLA-DPA1*01:03;
> preferably, the epitope peptide or variant thereof, nucleic acid molecule or vector or host cell, engineered antigen presenting cell (APC), or pharmaceutical composition is administered in combination with an additional therapeutic agent, for example, administered simultaneously, separately or sequentially; preferably, the additional therapeutic agent is an immunostimulant or an antitumor agent.

28. Use of the TCR or antigen-binding fragment thereof according to any one of claims 7-14, the conjugate according to claim 15, the fusion protein according to claim 16, a nucleic acid molecule or vector or host cell comprising a nucleotide sequence encoding the TCR or antigen-binding fragment thereof or the fusion protein, or the engineered immune cell according to claim 23, or the pharmaceutical composition according to claim 26, in the manufacture of a medicament, wherein the medicament is used for inducing an immune response against a tumor with RAS G12V mutation in a subject, and/or preventing or treating a tumor with RAS G12V mutation in a subject; wherein the nucleic acid molecule, vector or host cell comprises a nucleotide sequence encoding the TCR or antigen-binding fragment thereof or the fusion protein;

> preferably, the tumor with RAS G12V mutation is selected from the group consisting of colorectal cancer, pancreatic cancer, gastric cancer, lung cancer, endometrial cancer, ovarian cancer, multiple myeloma, melanoma, thyroid cancer, bladder cancer, prostate cancer, breast cancer, head and neck cancer, or acute myeloid leukemia;
> preferably, the subject is a human;
> preferably, the subject is positive for HLA-DPB1 *03:01 or positive for HLA-DPB 1 *14:01; preferably, the subject is further positive for HLA-DPA1*02:02, positive for HLA-DPA1* 02:01, or positive for HLA-DPA1*01:03;
> preferably, the TCR or antigen-binding fragment thereof, conjugate, fusion protein, nucleic acid molecule or vector or host cell, engineered immune cell, or pharmaceutical composition is administered in combination with an additional therapeutic agent, for example, administered simultaneously, separately or sequentially; preferably, the additional therapeutic agent is an immunostimulant or antitumor agent.

29. A method for inducing an immune response against a tumor with RAS G12V mutation in a subject, and/or preventing or treating a tumor with RAS G12V mutation in a subject, wherein the method comprises administering to the subject in need thereof an effective amount of the epitope peptide or variant thereof according to any one of claims 1-6, a nucleic acid molecule or vector or host cell comprising a nucleotide sequence encoding the epitope peptide or variant thereof, or the engineered antigen-presenting cell (APC) according to claim 21, or the pharmaceutical composition according to claim 25;

> preferably, the tumor with RAS G12V mutation is selected from the group consisting of colorectal cancer, pancreatic cancer, gastric cancer, lung cancer, endometrial cancer, ovarian cancer, multiple myeloma, melanoma, thyroid cancer, bladder cancer, prostate cancer, breast cancer, head and neck cancer, or acute myeloid leukemia;
> preferably, the subject is a human;
> preferably, the subject is positive for HLA-DPB1 *03:01 or positive for HLA-DPB 1 *14:01; preferably, the subject is further positive for HLA-DPA1*02:02, positive for HLA-DPA1* 02:01, or positive for HLA-DPA1*01:03;
> preferably, the method further comprises administering to the subject an additional therapeutic agent, such as an immunopotentiator or an antitumor agent.

30. A method for inducing an immune response against a tumor with RAS G12V mutation in a subject, and/or preventing or treating a tumor with RAS G12V mutation in a subject, wherein the method comprises administering to the subject in need thereof an effective amount of the TCR or antigen-binding fragment thereof according to any one of claims 7-14, the conjugate according to claim 15, the fusion protein according to claim 16, a nucleic acid molecule or vector or host cell comprising a nucleotide sequence encoding the TCR or antigen-binding fragment thereof or the fusion protein, or the engineered immune cell according to claim 23, or the pharmaceutical composition according to claim 26;

preferably, the tumor with RAS G12V mutation is selected from the group consisting of colorectal cancer, pancreatic cancer, gastric cancer, lung cancer, endometrial cancer, ovarian cancer, multiple myeloma, melanoma, thyroid cancer, bladder cancer, prostate cancer, breast cancer, head and neck cancer, or acute myeloid leukemia;

preferably, the subject is a human;

preferably, the subject is positive for HLA-DPB 1 *03:01 or positive for HLA-DPB 1 *14:01; preferably, the subject is further positive for HLA-DPA1*02:02, positive for HLA-DPA1* 02:01, or positive for HLA-DPA1*01:03;

preferably, the method further comprises administering to the subject an additional therapeutic agent, such as an immunopotentiator or an antitumor agent;

preferably, the method comprises: (1) providing an immune cell required by the subject; (2) introducing a nucleotide sequence encoding the TCR or antigen-binding fragment thereof according to any one of claims 7-14 into the immune cell of step (1), thereby obtaining an immune cell expressing on its surface the TCR or antigen-binding fragment thereof; (3) administering the immune cell obtained in step (2) to the subject;

preferably, the immune cell is a lymphocyte;

preferably, the immune cell is selected from the group consisting of T cell (e.g., $\alpha\beta$T cell, $\gamma\delta$T cell or iPSC-derived T cell), tumor infiltrating lymphocyte (TIL), natural killer (NK) cell, natural killer T (NKT) cell, or any combination thereof.

**FIG. 1**

**FIG. 2**

**FIG. 3**

**FIG. 4**

**FIG. 5**

**FIG. 6A**

**FIG. 6B**

**FIG. 7**

**FIG. 8**

**FIG. 9**

| INTERNATIONAL SEARCH REPORT | International application No. |
|---|---|
| | **PCT/CN2021/132337** |

**A. CLASSIFICATION OF SUBJECT MATTER**

C07K 14/82(2006.01)i; C07K 14/725(2006.01)i; A61K 39/00(2006.01)i; A61K 35/17(2015.01)i; A61P 35/00(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS, CNTXT, SIPOABS, DWPI, WOTXT, USTXT, EPTXT, CNKI, 万方数据库, WANFANG DATABASE, EMBL, NCBI: 表位肽, RAS,KRAS,G12V,MHC,HLA,序列检索, sequence search, 牟男, mounan, 于跃, yuyue, 袁纪军, yuanjijun, 上海吉利贝生物科技, genbase, PD-L1, PD-1, 抗体, 联合, immumotoxin, resimmune, 癌症, cancer, tumor

**C. DOCUMENTS CONSIDERED TO BE RELEVANT**

| Category* | Citation of document, with indication, where appropriate, of the relevant passages | Relevant to claim No. |
|---|---|---|
| PX | WO 2021173902 A1 (THE UNITED STATES OF AMERICA, AS REPRESENTED BY THE SECRETARY, DEPARTMENT OF HEALTH AND HUMAN SERVICES) 02 September 2021 (2021-09-02) abstract, claims 1-6 and 30-50, and sequence table, SEQ ID NO: 30 | 1-7, 15-30 |
| X | WO 2019246286 A1 (NEON THERAPEUTICS, INC.) 26 December 2019 (2019-12-26) abstract, claims 1-32 and 93-95, and figure 1 | 1-7, 15-30 |
| X | CN 106414493 A (TARGOVAX AS) 15 February 2017 (2017-02-15) claims 1-7, and sequence table, SEQ ID NO: 12 | 1, 3-6 |
| Y | CN 106414493 A (TARGOVAX AS) 15 February 2017 (2017-02-15) claims 1-7, and sequence table, SEQ ID NO: 12 | 2-7, 15-30 |
| Y | CN 111201237 A (UNITED STATES DEPARTMENT OF HEALTH AND HUMAN SERVICES) 26 May 2020 (2020-05-26) claims 1-33, embodiment 2, and sequence table, SEQ ID NOs: 21-24 | 2-7, 15-30 |
| A | CN 108395479 A (GAO, Jun et al.) 14 August 2018 (2018-08-14) description, paragraphs 8-71 | 1-30 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 February 2022** | **22 February 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/132337** |

**C.    DOCUMENTS CONSIDERED TO BE RELEVANT**

| Category* | Citation of document, with indication, where appropriate, of the relevant passages | Relevant to claim No. |
| --- | --- | --- |
| A | WO 2020089433 A2 (OSPEDALE SAN RAFFAELE S.R.L) 07 May 2020 (2020-05-07) <br>     entire document | 1-30 |
| A | CN 110856751 A (SYNIMMUNE GMBH) 03 March 2020 (2020-03-03) <br>     entire document | 1-30 |
| A | GenBank: ANO56466.1. "T-Cell Receptor Alpha Chain Variable Region, Partial [Homo Sapiens]" <br> *NCBI*, 05 July 2016 (2016-07-05), <br>     entire document | 1-30 |

Form PCT/ISA/210 (second sheet) (January 2015)

**INTERNATIONAL SEARCH REPORT**

| International application No. |
| --- |
| **PCT/CN2021/132337** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**

| International application No. |
| --- |
| **PCT/CN2021/132337** |

| Box No. II    Observations where certain claims were found unsearchable (Continuation of item 2 of first sheet) |
| --- |

This international search report has not been established in respect of certain claims under Article 17(2)(a) for the following reasons:

1. ☑ Claims Nos.: **29-30**
   because they relate to subject matter not required to be searched by this Authority, namely:

    [1]   Claims 29 and 30 relate to a method for treating tumors, and relate to the subject matter defined in PCT Rule 39.1 that does not warrant a search conducted by the international searching authority: (4) a method for treatment of a human body or animal body by surgery or therapy and a diagnostic method implemented on a human body or animal body. An international search is performed on the basis of a use in the preparation of drugs for treating tumors.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/132337**

| Patent document cited in search report | | | Publication date (day/month/year) | Patent family member(s) | | | Publication date (day/month/year) |
|---|---|---|---|---|---|---|---|
| WO | 2021173902 | A1 | 02 September 2021 | None | | | |
| WO | 2019246286 | A1 | 26 December 2019 | BR | 112020025764 | A2 | 11 May 2021 |
| | | | | IL | 279458 | D0 | 31 January 2021 |
| | | | | JP | 2021527426 | A | 14 October 2021 |
| | | | | EP | 3810182 | A1 | 28 April 2021 |
| | | | | US | 2021268091 | A1 | 02 September 2021 |
| | | | | AU | 2019290127 | A1 | 28 January 2021 |
| | | | | KR | 20210032393 | A | 24 March 2021 |
| | | | | CA | 3103983 | A1 | 26 December 2019 |
| | | | | SG | 11202012653X | A | 28 January 2021 |
| | | | | CN | 112638404 | A | 09 April 2021 |
| | | | | PH | 12020500677 | A1 | 04 October 2021 |
| | | | | TW | 202015719 | A | 01 May 2020 |
| CN | 106414493 | A | 15 February 2017 | BR | 112016025764 | A2 | 16 January 2018 |
| | | | | US | 2017065694 | A1 | 09 March 2017 |
| | | | | US | 9757439 | B2 | 12 September 2017 |
| | | | | CL | 2016002794 | A1 | 09 June 2017 |
| | | | | SG | 11201608712 P | A | 29 November 2016 |
| | | | | IL | 248671 | D0 | 31 January 2017 |
| | | | | IL | 248671 | A | 31 August 2021 |
| | | | | MX | 2016014414 | A | 23 February 2017 |
| | | | | ES | 2716923 | T3 | 18 June 2019 |
| | | | | RU | 2016147527 | A | 08 June 2018 |
| | | | | RU | 2016147527 | A3 | 21 January 2019 |
| | | | | RU | 2700929 | C2 | 24 September 2019 |
| | | | | AU | 2015257774 | A1 | 22 December 2016 |
| | | | | AU | 2015257774 | B2 | 20 December 2018 |
| | | | | EP | 3140320 | A1 | 15 March 2017 |
| | | | | EP | 3140320 | B1 | 26 December 2018 |
| | | | | WO | 2015169804 | A1 | 12 November 2015 |
| | | | | DK | 3140320 | T3 | 15 April 2019 |
| | | | | CA | 2947963 | A1 | 12 November 2015 |
| | | | | JP | 2017514847 | A | 08 June 2017 |
| | | | | SG | 10201809701T | A | 28 December 2018 |
| | | | | JP | 2020100650 | A | 02 July 2020 |
| | | | | KR | 20170002563 | A | 06 January 2017 |
| CN | 111201237 | A | 26 May 2020 | TW | 201920251 | A | 01 June 2019 |
| | | | | SG | 11202002425P | A | 29 April 2020 |
| | | | | JP | 2020534828 | A | 03 December 2020 |
| | | | | MA | 50180 | A | 26 May 2021 |
| | | | | CA | 3076339 | A1 | 28 March 2019 |
| | | | | AU | 2018335274 | A1 | 09 April 2020 |
| | | | | EP | 3684799 | A1 | 29 July 2020 |
| | | | | KR | 20200051804 | A | 13 May 2020 |
| | | | | IL | 273254 | D0 | 30 April 2020 |
| | | | | EA | 202090652 | A1 | 21 August 2020 |
| | | | | BR | 112020005469 | A2 | 29 September 2020 |
| | | | | WO | 2019060349 | A1 | 28 March 2019 |
| | | | | AR | 112902 | A1 | 26 December 2019 |
| | | | | US | 2019085046 | A1 | 21 March 2019 |

Form PCT/ISA/210 (patent family annex) (January 2015)

**INTERNATIONAL SEARCH REPORT**
Information on patent family members

International application No.

**PCT/CN2021/132337**

| Patent document cited in search report | | | Publication date (day/month/year) | Patent family member(s) | | | Publication date (day/month/year) |
|---|---|---|---|---|---|---|---|
| CN | 108395479 | A | 14 August 2018 | None | | | |
| WO | 2020089433 | A2 | 07 May 2020 | AU | 2019370989 | A1 | 10 June 2021 |
| | | | | GB | 201817821 | D0 | 19 December 2018 |
| | | | | CA | 3117272 | A1 | 07 May 2020 |
| | | | | EP | 3873925 | A2 | 08 September 2021 |
| | | | | CN | 113195527 | A | 30 July 2021 |
| | | | | WO | 2020089433 | A3 | 23 July 2020 |
| CN | 110856751 | A | 03 March 2020 | US | 2020289567 | A1 | 17 September 2020 |
| | | | | US | 2021393686 | A1 | 23 December 2021 |
| | | | | TW | 202023583 | A | 01 July 2020 |
| | | | | EP | 3842523 | A1 | 30 June 2021 |
| | | | | WO | 2020038492 | A1 | 27 February 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2003020763 A
- WO 2004033685 A
- WO 2011044186 A
- US 8361794 B
- US 8906383 B

### Non-patent literature cited in the description

- *Front Oncol.,* 2014, vol. 4, 378
- **LEFRANC ; LEFRANC.** T cell receptor Facts book. Academic Press, 2001
- **LEFRANC.** *The Immunologist,* 1999, vol. 7, 132-136
- **LEFRANC et al.** *Nucleic Acids Res,* 1999, vol. 27, 209-212
- **LEFRANC.** T cell receptor facts book. Academic Press, 2001
- **LEFRANC et al.** *Dev Comp Immunol,* 2003, vol. 27 (1), 55-77
- **KABAT et al.** *Sequences of proteins of immunological interest,* 1991
- **LEFRANC ; LEFRANC.** T cell receptor facts book. Academic Press, 2001
- **MALMQVIST M.** *Nature,* 1993, vol. 361, 186-187
- **DAVIES et al.** *Annual Rev Biochem,* 1990, vol. 59, 439-473
- **NEEDLEMAN et al.** *J. Mol. Biol.,* 1970, vol. 48, 443-453
- **E. MEYERS ; W. MILLER.** *Comput. Appl. Biosci,* 1988, vol. 4, 11-17
- **NEEDLEMAN ; WUNSCH.** *J. Mol. Biol.,* 1970, vol. 48, 444-453
- **BRUMMELL et al.** *Biochem.,* 1993, vol. 32, 1180-1187
- **KOBAYASHI et al.** *Protein Eng,* 1999, vol. 12 (10), 879-884
- **BURKS et al.** *Proc. Natl Acad. Set USA,* 1997, vol. 94, 412-417
- Immunology-A Synthesis. Sinauer Associates, 1991
- Remington's Pharmaceutical Sciences. Mack Publishing Company, 1995
- **J. SAMBROOK et al.** Molecular Cloning: A Laboratory Manual. Cold Spring Harbor Laboratory Press, 1989
- **F. M. AUSUBEL et al.** Short protocols in Molecular Biology. John Wiley & Sons, Inc, 1995